# EUROPEAN PATENT APPLICATION

(11) **EP 3 906 942 A1**
(43) Date of publication of application: **10.11.2021**
(21) Application number: 19909811.2
(22) Date of filing: 28.11.2019
(51) Int. Cl.: A61K 39/395, A61K 45/00, A61P 31/00, A61P 35/00, A61P 43/00, C12Q 1/02, G01N 33/15, G01N 33/50, G01N 33/574, G01N 33/68

(54) **PREDICTION AND/OR DETERMINATION MARKER FOR EFFECTIVENESS OF TREATMENT WITH DRUG CONTAINING PD-1 SIGNAL INHIBITOR**

(30) Priority: 04.01.2019 JP 2019000181
(71) Applicant: Kyoto University, Kyoto-shi, Kyoto 606-8501 (JP)
(72) Inventor: HONJO, Tasuku, Kyoto-shi, Kyoto 606-8501 (JP); CHAMOTO, Kenji, Kyoto-shi, Kyoto 606-8501 (JP); MATSUDA, Fumihiko, Kyoto-shi, Kyoto 606-8501 (JP)
(74) Representative: J A Kemp LLP
(86) International application number: PCT/JP2019/046582
(87) International publication number: WO 2020/149026

(57) **Abstract**

A marker for judging the efficacy of therapy with a PD-1 signal inhibitor-containing drug before or at an early stage of the therapy is provided. As biomarkers for predicting or judging the efficacy of therapy with a PD-1 signal inhibitor-containing drug, the following (i) and/or (ii) is used.
(i) at least one metabolite in serum and/or plasma selected from the group consisting of alanine, 4-cresol, cysteine, hippuric acid, oleic acid, indoxyl sulfate, ribose, indoleacetate, uric acid, trans-urocanic acid, pipecolic acid, N-acetylglucosamine, indolelactic acid, arabinose, arabitol, cystine, indoxyl sulfate, gluconic acid, citrulline, creatinine, N-acetylaspartic acid, pyroglutamic acid, trimethyllysine, asy-dimethylarginine, sym-dimethylarginine, methylhistidine, acylcarnitine, 3-aminoisobutyric acid, acetylcarnosine, arginine, N-acetylornitine, 3-hydroxyisovaleric acid, pyruvic acid, α-ketoglutaric acid, GSSG, 2-hydrobutyric acid, 1,5-anhydro-D-sorbitol, glutamine, glycine, lysine, taurine, AMP, acetylcarnosine, 3-hydroxybutyric acid, 2-hydroxyisovaleric acid, acetoacetic acid, tryptophan, 2-hydroxyglutaric acid, malic acid, quinolinic acid, caproic acid, isoleucine, GSH and 3-OH-kynurenine
(ii) at least one cellular marker in peripheral blood selected from the group consisting of the frequency of CD4⁺ T cells among peripheral blood mononuclear cells (% of CD4⁺ T cells among PBMC), the frequency of CD8⁺ T cells among peripheral blood mononuclear cells (% of CD8⁺ T cells among PBMC), the frequency of naive T cells among CD8⁺ T cells (% of Tnaive among CD8⁺ T cells), the frequency of central memory T cells among CD4⁺ T cells (% of Tcm among CD4⁺ T cells), the frequency of central memory T cells among CD8⁺ T cells (% of Tcm among CD8⁺ T cells), the frequency of effector memory T cells among CD8⁺ T cells (% of Tem among CD8⁺ T cells), the frequency of terminally differentiated effector memory T cells among CD4⁺ T cells (% of Temra among CD4⁺ T cells), the frequency of terminally differentiated effector memory T cells among CD8⁺ T cells (% of Temra among CD8⁺ T cells), the ratio of mitochondrial activation status in CD8⁺ T cells to mitochondrial activation status in CD4⁺ T cells [e.g., the ratio of mitochondrial reactive oxygen species production in CD8⁺ T cells to mitochondrial reactive oxygen species production in CD4⁺ T cells (Mito SOX CD8/CD4), the ratio of mitochondrial mass in CD8⁺ T cells to mitochondrial mass in CD4⁺ T cells (Mito mass CD8/CD4)], PGC-la and PGC-1β expression in CD8⁺ T cells (PGC-1αβ (MF1) of CD8⁺ T cells), the frequency of PD-1 highly expressing CD8⁺ T cell population (% of PD-1^{high} among CD8⁺ T cells), the frequency of FoxP3 lowly expressing CD45RA⁺ T cell population among CD4⁺ T cells (% of FoxP3^{low} CD45RA⁺ among CD4⁺ T cells), the frequency of T-bet highly expressing T cell population among CD4⁺ T cells (% of T-bet^{high} among CD4⁺ T cells), the frequency of T-bet lowly expressing T cell population among CD4⁺ T cells (% of T-bet^{low} among CD4⁺ T cells), the frequency of T-bet expressing T cell population among CD8⁺ T cells (% of T-bet⁺ among CD8⁺ T cells) and the frequency of T-bet and EOMES expressing T cell population among CD8⁺ T cells (% of T-bet⁺ EOMES⁺ among CD8⁺ T cells).

## Description

### TECHNICAL FIELD

The present invention relates to markers for predicting and/or judging the efficacy of therapies with PD-1 signal inhibitor-containing drugs.

### BACKGROUND ART

The results of recent clinical trials have revealed that the anti-PD-1 antibody therapy is more effective than conventional standard therapies in various cancers (Non-Patent Documents Nos. 1-3). The response rate of PD-1 antibody therapy was 20-30% when used alone and 60-70% when combined with other therapies, showing a dramatic improvement compared to conventional immunotherapies. However, about one half of the patients were non-responsive. Little is known about why those patients are non-responsive to the PD-1 antibody therapy.

At present, two markers are approved by the Food and Drug Administration (FDA), USA for anti-PD-1 antibody therapy.

One is PD-L1 expression on at least 50% of tumor cells. Pembrolizumab is applicable to those patients with 50% or greater expression in first-line treatment (Non-Patent Document No. 4).

The other relates to the number of mutations in tumor cells. When more than a specific number of mutations are found in solid tumor cells, Nivolumab may be applied to such tumors (Non-Patent Document No. 5).

While the markers approved by FDA focus on tumors, the present inventors have found markers focusing on hosts' blood metabolites and immunostimulation (Patent Document No. 1).

### PRIOR ART LITERATURE

### Non-Patent Documents

Non-Patent Document No. 1: Borghaei H, Paz-Ares L, Horn L, et al: Nivolumab versus Docetaxel in Advanced Nonsquamous Non-Small-Cell Lung Cancer. N Engl J Med, 373:1627-1639, 2015
Non-Patent Document No. 2: Hamanishi J, Mandai M, Ikeda T, et al: Safety and Antitumor Activity of Anti-PD-1 Antibody, Nivolumab, in Patients with Platinum-Resistant Ovarian Cancer. J Clin Oncol, 2015
Non-Patent Document No. 3: Motzer RJ, Escudier B, McDermott DF, et al: Nivolumab versus Everolimus in Advanced Renal-Cell Carcinoma. N Engl J Med, 373:1803-1813, 2015
Non-Patent Document No. 4: Pembrolizumab versus Chemotherapy for PD-L1-Positive Non-Small-Cell Lung Cancer. Martin Reck et al. KEYNOTE-024 Investigators. N Engl J Med. 2016 Nov 10;375(19):1823-1833. Epub 2016 Oct 8.
Non-Patent Document No. 5: Tumor Mutational Burden and Response Rate to PD-1 Inhibition. Yarchoan M, Hopkins A, Jaffee EM. N Engl J Med. 2017 Dec 21;377(25):2500-2501

### Patent Documents

Patent Document No. 1: WO2018/084204

### DISCLOSURE OF THE INVENTION

### PROBLEM FOR SOLUTION BY THE INVENTION

It is an object of the present invention to provide markers for predicting and/or judging the efficacy prior to or at an early stage of disease therapy with a PD-1 signal inhibitor-containing drug.

### MEANS TO SOLVE THE PROBLEM

The present inventors have investigated into plasma metabolites and cellular markers (including mitochondria-related markers) derived from the blood of non-small cell lung cancer (NSCLC) patients before and after treatment with an anti-PD-1 antibody Nivolumab. The results revealed that metabolites related to microbiome, energy metabolism and redox are correlated with responsiveness to Nivolumab. The present inventors have also revealed that cellular markers are correlated with plasma metabolites. The present invention has been achieved based on these findings.

A summary of the present invention is described as below.
(1) A test method comprising predicting and/or judging the efficacy of therapy with a PD-1 signal inhibitor-containing drug using the following (i) and/or (ii) as indicator(s):
   (i) at least one metabolite in serum and/or plasma selected from the group consisting of alanine, 4-cresol, cysteine, hippuric acid, oleic acid, indoxyl sulfate, ribose, indoleacetate, uric acid, trans-urocanic acid, pipecolic acid, N-acetylglucosamine, indolelactic acid, arabinose, arabitol, cystine, indoxyl sulfate, gluconic acid, citrulline, creatinine, N-acetylaspartic acid, pyroglutamic acid, trimethyllysine, asy-dimethylarginine, sym-dimethylarginine, methylhistidine, acylcarnitine, 3-aminoisobutyric acid, acetylcarnosine, arginine, N-acetylornitine, 3-hydroxyisovaleric acid, pyruvic acid, α-ketoglutaric acid, GSSG, 2-hydrobutyric acid, 1,5-anhydro-D-sorbitol, glutamine, glycine, lysine, taurine, AMP, acetylcarnosine, 3-hydroxybutyric acid, 2-hydroxyisovaleric acid, acetoacetic acid, tryptophan, 2-hydroxyglutaric acid, malic acid, quinolinic acid, caproic acid, isoleucine, GSH and 3-OH-kynurenine
   (ii) at least one cellular marker in peripheral blood selected from the group consisting of the frequency of CD4⁺ T cells among peripheral blood mononuclear cells (% of CD4⁺ T cells among PBMC), the frequency of CD8⁺ T cells among peripheral blood mononuclear cells (% of CD8⁺ T cells among PBMC), the frequency of naive T cells among CD8⁺ T cells (% of Tnaive among CD8⁺ T cells), the frequency of central memory T cells among CD4⁺ T cells (% of Tcm among CD4⁺ T cells), the frequency of central memory T cells among CD8⁺ T cells (% of Tcm among CD8⁺ T cells), the frequency of effector memory T cells among CD8⁺ T cells (% of Tem among CD8⁺ T cells), the frequency of terminally differentiated effector memory T cells among CD4⁺ T cells (% of Temra among CD4⁺ T cells), the frequency of terminally differentiated effector memory T cells among CD8⁺ T cells (% of Temra among CD8⁺ T cells), the ratio of mitochondrial activation status in CD8⁺ T cells to mitochondrial activation status in CD4⁺ T cells [e.g., the ratio of mitochondrial reactive oxygen species production in CD8⁺ T cells to mitochondrial reactive oxygen species production in CD4⁺ T cells (Mito SOX CD8/CD4), the ratio of mitochondrial mass in CD8⁺ T cells to mitochondrial mass in CD4⁺ T cells (Mito mass CD8/CD4)], PGC-1α and PGC-1β expression in CD8⁺ T cells (PGC-1αβ (MF1) of CD8⁺ T cells), the frequency of PD-1 highly expressing CD8⁺ T cell population (% of PD-1^{high} among CD8⁺ T cells), the frequency of FoxP3 lowly expressing CD45RA⁺ T cell population among CD4⁺ T cells (% of FoxP3^{low} CD45RA⁺ among CD4⁺ T cells), the frequency of T-bet highly expressing T cell population among CD4⁺ T cells (% of T-bet^{high} among CD4⁺ T cells), the frequency of T-bet lowly expressing T cell population among CD4⁺ T cells (% of T-bet^{low} among CD4⁺ T cells), the frequency of T-bet expressing T cell population among CD8⁺ T cells (% of T-bet⁺ among CD8⁺ T cells) and the frequency of T-bet and EOMES expressing T cell population among CD8⁺ T cells (% of T-bet⁺ EOMES⁺ among CD8⁺ T cells).
(2) The method according to (1) above, comprising predicting and/or judging the efficacy of therapy with a PD-1 signal inhibitor-containing drug based on a criterion whether the level(s) of metabolite and/or cellular marker at the time point or the ratio of two time points as indicated in Tables 4 and 5 is(are) high or low (Changes in R relative to NR).
(3) The method according to (1) or (2) above, wherein the metabolite of (i) is at least one metabolite selected from the group consisting of hippuric acid, arabinose and acylcarnitine, and the cellular marker of (ii) is at least one cellular marker selected from the group consisting of the frequency of PD-1 highly expressing CD8⁺ T cell population, the ratio of mitochondrial reactive oxygen species production in CD8⁺ T cells to mitochondrial reactive oxygen species production in CD4⁺ T cells, and PGC-1α and PGC-1β expression in CD8⁺ T cells.
(4) The method according to (1) above, wherein the therapy with the drug is predicted effective when the level of cysteine in serum and/or plasma before administration of the drug is high and the level of hippuric acid in serum and/or plasma before administration of the drug is high.
(5) The method according to (1) above, wherein the therapy with the drug is judged effective when the level of arabinose in serum and/or plasma after 1^{st} administration of the drug is high; the level of arginine in serum and/or plasma after 1^{st} administration of the drug is high; and the level of butyrylcarnitine in serum and/or plasma after 1^{st} administration of the drug is low.
(6) The method according to (1) above, wherein the therapy with the drug is judged effective when the level of hippuric acid in serum and/or plasma before administration of the drug is high; the level of cystine in serum and/or plasma after 1^{st} administration of the drug is high; the level of glutathione disulfide in serum and/or plasma after 2^{nd} administration of the drug is high; and the level of butyrylcarnitine in serum and/or plasma after 2^{nd} administration of the drug is low.
(7) The method according to any one of (1) to (6) above, wherein the therapy with the drug is predicted effective when the frequency of PD-1 highly expressing CD8⁺ T cell population in peripheral blood before administration of the drug is low and the ratio of mitochondrial reactive oxygen species production in CD8⁺ T cells to mitochondrial reactive oxygen species production in CD4⁺ T cells in peripheral blood before administration of the drug is high.
(8) The method according to any one of (1) to (6) above, wherein the therapy with the drug is judged effective when the frequency of PD-1 highly expressing CD8⁺ T cell population in peripheral blood before administration of the drug is low; the ratio of mitochondrial reactive oxygen species production in CD8⁺ T cells to mitochondrial reactive oxygen species production in CD4⁺ T cells in peripheral blood before administration of the drug is high; the ratio of PGC-1α and PGC-1β expression in CD8⁺ T cells in peripheral blood after 1^{st} administration of the drug to the corresponding expression before administration of the drug is low; and the ratio of the frequency of CD4⁺ T cells in PBMC after 1^{st} administration of the drug to the corresponding frequency before administration of the drug is high.
(9) The method according to any one of (1) to (6) above, wherein the therapy with the drug is judged effective when the frequency of PD-1 highly expressing CD8⁺ T cell population in peripheral blood before administration of the drug is low; the ratio of mitochondrial reactive oxygen species production in CD8⁺ T cells to mitochondrial reactive oxygen species production in CD4⁺ T cells in peripheral blood before administration of the drug is high; the ratio of PGC-1α and PGC-1β expression in CD8⁺ T cells in peripheral blood after 2^{nd} administration of the drug to the corresponding expression after 1^{st} administration of the drug is high; and the ratio of the frequency of CD4⁺ T cells in PBMC after 1^{st} administration of the drug to the corresponding frequency before administration of the drug is high.
(10) The method according to any one of (1) to (9) above, wherein the PD-1 signal inhibitor is an antibody.
(11) The method according to (10) above, wherein the antibody is at least one antibody selected from the group consisting of anti-PD-1 antibody, anti-PD-L1 antibody and anti-PD-L2 antibody.
(12) The method according to any one of (1) to (11) above, wherein the PD-1 signal inhibitor-containing drug is used as an active ingredient of an anticancer agent, an anti-infective agent or a combination thereof.
(13) A method of diagnosis and therapy for a disease, comprising predicting and/or judging the efficacy of therapy with a PD-1 signal inhibitor-containing drug in a subject using the following (i) and/or (ii) as indicator(s), and administering to the subject in a therapeutically effective amount when the therapy with the PD-1 signal inhibitor-containing drug has been predicted and/or judged effective:
   (i) at least one metabolite in serum and/or plasma selected from the group consisting of alanine, 4-cresol, cysteine, hippuric acid, oleic acid, indoxyl sulfate, ribose, indoleacetate, uric acid, trans-urocanic acid, pipecolic acid, N-acetylglucosamine, indolelactic acid, arabinose, arabitol, cystine, indoxyl sulfate, gluconic acid, citrulline, creatinine, N-acetylaspartic acid, pyroglutamic acid, trimethyllysine, asy-dimethylarginine, sym-dimethylarginine, methylhistidine, acylcarnitine, 3-aminoisobutyric acid, acetylcarnosine, arginine, N-acetylornitine, 3-hydroxyisovaleric acid, pyruvic acid, α-ketoglutaric acid, GSSG, 2-hydrobutyric acid, 1,5-anhydro-D-sorbitol, glutamine, glycine, lysine, taurine, AMP, acetylcarnosine, 3-hydroxybutyric acid, 2-hydroxyisovaleric acid, acetoacetic acid, tryptophan, 2-hydroxyglutaric acid, malic acid, quinolinic acid, caproic acid, isoleucine, GSH and 3-OH-kynurenine
   (ii) at least one cellular marker in peripheral blood selected from the group consisting of the frequency of CD4⁺ T cells among peripheral blood mononuclear cells (% of CD4⁺ T cells among PBMC), the frequency of CD8⁺ T cells among peripheral blood mononuclear cells (% of CD8⁺ T cells among PBMC), the frequency of naive T cells among CD8⁺ T cells (% of Tnaive among CD8⁺ T cells), the frequency of central memory T cells among CD4⁺ T cells (% of Tcm among CD4⁺ T cells), the frequency of central memory T cells among CD8⁺ T cells (% of Tcm among CD8⁺ T cells), the frequency of effector memory T cells among CD8⁺ T cells (% of Tem among CD8⁺ T cells), the frequency of terminally differentiated effector memory T cells among CD4⁺ T cells (% of Temra among CD4⁺ T cells), the frequency of terminally differentiated effector memory T cells among CD8⁺ T cells (% of Temra among CD8⁺ T cells), the ratio of mitochondrial activation status in CD8⁺ T cells to mitochondrial activation status in CD4⁺ T cells [e.g., the ratio of mitochondrial reactive oxygen species production in CD8⁺ T cells to mitochondrial reactive oxygen species production in CD4⁺ T cells (Mito SOX CD8/CD4), the ratio of mitochondrial mass in CD8⁺ T cells to mitochondrial mass in CD4⁺ T cells (Mito mass CD8/CD4)], PGC-1α and PGC-1β expression in CD8⁺ T cells (PGC-1αβ (MF1) of CD8⁺ T cells), the frequency of PD-1 highly expressing CD8⁺ T cell population (% of PD-1^{high} among CD8⁺ T cells), the frequency of FoxP3 lowly expressing CD45RA⁺ T cell population among CD4⁺ T cells (% of FoxP3^{low} CD45RA⁺ among CD4⁺ T cells), the frequency of T-bet highly expressing T cell population among CD4⁺ T cells (% of T-bet^{high} among CD4⁺ T cells), the frequency of T-bet lowly expressing T cell population among CD4⁺ T cells (% of T-bet^{low} among CD4⁺ T cells), the frequency of T-bet expressing T cell population among CD8⁺ T cells (% of T-bet⁺ among CD8⁺ T cells) and the frequency of T-bet and EOMES expressing T cell population among CD8⁺ T cells (% of T-bet⁺ EOMES⁺ among CD8⁺ T cells).
(14) A pharmaceutical composition comprising a PD-1 signal inhibitor-containing drug as an active ingredient, which is for use in a method of diagnosis and therapy for a disease, comprising predicting and/or judging the efficacy of therapy with the PD-1 signal inhibitor-containing drug in a subject using the following (i) and/or (ii) as indicator(s), and administering to the subject in a therapeutically effective amount when the therapy with the PD-1 signal inhibitor-containing drug has been predicted and/or judged effective:
   (i) at least one metabolite in serum and/or plasma selected from the group consisting of alanine, 4-cresol, cysteine, hippuric acid, oleic acid, indoxyl sulfate, ribose, indoleacetate, uric acid, trans-urocanic acid, pipecolic acid, N-acetylglucosamine, indolelactic acid, arabinose, arabitol, cystine, indoxyl sulfate, gluconic acid, citrulline, creatinine, N-acetylaspartic acid, pyroglutamic acid, trimethyllysine, asy-dimethylarginine, sym-dimethylarginine, methylhistidine, acylcarnitine, 3-aminoisobutyric acid, acetylcarnosine, arginine, N-acetylornitine, 3-hydroxyisovaleric acid, pyruvic acid, α-ketoglutaric acid, GSSG, 2-hydrobutyric acid, 1,5-anhydro-D-sorbitol, glutamine, glycine, lysine, taurine, AMP, acetylcarnosine, 3-hydroxybutyric acid, 2-hydroxyisovaleric acid, acetoacetic acid, tryptophan, 2-hydroxyglutaric acid, malic acid, quinolinic acid, caproic acid, isoleucine, GSH and 3-OH-kynurenine
   (ii) at least one cellular marker in peripheral blood selected from the group consisting of the frequency of CD4⁺ T cells among peripheral blood mononuclear cells (% of CD4⁺ T cells among PBMC), the frequency of CD8⁺ T cells among peripheral blood mononuclear cells (% of CD8⁺ T cells among PBMC), the frequency of naive T cells among CD8⁺ T cells (% of Tnaive among CD8⁺ T cells), the frequency of central memory T cells among CD4⁺ T cells (% of Tcm among CD4⁺ T cells), the frequency of central memory T cells among CD8⁺ T cells (% of Tcm among CD8⁺ T cells), the frequency of effector memory T cells among CD8⁺ T cells (% of Tem among CD8⁺ T cells), the frequency of terminally differentiated effector memory T cells among CD4⁺ T cells (% of Temra among CD4⁺ T cells), the frequency of terminally differentiated effector memory T cells among CD8⁺ T cells (% of Temra among CD8⁺ T cells), the ratio of mitochondrial activation status in CD8⁺ T cells to mitochondrial activation status in CD4⁺ T cells [e.g., the ratio of mitochondrial reactive oxygen species production in CD8⁺ T cells to mitochondrial reactive oxygen species production in CD4⁺ T cells (Mito SOX CD8/CD4), the ratio of mitochondrial mass in CD8⁺ T cells to mitochondrial mass in CD4⁺ T cells (Mito mass CD8/CD4)], PGC-1α and PGC-1β expression in CD8⁺ T cells (PGC-1αβ (MF1) of CD8⁺ T cells), the frequency of PD-1 highly expressing CD8⁺ T cell population (% of PD-1^{high} among CD8⁺ T cells), the frequency of FoxP3 lowly expressing CD45RA⁺ T cell population among CD4⁺ T cells (% of FoxP3^{low} CD45RA⁺ among CD4⁺ T cells), the frequency of T-bet highly expressing T cell population among CD4⁺ T cells (% of T-bet^{high} among CD4⁺ T cells), the frequency of T-bet lowly expressing T cell population among CD4⁺ T cells (% of T-bet^{low} among CD4⁺ T cells), the frequency of T-bet expressing T cell population among CD8⁺ T cells (% of T-bet⁺ among CD8⁺ T cells) and the frequency of T-bet and EOMES expressing T cell population among CD8⁺ T cells (% of T-bet⁺ EOMES⁺ among CD8⁺ T cells).

### EFFECT OF THE INVENTION

Since the therapy using anti-PD-1 antibody, a representative of PD-1 signal inhibitors, is very expensive, judging the efficacy prior to or at an early stage of such therapy will lead to reduction of the cost of the treatment.

The present specification encompasses the contents disclosed in the specification and/or drawings of Japanese Patent Application No. 2019-000181 based on which the present patent application claims priority.

### BRIEF DESCRIPTION OF THE DRAWINGS

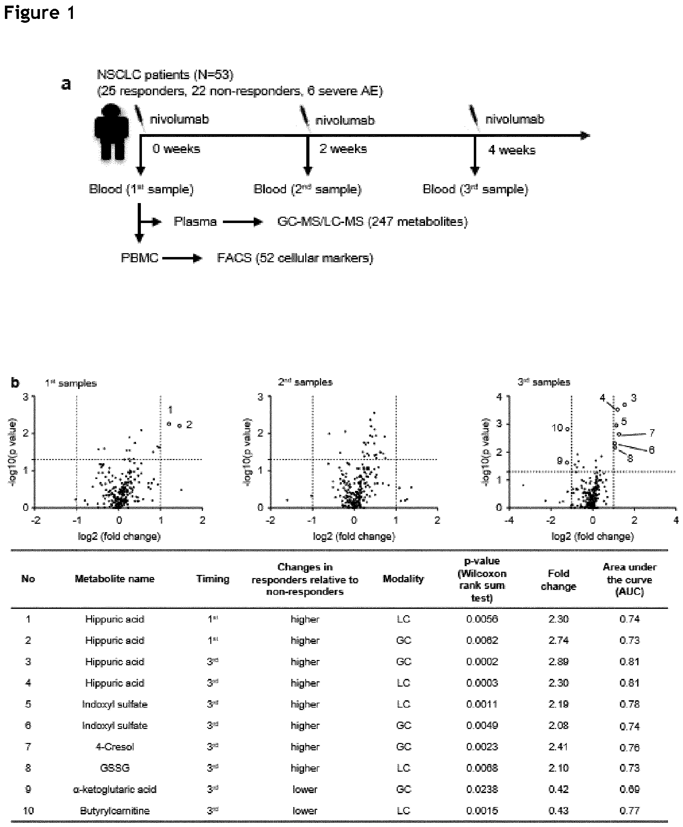

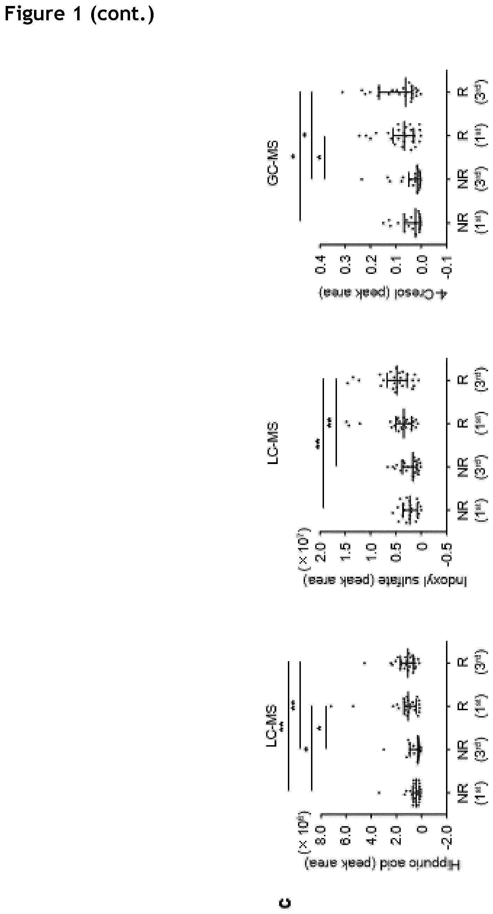

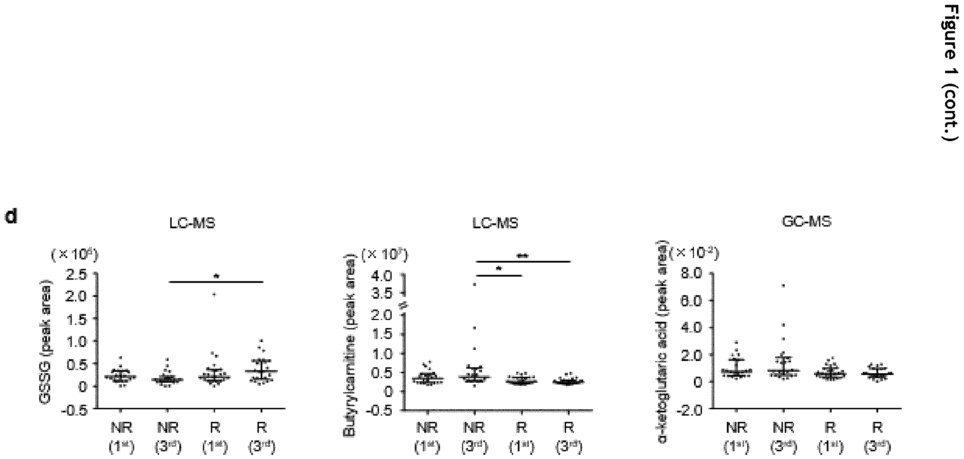

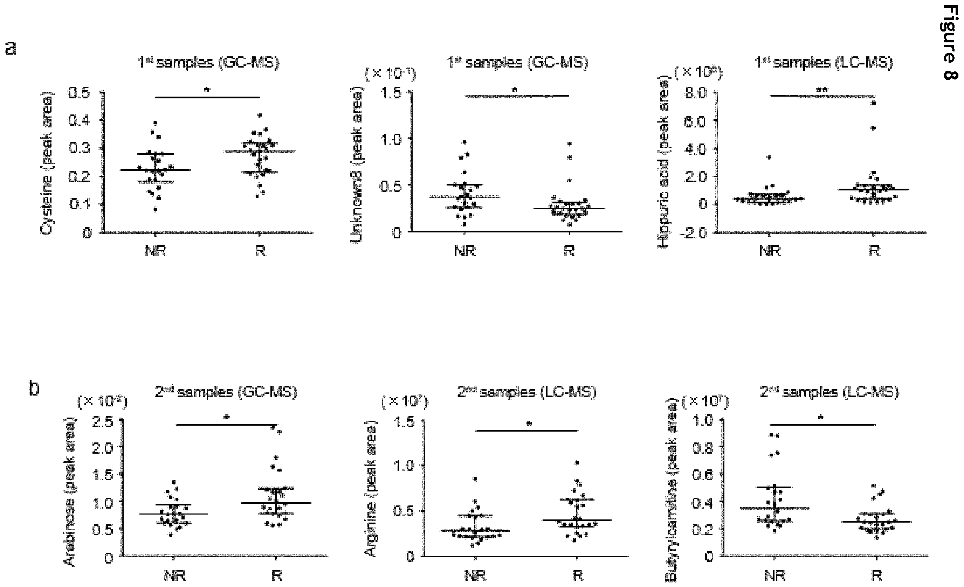

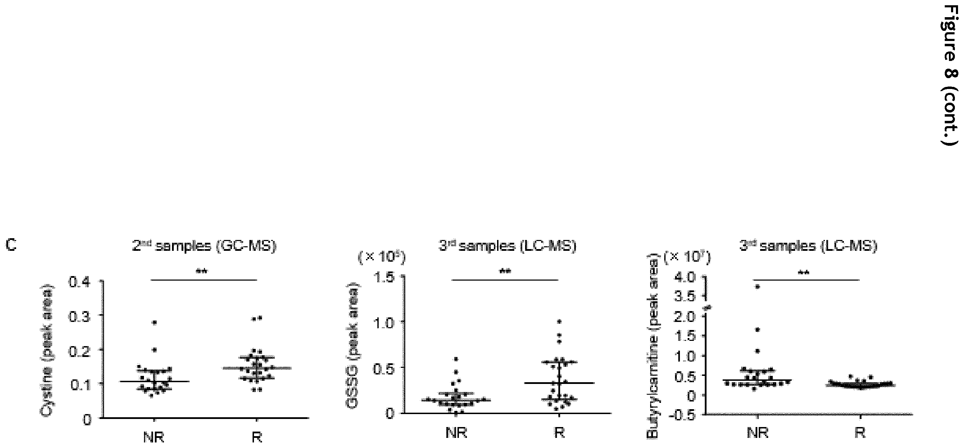

[Fig. 1] Particular plasma metabolites are associated with responsiveness to Nivolumab treatment.
   a) A schematic diagram of this study. b) Comparison of 247 metabolites between non-responders and responders at each time point was summarized in volcano plots. Metabolites with log2 |fold change|>1.0 and -log10 (p-value) >1.3 were considered significant. Metabolites showing significant differences between responders and non-responders are listed in table. Modality indicates the analytical platform (GC-MS or LC-MS) used for metabolite measurement. The area under the curve (AUC) of each metabolite in relation to responsiveness was calculated from the univariate logistic regression analysis and is shown in the rightmost column of the table. c) The peak areas measured by GC- or LC-MS of each gut microbiota-related metabolite in non-responders (NR) and responders (R). d) The peak areas of redox/energy metabolism-related metabolites. Each dot represents one patient. Error bars show median and interquartile range. ^{∗}p < 0.05, ^{∗∗}p < 0.01 by Kruskal-Wallis test followed by Dunn's multiple comparisons test (c and d).

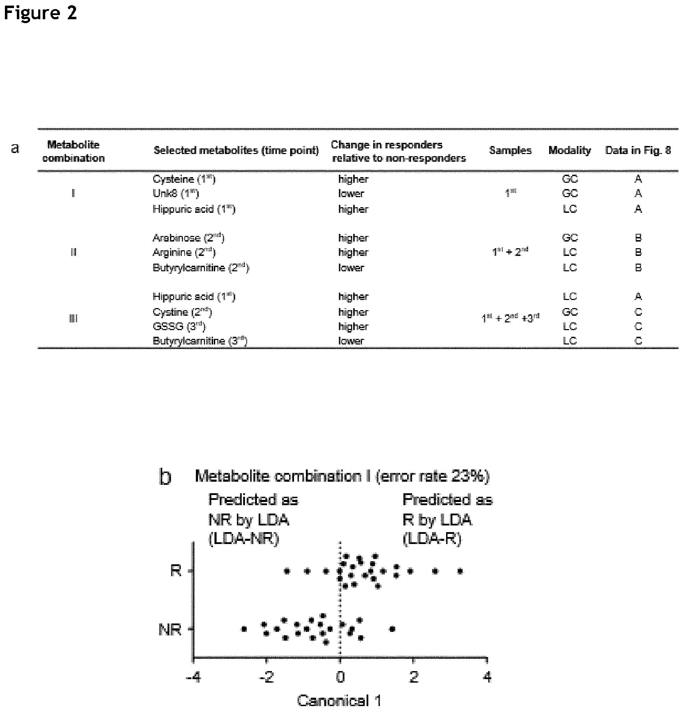

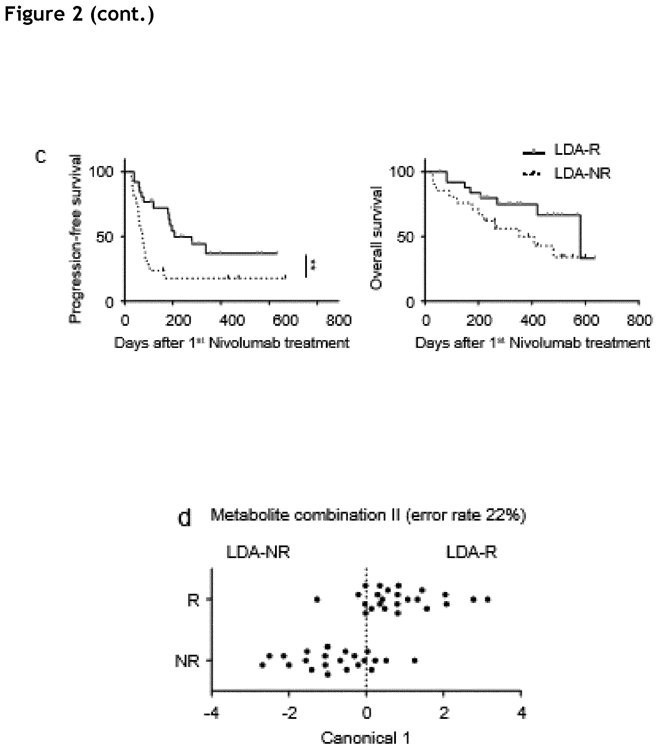

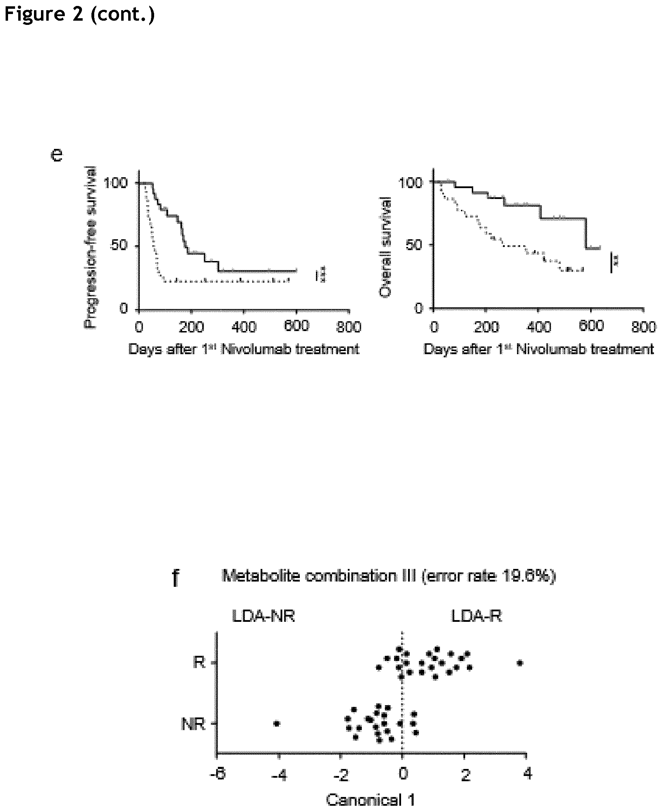

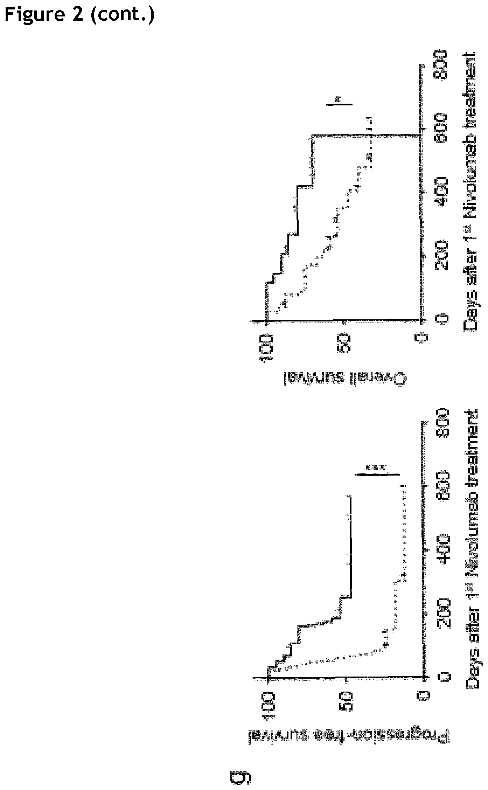

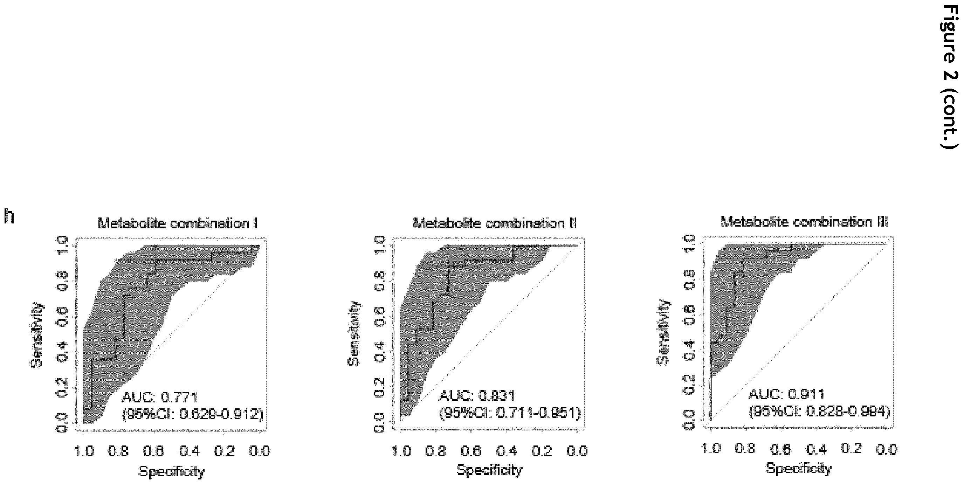

[Fig. 2] A combination of plasma metabolites predicts responsiveness to Nivolumab treatment.
   a) The stepwise Akaike's information criterion (AIC) regression procedure selected the best predictive combinations of metabolites I (in the 1^{st} sample alone), II (in the 1^{st} and 2^{nd} samples) and III (in the 1^{st}, 2^{nd} and 3^{rd} samples). Detailed data on each metabolite are shown in Fig. 8. b) Linear discriminant analysis (LDA) was used to evaluate the accuracy of metabolite combination I as a predictive biomarker. Canonical plot of LDA for determination of LDA-R and LDA-NR is shown. Each dot represents one patient. The vertical dotted line indicates the cut-off value. c) Kaplan-Meier plots of Progression Free Survival (PFS) and Overall Survival (OS) of LDA-R and LDA-NR determined by metabolite combination I. d) Canonical plot of LDA based on metabolite combination II. e) Kaplan-Meier plots of PFS and OS of LDA-R and LDA-NR determined by metabolite combination II. f) Canonical plot for LDA based on metabolite combination III. g) Kaplan-Meier plots of PFS and OS of patients LDA-R and LDA-NR determined by metabolite combination III. ^{∗}p < 0.05, ^{∗∗}p < 0.01, ^{∗∗∗}p < 0.001 by Gehan-Breslow-Wilcoxon test (c, e, and g). h) The ROC curve of five-fold cross validation for metabolite combinations I, II and III.

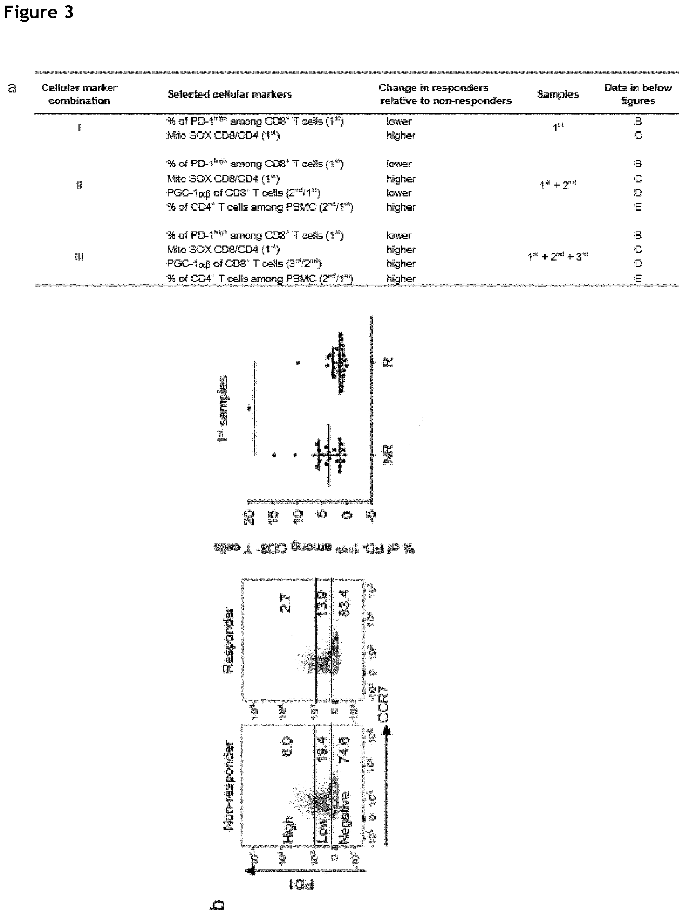

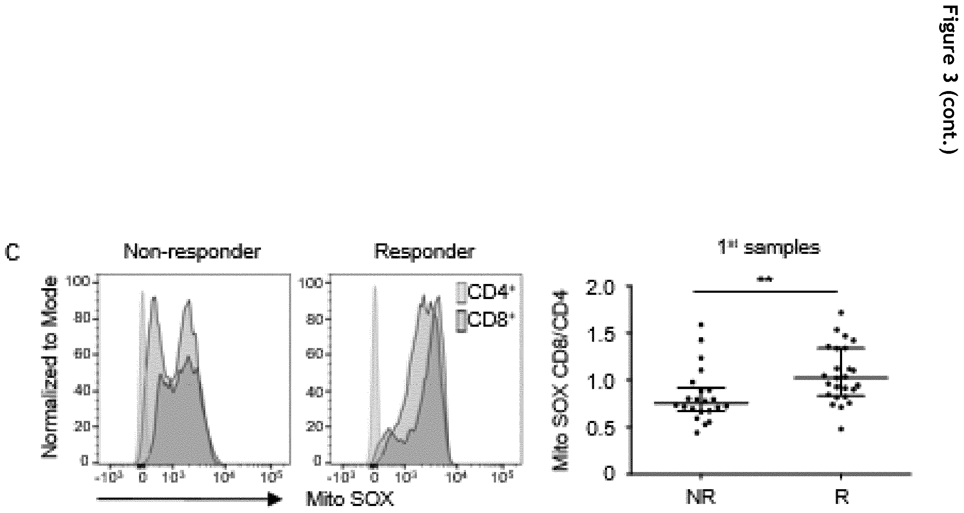

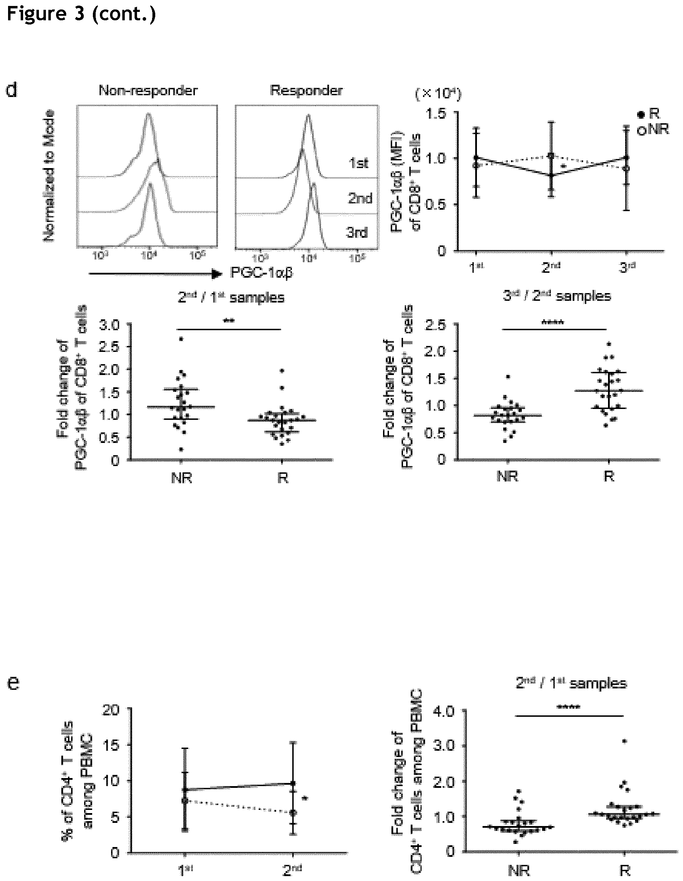

[Fig. 3] Particular cellular markers including mitochondrial status were selected to make up a combinatorial predictive marker.
   a) A stepwise AIC regression procedure selected the best predictive combinations of cellular markers I (in the 1^{st} sample alone), II (in the 1^{st} and 2^{nd} samples) and III (in the 1^{st}, 2^{nd} and 3^{rd} samples). b) Two representative NSCLC samples (non-responder and responder) after gating on CD8⁺ PBMC (left). X axis represents CCR7 and Y axis PD-1. Frequency of PD-1^{high} CD8⁺ T cells in non-responders and responders in 1st samples (right). c) Representative histograms of Mito SOX on gated CD4⁺ and CD8⁺ T cells (left). Ratio of Mito SOX levels in CD8⁺ and CD4⁺ T cells (Mito SOX CD8/CD4) for non-responders and responders in the 1^{st} samples (right). d) PGC-1αβ of the 1^{st}, 2^{nd} and 3^{rd} samples among CD⁸⁺ PBMC (upper left). Change in MFI of PGC-1αβ between the 1^{st}, 2^{nd} and 3^{rd} samples (upper right). The solid line and the dotted line represent responders and non-responders, respectively. Fold change of PGC-1αβ expression between non-responders and responders in the 2^{nd} relative to 1^{st} samples (lower left) and in the 3^{rd} relative to 2^{nd} samples (lower right). e) Frequency of CD4⁺ T cells among PBMC in the 1^{st} and 2^{nd} samples (left). The solid line and the dotted line represent responders and non-responders, respectively. Fold change of CD4⁺ T cell frequency in the 2^{nd} relative to 1^{st} samples between non-responders and responders (right). Each dot represents one patient. Error bars show median and interquartile range. ^{∗}p < 0.05, ^{∗∗}p < 0.01, ^{∗∗∗∗}p < 0.0001 by Wilcoxon rank sum test.

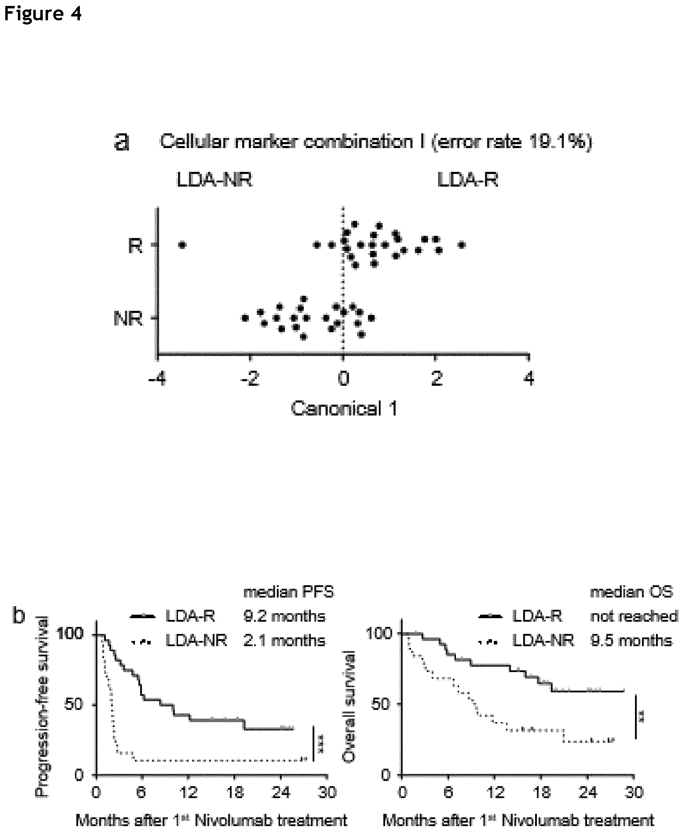

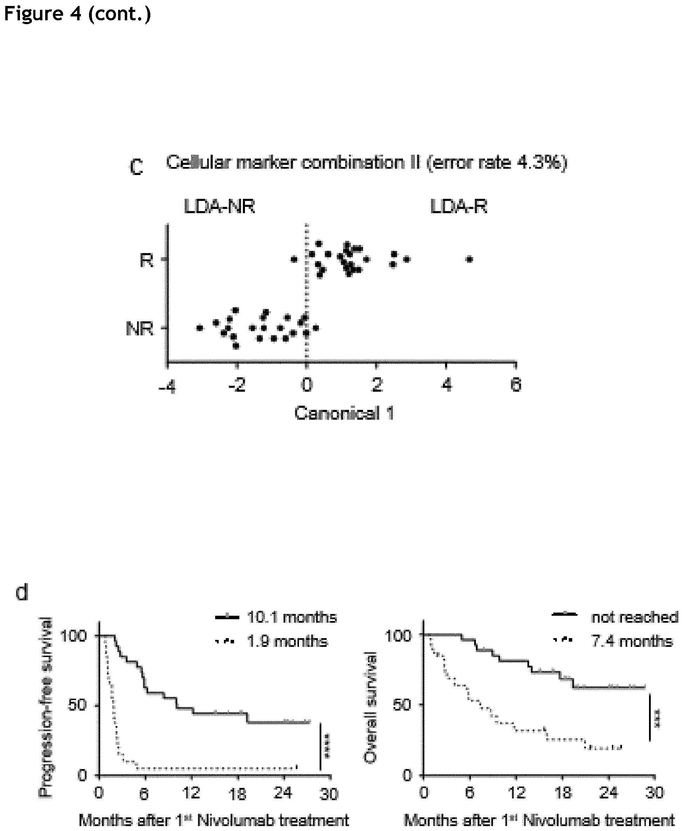

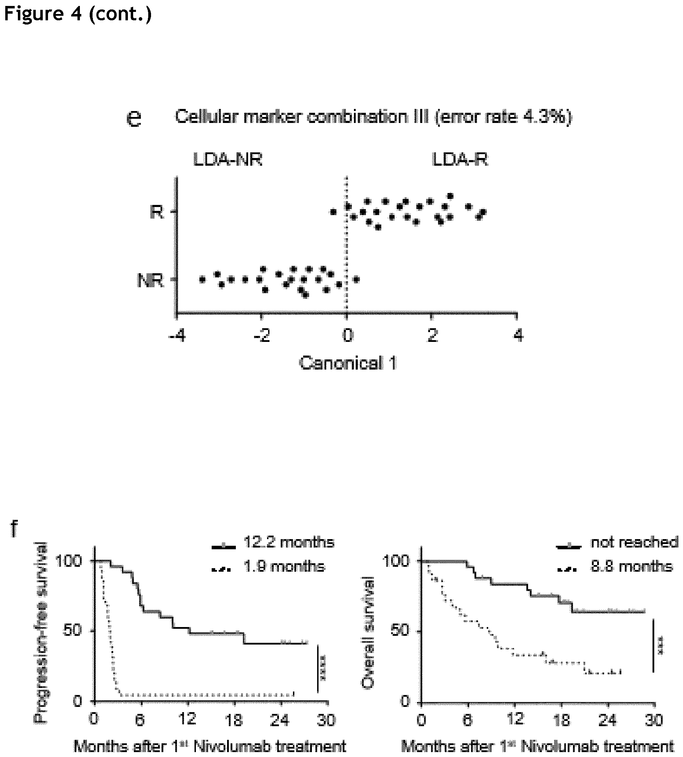

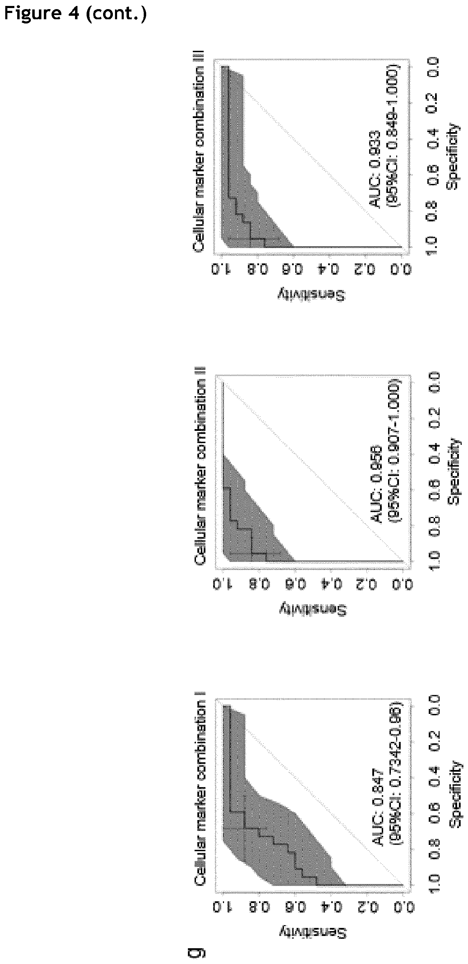

[Fig. 4] A combination of cellular markers could predict survival more precisely. a) LDA evaluated the accuracy of cellular marker combination I. Canonical plot of LDA for determination of LDA-R and LDA-NR is shown. Each dot represents one patient. The vertical dotted line indicates the cut-off value. b) Kaplan-Meier plots of PFS and OS of LDA-R and LDA-NR determined by cellular marker combination I. c) Canonical plot for LDA based on cellular marker combination II. d) Kaplan-Meier plots of PFS and OS of LDA-R and LDA-NR determined by the cellular marker combination II. e) Canonical plot for LDA based on cellular marker combination III. f) Kaplan-Meier plots of PFS and OS of LDA-R and LDA-NR determined by cellular marker combination III. ^{∗}p < 0.05, ^{∗∗}p < 0.01, ^{∗∗∗}p < 0.001 by Gehan-Breslow-Wilcoxon test (b, d, and f). g) The ROC curve of five-fold cross validation for cellular marker combinations I, II and III.

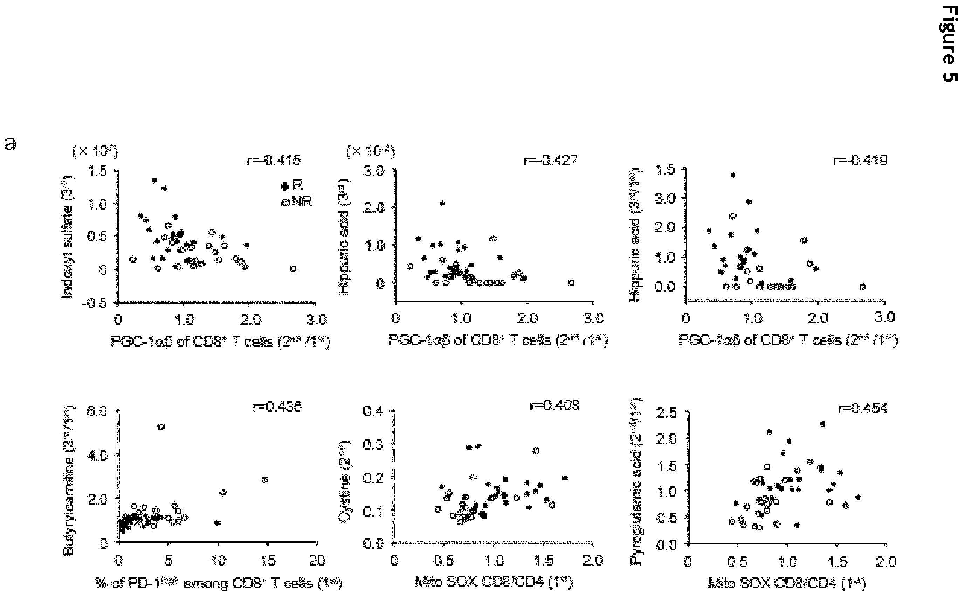

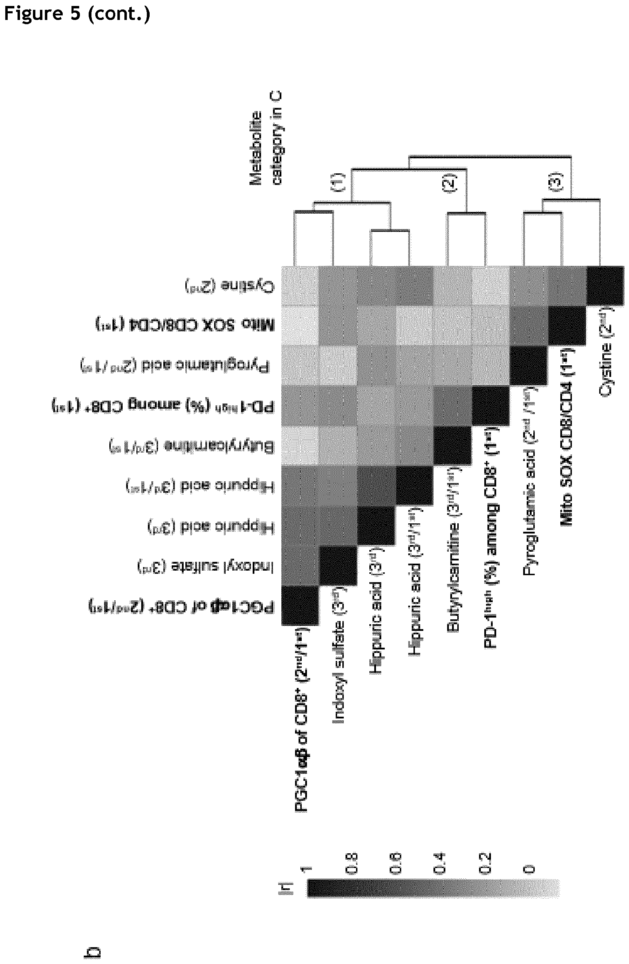

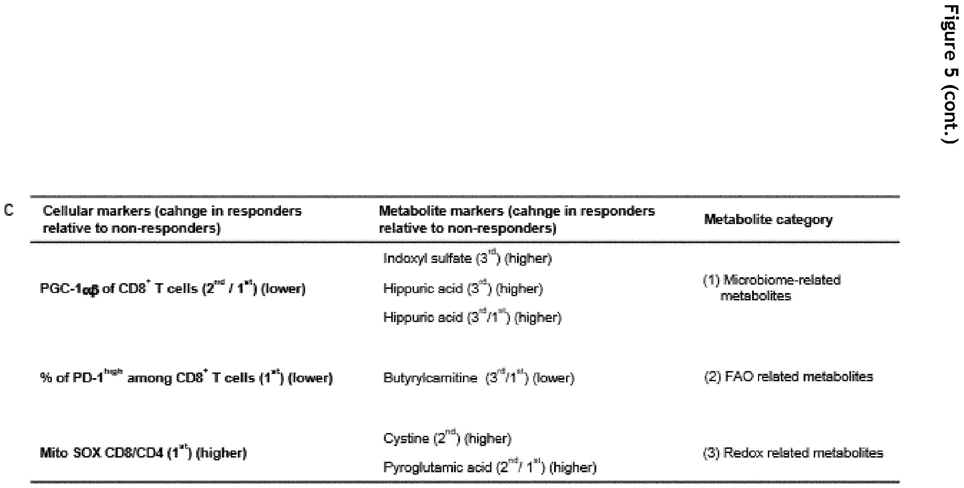

[Fig. 5] Strong correlation between particular cellular and metabolite markers excludes metabolite makers from candidates for combinatorial biomarkers.
   a) Scatter plots between cellular markers (X-axis) and metabolite markers (Y-axis). Filled circles represent responders and open circles non-responders. r: Spearman correlation coefficients. b) A clustered heatmap of absolute correlation coefficients over all marker pairs detected in a) above (using Spearman correlation distance, and complete linkage). Dark denotes higher correlation (|r| close to 1) and light lower correlation (|r| close to 0). The markers clustered into 3 groups, which were designated as metabolic categories 1, 2 and 3. c) Summary of the Spearman correlation analysis. Metabolic category 1 (gut microbiota-related metabolites) is correlated with PGC-1αβ of CD8⁺ T cells; metabolic category 2 (FAO-related metabolites) is correlated with the frequency of PD-1 ^{high} CD8⁺ T cells; and metabolic category 3 (redox-related metabolites) is correlated with the T cell Mito SOX marker.

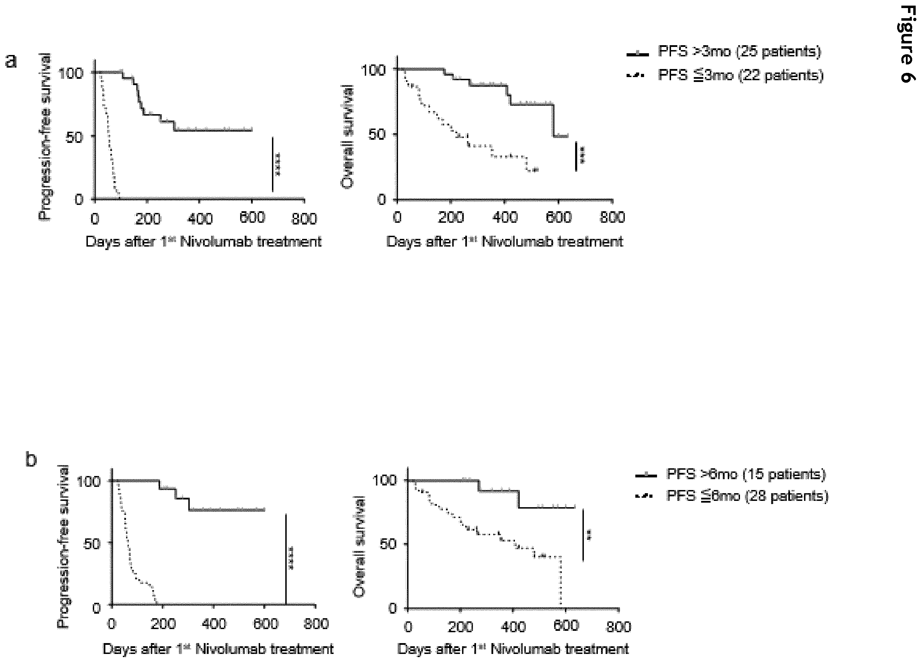

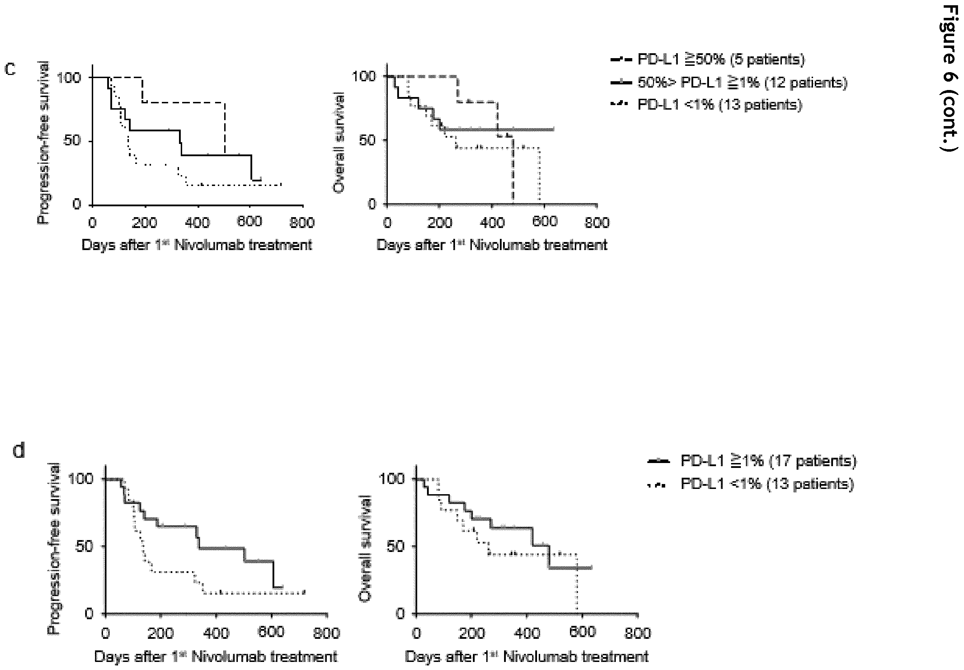

[Fig. 6] Survival rates are compared between sorted groups based on different criteria.
   a) Kaplan-Meier plots of PFS and OS of patients sorted by the criteria of PFS > 3 months (solid line) and PFS ≤ 3 months (dotted line). b) Kaplan-Meier plots of PFS and OS of patients sorted by the criteria of PFS > 6 months (solid line) and PFS ≤ 6 months (dotted line). ^{∗∗}p < 0.01, ^{∗∗∗}p < 0.001, ^{∗∗∗∗}p < 0.0001 by Gehan-Breslow-Wilcoxon test. c) Kaplan-Meier plots of PFS and OS of patients sorted by frequency of PD-L1 expression on tumors. The dashed line, solid line, and dotted line show patients with high PD-L1 expression (> 50%), low PD-L1 expression (1-50%), and rare PD-L1 expression (< 1%), respectively. d) The solid line and dotted line show patients with positive expression (> 1%) and negative expression (< 1%) of PD-L1, respectively.

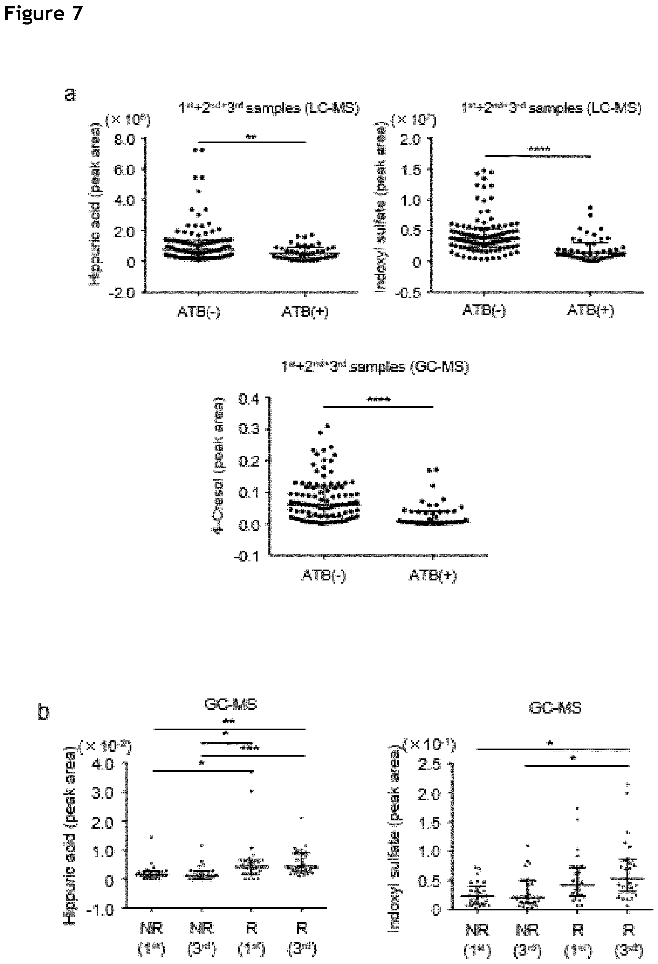

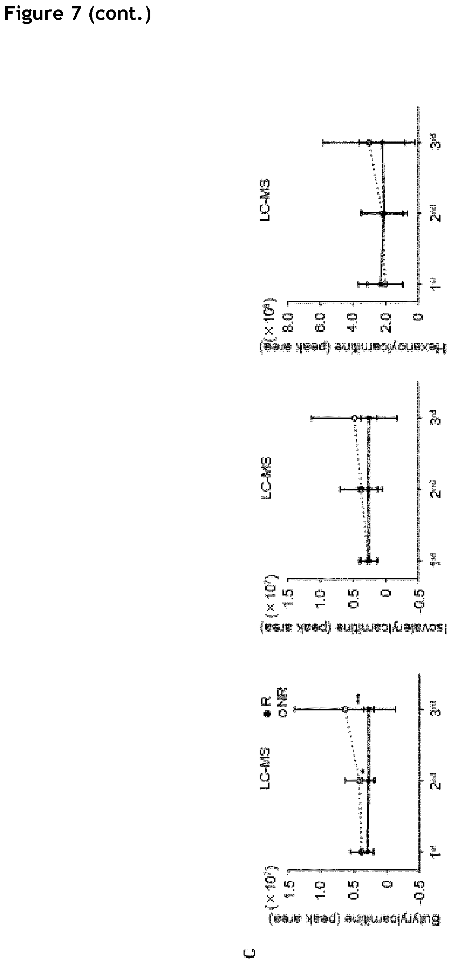

[Fig. 7] Behaviors of gut microbiota-derived metabolites and acylcarnitine species. a) The peak area measured by GC- or LC-MS of each microbiota related metabolite in patients without pre-antibiotics treatment (ATB(-)) and with pre-antibiotics treatment (ATB(+)) are shown. These graphs display the data of the 1^{st} + 2^{nd} + 3^{rd} samples. ^{∗∗}p < 0.01, ^{∗∗∗∗}p < 0.0001 by Wilcoxon rank sum test. b) The peak areas measured by GC-MS of hippuric acid and indoxyl sulfate in non-responders (NR) and responders (R) are shown. Each dot represents one patient. Error bars show median and interquartile range. ^{∗}p < 0.05, ^{∗∗}p < 0.01, ^{∗∗∗}p < 0.001 by Kruskal-Wallis test followed by Dunn's multiple comparisons test. c) The peak areas of acylcarnitine species between 1^{st}, 2^{nd} and 3^{rd} samples are shown. The solid line and dotted line represent responders and non-responders, respectively. ^{∗}p < 0.05, ^{∗∗}p < 0.01 by Wilcoxon rank sum test.
[Fig. 8] Detailed data of metabolite markers selected by stepwise regression analysis.
   a―c) Graphs show comparison of peak areas of metabolite markers selected by stepwise regression analysis between non-responders and responders. Each dot represents one patient. Error bars indicate the median and interquartile range. ^{∗}p < 0.05, ^{∗∗}p < 0.01 by Wilcoxon rank sum test.
[Fig. 9] Detailed phenotypes of cellular marker-positive subsets in CD8⁺ and CD4⁺ T cells.
   a) Graph shows comparison of PD-1 positive frequency among CD8⁺ T cells in non-responders and responders in the 1^{st} samples. b) FACS data show the expression levels of PD-1, Ki-67, Granzyme B, IFN-γ, T-bet and EOMES among CD8⁺ T cells in PBMC. Representative NSCLC samples are depicted in left panels. Right panels show the frequencies of Ki67⁺, Granzyme B⁺, IFN-γ⁺, T-bet⁺ and EOMES⁺ cells in PD-1^{hi}, PD-1^{low}, and PD-1(-) CD8⁺ T cells. ^{∗}p < 0.05, ^{∗∗}p < 0.01, ^{∗∗∗}p < 0.001, ^{∗∗∗∗}p < 0.0001 by Kruskal-Wallis test followed by Dunn's multiple comparisons test. c) CD4⁺ T cells were sorted into naive, Tcm, Tem and Temra subsets according to the expression of CD45RO and CCR7 (left panel). The frequencies of CD4⁺ Tcm and CD4⁺ Temra in non-responders and responders are shown (right panels). ^{∗∗}p < 0.01 by Wilcoxon rank sum test.

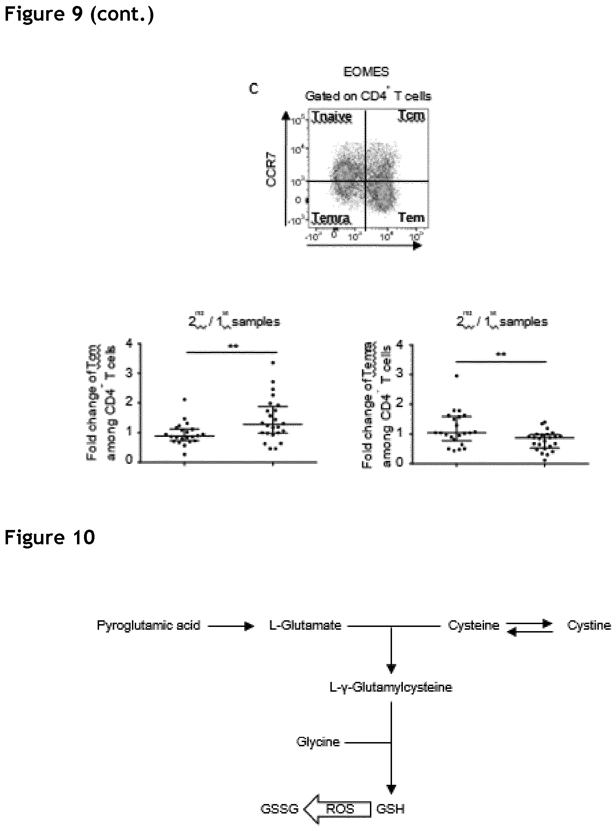

[Fig. 10] Schema showing biosynthesis and metabolism of glutathione. L-Glutamate and cysteine are bound to form L-γ-glutamylcysteine, which then binds to glycine to yield GSH. GSH is oxidized into its oxidized form (GSSG) after reaction with reactive oxygen species (ROS).

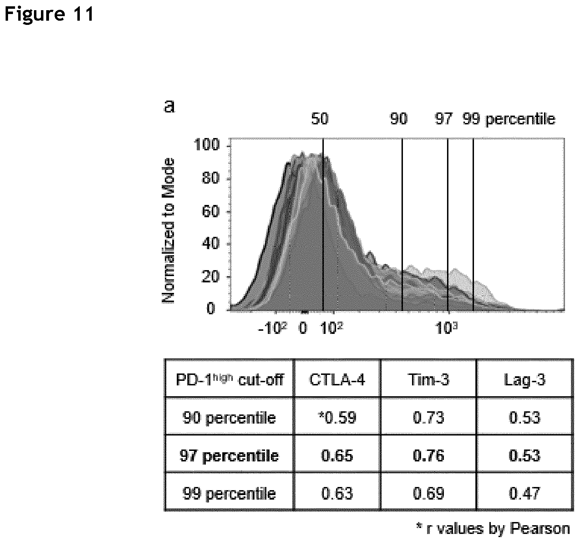

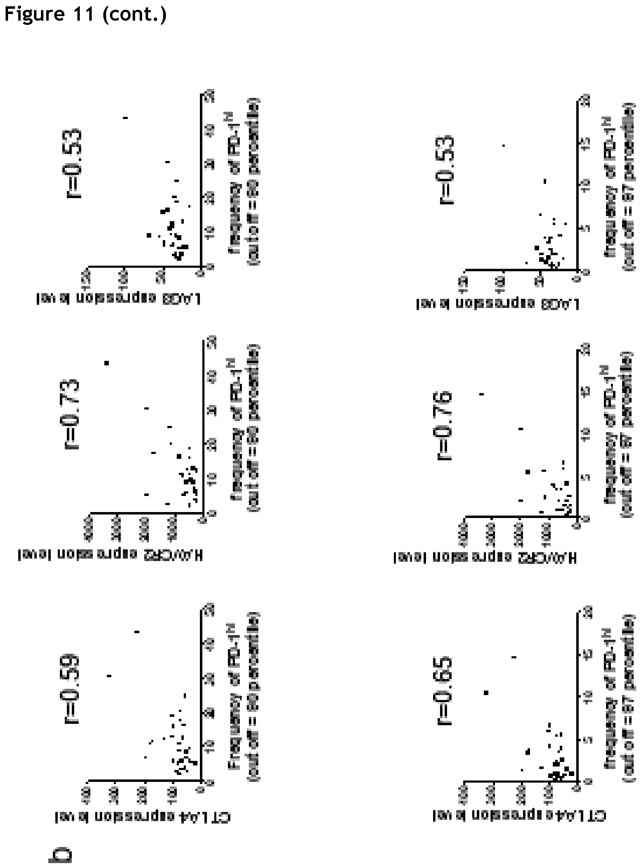

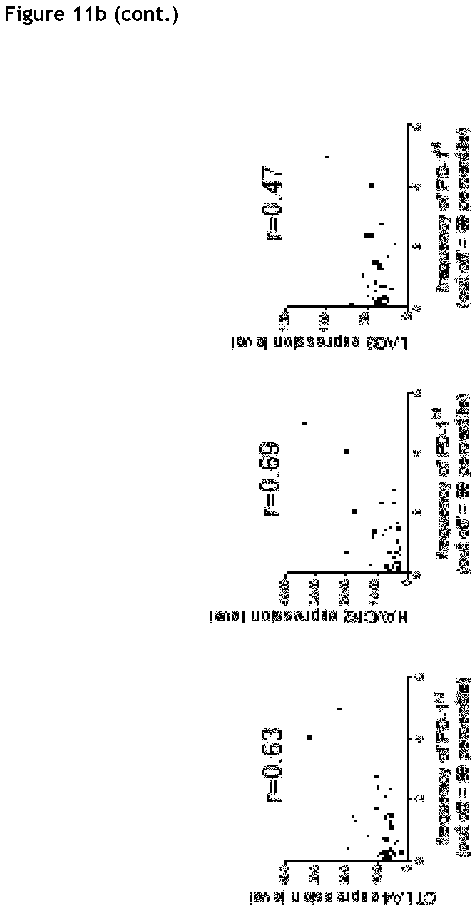

[Fig. 11] Definition of PD-1^{high}. A) CD8⁺ T cells from age matched 30 healthy donors were gated and PD-1 staining data were overlaid. X axis represents PD-1 expression level and Y axis relative cell count. Vertical lines indicate the 50^{th}, 90^{th}, 97^{th} and 99^{th} percentiles of PD-1 expression average. The value of correlation coefficient (r) between the frequency (%) of PD-1^{high} CD8⁺ T cells and the gene expression of exhaustion markers (CTLA-4, Tim-3 and Lag-3) of CD8⁺ T cells in patients at each percentile is shown in the table. B) Right panel shows correlation diagrams. At 97^{th} percentile, correlation was high with expression of any of the exhaustion marker genes. Therefore, 97^{th} percentile was determined as the PD-1^{high} cut-off value.

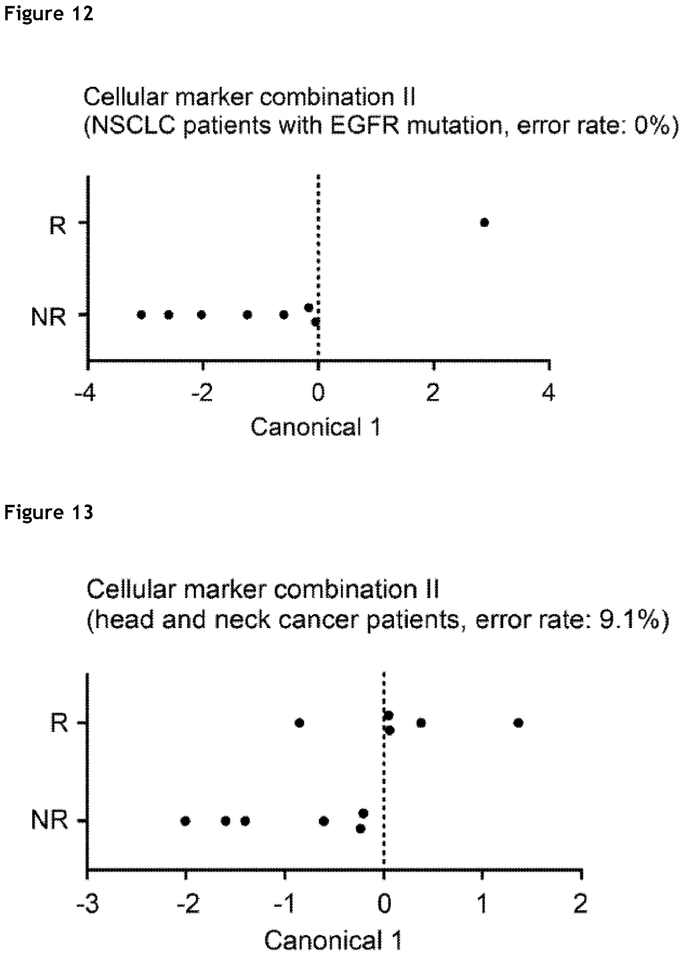

[Fig. 12] It is a known fact that PD-1 antibody therapy does not work well when lung cancer cells have an EGFR mutation. LDA analysis was performed on whether the cellular marker combination II of the present invention can discriminate the non-responsiveness resulting from the presence of EGFR mutation. As a result, the error rate was 0%. It has become clear that the cellular marker combination II is capable of discriminating the non-responsiveness due to EGFR mutation.
[Fig. 13] In order to examine whether the cellular marker combination II can also judge therapeutic efficacy in other cancer species, samples from 11 head & neck cancer patients were stained in the same manner and subjected to LDA analysis. As a result, efficacy could be judged at an error rate of 9.1%.

### BEST MODES FOR CARRYING OUT THE INVENTION

Hereinbelow, the present invention will be described in detail.

The present invention provides a test method comprising predicting and/or judging the efficacy of therapy with a PD-1 signal inhibitor-containing drug using the following (i) and/or (ii) as indicator(s):
(i) at least one metabolite in serum and/or plasma selected from the group consisting of alanine, 4-cresol, cysteine, hippuric acid, oleic acid, indoxyl sulfate, ribose, indoleacetate, uric acid, trans-urocanic acid, pipecolic acid, N-acetylglucosamine, indolelactic acid, arabinose, arabitol, cystine, indoxyl sulfate, gluconic acid, citrulline, creatinine, N-acetylaspartic acid, pyroglutamic acid, trimethyllysine, asy-dimethylarginine, sym-dimethylarginine, methylhistidine, acylcarnitine, 3-aminoisobutyric acid, acetylcarnosine, arginine, N-acetylornitine, 3-hydroxyisovaleric acid, pyruvic acid, α-ketoglutaric acid, GSSG, 2-hydrobutyric acid, 1,5-anhydro-D-sorbitol, glutamine, glycine, lysine, taurine, AMP, acetylcarnosine, 3-hydroxybutyric acid, 2-hydroxyisovaleric acid, acetoacetic acid, tryptophan, 2-hydroxyglutaric acid, malic acid, quinolinic acid, caproic acid, isoleucine, GSH and 3-OH-kynurenine
(ii) at least one cellular marker in peripheral blood selected from the group consisting of the frequency of CD4⁺ T cells among peripheral blood mononuclear cells (% of CD4⁺ T cells among PBMC), the frequency of CD8⁺ T cells among peripheral blood mononuclear cells (% of CD8⁺ T cells among PBMC), the frequency of naive T cells among CD8⁺ T cells (% of Tnaive among CD8⁺ T cells), the frequency of central memory T cells among CD4⁺ T cells (% of Tcm among CD4⁺ T cells), the frequency of central memory T cells among CD8⁺ T cells (% of Tcm among CD8⁺ T cells), the frequency of effector memory T cells among CD8⁺ T cells (% of Tem among CD8⁺ T cells), the frequency of terminally differentiated effector memory T cells among CD4⁺ T cells (% of Temra among CD4⁺ T cells), the frequency of terminally differentiated effector memory T cells among CD8⁺ T cells (% of Temra among CD8⁺ T cells), the ratio of mitochondrial activation status in CD8⁺ T cells to mitochondrial activation status in CD4⁺ T cells [e.g., the ratio of mitochondrial reactive oxygen species production in CD8⁺ T cells to mitochondrial reactive oxygen species production in CD4⁺ T cells (Mito SOX CD8/CD4), the ratio of mitochondrial mass in CD8⁺ T cells to mitochondrial mass in CD4⁺ T cells (Mito mass CD8/CD4)], PGC-1α and PGC-1β expression in CD8⁺ T cells (PGC-1αβ (MF1) of CD8⁺ T cells), the frequency of PD-1 highly expressing CD8⁺ T cell population (% of PD-1^{high} among CD8⁺ T cells), the frequency of FoxP3 lowly expressing CD45RA⁺ T cell population among CD4⁺ T cells (% of FoxP3^{low} CD45RA⁺ among CD4⁺ T cells), the frequency of T-bet highly expressing T cell population among CD4⁺ T cells (% of T-bet^{high} among CD4⁺ T cells), the frequency of T-bet lowly expressing T cell population among CD4⁺ T cells (% of T-bet^{low} among CD4⁺ T cells), the frequency of T-bet expressing T cell population among CD8⁺ T cells (% of T-bet⁺ among CD8⁺ T cells) and the frequency of T-bet and EOMES expressing T cell population among CD8⁺ T cells (% of T-bet⁺ EOMES⁺ among CD8⁺ T cells).

In one embodiment of the present invention, the therapeutic efficacy in a subject of a PD-1 signal inhibitor-containing drug may be predicted and/or judged from the measured values of the patient based on a criterion whether the level(s) of metabolite and/or cellular marker at the time point or the ratio of two time points as indicated in Tables 4 and 5 (Example 1 described later) is(are) high or low (Changes in R relative to NR).

Examples of such criteria are described below. Each of these criteria may be used alone or in combination.

### Table 4 (Metabolites Detected in Serum and/or Plasma)

### (Before treatment (drug administration) (Time point: 1st))

- When the level of alanine (metabolite) is high (Changes in R relative to NR: higher) before treatment (drug administration) (time point: 1st), therapy with a PD-1 signal inhibitor-containing drug is predicted effective.
- When the level of 4-cresol is high before treatment, therapy with a PD-1 signal inhibitor-containing drug is predicted effective.
- When the level of cysteine is high before treatment, therapy with a PD-1 signal inhibitor-containing drug is predicted effective.
- When the level of hippuric acid is high before treatment, therapy with a PD-1 signal inhibitor-containing drug is predicted effective.
- When the level of oleic acid is high before treatment, therapy with a PD-1 signal inhibitor-containing drug is predicted effective.
- When the level of indoxyl sulfate is high before treatment, therapy with a PD-1 signal inhibitor-containing drug is predicted effective.
- When the level of ribose is high before treatment, therapy with a PD-1 signal inhibitor-containing drug is predicted effective.
- When the level of indoleacetate is high before treatment, therapy with a PD-1 signal inhibitor-containing drug is predicted effective.
- When the level of uric acid is high before treatment, therapy with a PD-1 signal inhibitor-containing drug is predicted effective.
- When the level of trans-urocanic acid is high before treatment, therapy with a PD-1 signal inhibitor-containing drug is predicted effective.
- When the level of pipecolic acid is low before treatment, therapy with a PD-1 signal inhibitor-containing drug is predicted effective.
- When the level of N-acetylglucosamine is high before treatment, therapy with a PD-1 signal inhibitor-containing drug is predicted effective.

### (After 1^{st} Administration of Drug (Time point: 2nd))

- When the level of uric acid is high after 1^{st} administration of a PD-1 signal inhibitor-containing drug (Time point: 2nd), therapy with the drug is predicted and/or judged effective.
- When the level of indolelactic acid is high after 1^{st} administration of a PD-1 signal inhibitor-containing drug, therapy with the drug is predicted and/or judged effective.
- When the level of arabinose is high after 1^{st} administration of a PD-1 signal inhibitor-containing drug, therapy with the drug is predicted and/or judged effective.
- When the level of arabitol is high after 1^{st} administration of a PD-1 signal inhibitor-containing drug, therapy with the drug is predicted and/or judged effective.
- When the level of hippuric acid is high after 1^{st} administration of a PD-1 signal inhibitor-containing drug, therapy with the drug is predicted and/or judged effective.
- When the level of cystine is high after 1^{st} administration of a PD-1 signal inhibitor-containing drug, therapy with the drug is predicted and/or judged effective.
- When the level of indoxyl sulfate is high after 1^{st} administration of a PD-1 signal inhibitor-containing drug, therapy with the drug is predicted and/or judged effective.
- When the level of gluconic acid is high after 1^{st} administration of a PD-1 signal inhibitor-containing drug, therapy with the drug is predicted and/or judged effective.
- When the level of citrulline is high after 1^{st} administration of a PD-1 signal inhibitor-containing drug, therapy with the drug is predicted and/or judged effective.
- When the level of creatinine is high after 1^{st} administration of a PD-1 signal inhibitor-containing drug, therapy with the drug is predicted and/or judged effective.
- When the level of N-acetylaspartic acid is high after 1^{st} administration of a PD-1 signal inhibitor-containing drug, therapy with the drug is predicted and/or judged effective.
- When the level of pyroglutamic acid is high after 1^{st} administration of a PD-1 signal inhibitor-containing drug, therapy with the drug is predicted and/or judged effective.
- When the level of trimethyllysine is high after 1^{st} administration of a PD-1 signal inhibitor-containing drug, therapy with the drug is predicted and/or judged effective.
- When the level of asy-dimethylarginine is high after 1^{st} administration of a PD-1 signal inhibitor-containing drug, therapy with the drug is predicted and/or judged effective.
- When the level of sym-dimethylarginine is high after 1^{st} administration of a PD-1 signal inhibitor-containing drug, therapy with the drug is predicted and/or judged effective.
- When the level of pipecolic acid is low after 1^{st} administration of a PD-1 signal inhibitor-containing drug, therapy with the drug is predicted and/or judged effective.
- When the level of methylhistidine is high after 1^{st} administration of a PD-1 signal inhibitor-containing drug, therapy with the drug is predicted and/or judged effective.
- When the level of acylcarnitine (e.g., butyrylcarnitine (C4)) is low after 1^{st} administration of a PD-1 signal inhibitor-containing drug, therapy with the drug is predicted and/or judged effective.
- When the level of 3-aminoisobutyric acid is high after 1^{st} administration of a PD-1 signal inhibitor-containing drug, therapy with the drug is predicted and/or judged effective.
- When the level of acetylcarnosine is high after 1^{st} administration of a PD-1 signal inhibitor-containing drug, therapy with the drug is predicted and/or judged effective.
- When the level of alanine is high after 1^{st} administration of a PD-1 signal inhibitor-containing drug, therapy with the drug is predicted and/or judged effective.
- When the level of arginine is high after 1^{st} administration of a PD-1 signal inhibitor-containing drug, therapy with the drug is predicted and/or judged effective.
- When the level of N-acetylornitine is high after 1^{st} administration of a PD-1 signal inhibitor-containing drug, therapy with the drug is predicted and/or judged effective.

### (After 2^{nd} Administration of Drug (Time point: 3rd))

- • When the level of 4-cresol is high after 2^{nd} administration of a PD-1 signal inhibitor-containing drug (Time point: 3rd), therapy with the drug is predicted and/or judged effective.
- • When the level of 3-hydroxyisovaleric acid is low after 2^{nd} administration of a PD-1 signal inhibitor-containing drug, therapy with the drug is predicted and/or judged effective.
- • When the level of pyruvic acid is low after 2^{nd} administration of a PD-1 signal inhibitor-containing drug, therapy with the drug is predicted and/or judged effective.
- • When the level of α-ketoglutaric acid is low after 2^{nd} administration of a PD-1 signal inhibitor-containing drug, therapy with the drug is predicted and/or judged effective.
- • When the level of hippuric acid is high after 2^{nd} administration of a PD-1 signal inhibitor-containing drug, therapy with the drug is predicted and/or judged effective.
- • When the level of cystine is high after 2^{nd} administration of a PD-1 signal inhibitor-containing drug, therapy with the drug is predicted and/or judged effective.
- • When the level of indoxyl sulfate is high after 2^{nd} administration of a PD-1 signal inhibitor-containing drug, therapy with the drug is predicted and/or judged effective.
- • When the level of GSSG is high after 2^{nd} administration of a PD-1 signal inhibitor-containing drug, therapy with the drug is predicted and/or judged effective.
- • When the level of uric acid is high after 2^{nd} administration of a PD-1 signal inhibitor-containing drug, therapy with the drug is predicted and/or judged effective.
- • When the level of 2-hydrobutyric acid is low after 2^{nd} administration of a PD-1 signal inhibitor-containing drug, therapy with the drug is predicted and/or judged effective.
- • When the level of pipecolic acid is low after 2^{nd} administration of a PD-1 signal inhibitor-containing drug, therapy with the drug is predicted and/or judged effective.
- • When the level of acylcarnitine (e.g., butyrylcarnitine (C4)) is low after 2^{nd} administration of a PD-1 signal inhibitor-containing drug, therapy with the drug is predicted and/or judged effective.

(Ratio of Level after 1^{st} Administration of Drug to Level before Treatment (Drug Administration) (Ratio of two time points: 2nd/1st))
- • • When the ratio of creatinine level after 1^{st} administration of a PD-1 signal inhibitor-containing drug to creatinine level before treatment (drug administration) (Ratio of two points) is low, therapy with the drug is predicted and/or judged effective.
- • • When the ratio of 1,5-anhydro-D-sorbitol level after 1^{st} administration of a PD-1 signal inhibitor-containing drug to 1,5-anhydro-D-sorbitol level before treatment (drug administration) is high, therapy with the drug is predicted and/or judged effective.
- • • When the ratio of cystine level after 1^{st} administration of a PD-1 signal inhibitor-containing drug to cystine level before treatment (drug administration) is high, therapy with the drug is predicted and/or judged effective.
- • • When the ratio of glutamine level after 1^{st} administration of a PD-1 signal inhibitor-containing drug to glutamine level before treatment (drug administration) is high, therapy with the drug is predicted and/or judged effective.
- • • When the ratio of glycine level after 1^{st} administration of a PD-1 signal inhibitor-containing drug to glycine level before treatment (drug administration) is high, therapy with the drug is predicted and/or judged effective.
- • • When the ratio of lysine level after 1^{st} administration of a PD-1 signal inhibitor-containing drug to lysine level before treatment (drug administration) is high, therapy with the drug is predicted and/or judged effective.
- • • When the ratio of pyroglutamic acid level after 1^{st} administration of a PD-1 signal inhibitor-containing drug to pyroglutamic acid level before treatment (drug administration) is high, therapy with the drug is predicted and/or judged effective.
- • • When the ratio of taurine level after 1^{st} administration of a PD-1 signal inhibitor-containing drug to taurine level before treatment (drug administration) is low, therapy with the drug is predicted and/or judged effective.
- • • When the ratio of asy-dimethylarginine level after 1^{st} administration of a PD-1 signal inhibitor-containing drug to asy-dimethylarginine level before treatment (drug administration) is high, therapy with the drug is predicted and/or judged effective.
- • • When the ratio of AMP level after 1^{st} administration of a PD-1 signal inhibitor-containing drug to AMP level before treatment (drug administration) is low, therapy with the drug is predicted and/or judged effective.
- • • When the ratio of acylcarnitine (e.g., isovalerylcarnitine (C5)) level after 1^{st} administration of a PD-1 signal inhibitor-containing drug to acylcarnitine (e.g., isovalerylcarnitine (C5)) level before treatment (drug administration) is low, therapy with the drug is predicted and/or judged effective.
- • • When the ratio of acylcarnitine (e.g., hexanoylcarnitine (C6)) level after 1^{st} administration of a PD-1 signal inhibitor-containing drug to acylcarnitine (e.g., hexanoylcarnitine (C6)) level before treatment (drug administration) is low, therapy with the drug is predicted and/or judged effective.
- • • When the ratio of acetylcarnosine level after 1^{st} administration of a PD-1 signal inhibitor-containing drug to acetylcarnosine level before treatment (drug administration) is high, therapy with the drug is predicted and/or judged effective.
- • • When the ratio of arginine level after 1^{st} administration of a PD-1 signal inhibitor-containing drug to arginine level before treatment (drug administration) is high, therapy with the drug is predicted and/or judged effective.
- • • When the ratio of citrulline level after 1^{st} administration of a PD-1 signal inhibitor-containing drug to citrulline level before treatment (drug administration) is high, therapy with the drug is predicted and/or judged effective.
- • • When the ratio of N-acetylornitine level after 1^{st} administration of a PD-1 signal inhibitor-containing drug to N-acetylornitine level before treatment (drug administration) is high, therapy with the drug is predicted and/or judged effective.

(Ratio of Level after 2^{nd} Administration of Drug to Level before Treatment (Drug Administration) (Ratio of two time points: 3rd/1st))
- • • • When the ratio of 3-hydroxybutyric acid level after 2^{nd} administration of a PD-1 signal inhibitor-containing drug to 3-hydroxybutyric acid level before treatment (drug administration) is low, therapy with the drug is predicted and/or judged effective.
- • • • When the ratio of 2-hydroxyisovaleric acid level after 2^{nd} administration of a PD-1 signal inhibitor-containing drug to 2-hydroxyisovaleric acid level before treatment (drug administration) is low, therapy with the drug is predicted and/or judged effective.
- • • • When the ratio of creatinine level after 2^{nd} administration of a PD-1 signal inhibitor-containing drug to creatinine level before treatment (drug administration) is low, therapy with the drug is predicted and/or judged effective.
- • • • When the ratio of hippuric acid level after 2^{nd} administration of a PD-1 signal inhibitor-containing drug to hippuric acid level before treatment (drug administration) is high, therapy with the drug is predicted and/or judged effective.
- • • • When the ratio of oleic acid level after 2^{nd} administration of a PD-1 signal inhibitor-containing drug to oleic acid level before treatment (drug administration) is low, therapy with the drug is predicted and/or judged effective.
- • • • When the ratio of acetoacetic acid level after 2^{nd} administration of a PD-1 signal inhibitor-containing drug to acetoacetic acid level before treatment (drug administration) is low, therapy with the drug is predicted and/or judged effective.
- • • • When the ratio of ribose level after 2^{nd} administration of a PD-1 signal inhibitor-containing drug to ribose level before treatment (drug administration) is low, therapy with the drug is predicted and/or judged effective.
- • • • When the ratio of GSSG level after 2^{nd} administration of a PD-1 signal inhibitor-containing drug to GSSG level before treatment (drug administration) is high, therapy with the drug is predicted and/or judged effective.
- • • • When the ratio of tryptophan level after 2^{nd} administration of a PD-1 signal inhibitor-containing drug to tryptophan level before treatment (drug administration) is high, therapy with the drug is predicted and/or judged effective.
- • • • When the ratio of 2-hydroxyglutaric acid level after 2^{nd} administration of a PD-1 signal inhibitor-containing drug to 2-hydroxyglutaric acid level before treatment (drug administration) is low, therapy with the drug is predicted and/or judged effective.
- • • • When the ratio of malic acid level after 2^{nd} administration of a PD-1 signal inhibitor-containing drug to malic acid level before treatment (drug administration) is low, therapy with the drug is predicted and/or judged effective.
- • • • When the ratio of quinolinic acid level after 2^{nd} administration of a PD-1 signal inhibitor-containing drug to quinolinic acid level before treatment (drug administration) is low, therapy with the drug is predicted and/or judged effective.
- • • • When the ratio of acylcarnitine (e.g., butyrylcarnitine (C4)) level after 2^{nd} administration of a PD-1 signal inhibitor-containing drug to acylcarnitine (e.g., butyrylcarnitine (C4)) level before treatment (drug administration) is low, therapy with the drug is predicted and/or judged effective.

(Ratio of Level after 2^{nd} Administration of Drug to Level after 1^{st} Administration of Drug (Ratio of two time points: 3rd/2nd))
- • • • When the ratio of caproic acid level after 2^{nd} administration of a PD-1 signal inhibitor-containing drug to caproic acid level after 1^{st} administration of the PD-1 signal inhibitor-containing drug is high, therapy with the drug is predicted and/or judged effective.
- • • • When the ratio of 4-cresol level after 2^{nd} administration of a PD-1 signal inhibitor-containing drug to 4-cresol level after 1^{st} administration of the PD-1 signal inhibitor-containing drug is high, therapy with the drug is predicted and/or judged effective.
- • • • When the ratio of isoleucine level after 2^{nd} administration of a PD-1 signal inhibitor-containing drug to isoleucine level after 1^{st} administration of the PD-1 signal inhibitor-containing drug is high, therapy with the drug is predicted and/or judged effective.
- • • • When the ratio of arabinose level after 2^{nd} administration of a PD-1 signal inhibitor-containing drug to arabinose level after 1^{st} administration of the PD-1 signal inhibitor-containing drug is low, therapy with the drug is predicted and/or judged effective.
- • • • When the ratio of ribose level after 2^{nd} administration of a PD-1 signal inhibitor-containing drug to ribose level after 1^{st} administration of the PD-1 signal inhibitor-containing drug is low, therapy with the drug is predicted and/or judged effective.
- • • • When the ratio of GSH level after 2^{nd} administration of a PD-1 signal inhibitor-containing drug to GSH level after 1^{st} administration of the PD-1 signal inhibitor-containing drug is high, therapy with the drug is predicted and/or judged effective.
- • • • When the ratio of GSSG level after 2^{nd} administration of a PD-1 signal inhibitor-containing drug to GSSG level after 1^{st} administration of the PD-1 signal inhibitor-containing drug is high, therapy with the drug is predicted and/or judged effective.
- • • • When the ratio of 3-OH-kynurenine level after 2^{nd} administration of a PD-1 signal inhibitor-containing drug to 3-OH-kynurenine level after 1^{st} administration of the PD-1 signal inhibitor-containing drug is low, therapy with the drug is predicted and/or judged effective.
- • • • When the ratio of hippuric acid level after 2^{nd} administration of a PD-1 signal inhibitor-containing drug to hippuric acid level after 1^{st} administration of the PD-1 signal inhibitor-containing drug is high, therapy with the drug is predicted and/or judged effective.
- • • • When the ratio of acylcarnitine (e.g., isobutyrylcarnitine (C4)) level after 2^{nd} administration of a PD-1 signal inhibitor-containing drug to acylcarnitine (e.g., isobutyrylcarnitine (C4)) level after 1^{st} administration of the PD-1 signal inhibitor-containing drug is high, therapy with the drug is predicted and/or judged effective.

### Table 5 (Cellular Markers in Peripheral Blood)

- When the frequency of CD4⁺ T cells in peripheral blood mononuclear cells (PBMC) (% of CD4⁺ T cells among PBMC) is high after 1^{st} administration of a PD-1 signal inhibitor-containing drug, therapy with the drug is predicted and/or judged effective.
- When the ratio of the frequency of CD4⁺ T cells in PBMC (% of CD4⁺ T cells among PBMC) after 1^{st} administration of a PD-1 signal inhibitor-containing drug to the frequency of CD4⁺ T cells in PBMC (% of CD4⁺ T cells among PBMC) before treatment (drug administration) is high, therapy with the drug is predicted and/or judged effective.
- When the frequency of CD8⁺ T cells in PBMC (% of CD8⁺ T cells among PBMC) is high after 1^{st} administration of a PD-1 signal inhibitor-containing drug, therapy with the drug is predicted and/or judged effective.
- When the ratio of the frequency of CD8⁺ T cells in PBMC (% of CD8⁺ T cells among PBMC) after 1^{st} administration of a PD-1 signal inhibitor-containing drug to the frequency of CD8⁺ T cells in PBMC (% of CD8⁺ T cells among PBMC) before treatment (drug administration) is high, therapy with the drug is predicted and/or judged effective.
- When the ratio of the frequency of naive T cells in CD8⁺ T cells (% of Tnaive among CD8⁺ T cells) after 1^{st} administration of a PD-1 signal inhibitor-containing drug to the frequency of naive T cells in CD8⁺ T cells (% of Tnaive among CD8⁺ T cells) before treatment (drug administration) is high, therapy with the drug is predicted and/or judged effective.
- When the ratio of the frequency of central memory T cells in CD4⁺ T cells (% of Tcm among CD4⁺ T cells) after 1^{st} administration of a PD-1 signal inhibitor-containing drug to the frequency of central memory T cells in CD4⁺ T cells (% of Tcm among CD4⁺ T cells) before treatment (drug administration) is high, therapy with the drug is predicted and/or judged effective.
- When the ratio of the frequency of central memory T cells in CD8⁺ T cells (% of Tcm among CD8⁺ T cells) after 1^{st} administration of a PD-1 signal inhibitor-containing drug to the frequency of central memory T cells in CD8⁺ T cells (% of Tcm among CD8⁺ T cells) before treatment (drug administration) is high, therapy with the drug is predicted and/or judged effective.
- When the ratio of the frequency of effector memory T cells in CD8⁺ T cells (% of Tem among CD8⁺ T cells) after 2^{nd} administration of a PD-1 signal inhibitor-containing drug to the frequency of effector memory T cells in CD8⁺ T cells (% of Tem among CD8⁺ T cells) before treatment (drug administration) is high, therapy with the drug is predicted and/or judged effective.
- When the ratio of the frequency of terminally differentiated effector memory T cells in CD4⁺ T cells (% of Temra among CD4⁺ T cells) after 1^{st} administration of a PD-1 signal inhibitor-containing drug to the frequency of terminally differentiated effector memory T cells in CD4⁺ T cells (% of Temra among CD4⁺ T cells) before treatment (drug administration) is low, therapy with the drug is predicted and/or judged effective.
- When the ratio of the frequency of terminally differentiated effector memory T cells in CD4⁺ T cells (% of Temra among CD4⁺ T cells) after 2^{nd} administration of a PD-1 signal inhibitor-containing drug to the frequency of terminally differentiated effector memory T cells in CD4⁺ T cells (% of Temra among CD4⁺ T cells) after 1^{st} administration of the PD-1 signal inhibitor-containing drug is high, therapy with the drug is predicted and/or judged effective.
- When the ratio of the frequency of terminally differentiated effector memory T cells in CD8⁺ T cells (% of Temra among CD8⁺ T cells) after 1^{st} administration of a PD-1 signal inhibitor-containing drug to the frequency of terminally differentiated effector memory T cells in CD8⁺ T cells (% of Temra among CD8⁺ T cells) before treatment (drug administration) is low, therapy with the drug is predicted and/or judged effective.
- When the ratio of mitochondrial reactive oxygen species production in CD8⁺ T cells to mitochondrial reactive oxygen species production in CD4⁺ T cells (Mito SOX CD8/CD4) is high before treatment (drug administration), therapy with a PD-1 signal inhibitor-containing drug is predicted effective.
- When the ratio of mitochondrial reactive oxygen species production in CD8⁺ T cells to mitochondrial reactive oxygen species production in CD4⁺ T cells (Mito SOX CD8/CD4) is high after 1^{st} administration of a PD-1 signal inhibitor-containing drug, therapy with the drug is predicted and/or judged effective.
- When the ratio of mitochondrial reactive oxygen species production in CD8⁺ T cells to mitochondrial reactive oxygen species production in CD4⁺ T cells (Mito SOX CD8/CD4) is high after 2^{nd} administration of a PD-1 signal inhibitor-containing drug, therapy with the drug is predicted and/or judged effective.
- When the ratio of mitochondrial reactive oxygen species production in CD8⁺ T cells to mitochondrial reactive oxygen species production in CD4⁺ T cells (Mito SOX CD8/CD4) after 2^{nd} administration of a PD-1 signal inhibitor-containing drug as relative to the ratio of mitochondrial reactive oxygen species production in CD8⁺ T cells to mitochondrial reactive oxygen species production in CD4⁺ T cells (Mito SOX CD8/CD4) before treatment (drug administration) is low, therapy with the drug is predicted and/or judged effective.
- When the ratio of mitochondrial mass in CD8⁺ cells to mitochondrial mass in CD4⁺ cells (Mito mass CD8/CD4) is high before treatment (drug administration), therapy with a PD-1 signal inhibitor-containing drug is predicted effective.
- When the ratio of mitochondrial mass in CD8⁺ cells to mitochondrial mass in CD4⁺ cells (Mito mass CD8/CD4) is high after 2^{nd} administration of a PD-1 signal inhibitor-containing drug, therapy with the drug is predicted and/or judged effective.
- When PGC-1α and PGC-1β expression in CD8⁺ T cells (PGC-1αβ (MFI) of CD8⁺ T cells) is low after 1^{st} administration of a PD-1 signal inhibitor-containing drug, therapy with the drug is predicted and/or judged effective.
- When PGC-1α and PGC-1β expression in CD8⁺ T cells (PGC-1αβ (MFI) of CD8⁺ T cells) after 1^{st} administration of a PD-1 signal inhibitor-containing drug is lower than PGC-1α and PGC-1β expression in CD8⁺ T cells (PGC-1αβ (MFI) of CD8⁺ T cells) before treatment (drug administration), therapy with the drug is predicted and/or judged effective.
- When PGC-1α and PGC-1β expression in CD8⁺ T cells (PGC-1αβ (MFI) of CD8⁺ T cells) after 2^{nd} administration of a PD-1 signal inhibitor-containing drug is high relative to PGC-1α and PGC-1β expression in CD8⁺ T cells (PGC-1αβ (MFI) of CD8⁺ T cells) after 1^{st} administration of the PD-1 signal inhibitor-containing drug, therapy with the drug is predicted and/or judged effective.
- When the frequency of PD-1 highly expressing CD8⁺ T cell population (% of PD-1^{high} among CD8⁺ T cells) is low before treatment (drug administration), therapy with a PD-1 signal inhibitor-containing drug is predicted effective.
- When the frequency of FoxP3 lowly expressing CD45RA⁺ T cell population in CD4⁺ T cells (% of FoxP3^{low} CD45RA⁺ among CD4⁺ T cells) is low before treatment (drug administration), therapy with a PD-1 signal inhibitor-containing drug is predicted effective.
- When the frequency of T-bet highly expressing T cell population in CD4⁺ T cells (% of T-bet^{high} among CD4⁺ T cells) after 2^{nd} administration of a PD-1 signal inhibitor-containing drug is high relative to the frequency of T-bet highly expressing T cell population in CD4⁺ T cells (% of T-bet^{high} among CD4⁺ T cells) before treatment (drug administration), therapy with the drug is predicted and/or judged effective.
- When the frequency of T-bet lowly expressing T cell population in CD4⁺ T cells (% of T-bet^{low} among CD4⁺ T cells) after 2^{nd} administration of a PD-1 signal inhibitor-containing drug is high relative to the frequency of T-bet lowly expressing T cell population in CD4⁺ T cells (% of T-bet^{low} among CD4⁺ T cells) before treatment (drug administration), therapy with the drug is predicted and/or judged effective.
- When the frequency of T-bet expressing T cell population in CD8⁺ T cells (% of T-bet⁺ among CD8⁺ T cells) after 2^{nd} administration of a PD-1 signal inhibitor-containing drug is high relative to the frequency of T-bet expressing T cell population in CD8⁺ T cells (% of T-bet⁺ among CD8⁺ T cells) after 1^{st} administration of the PD-1 signal inhibitor-containing drug, therapy with the drug is predicted and/or judged effective.
- When the frequency of T-bet and EOMES expressing T cell population in CD8⁺ T cells (% of T-bet⁺ EOMES⁺ among CD8⁺ T cells) after 2^{nd} administration of a PD-1 signal inhibitor-containing drug is high relative to the frequency of T-bet and EOMES expressing T cell population in CD8⁺ T cells (% of T-bet⁺ EOMES⁺ among CD8⁺ T cells) after 1^{st} administration of the PD-1 signal inhibitor-containing drug, therapy with the drug is predicted and/or judged effective.

In one preferred embodiment of the present invention, the metabolite of (i) may be at least one metabolite selected from the group consisting of hippuric acid, arabinose and acylcarnitine, and the cellular marker of (ii) may be at least one cellular marker selected from the group consisting of the frequency of PD-1 highly expressing CD8⁺ T cell population, the ratio of mitochondrial reactive oxygen species production in CD8⁺ T cells to mitochondrial reactive oxygen species production in CD4⁺ T cells, and PGC-1α and PGC-1β expression in CD8⁺ T cells.

As used herein, the term "PD-1 signal" refers to the signal transduction mechanism which PD-1 bears. As one aspect of this mechanism, PD-1 inhibits T cell activation in collaboration with its ligands PD-L1 and PD-L2. PD-1 (Programmed cell death-1) is a membrane protein expressed in activated T cells and B cells. Its ligands PD-L1 and PD-L2 are expressed in various cells such as antigen-presenting cells (monocytes, dendritic cells, etc.) and cancer cells. PD-1, PD-L1 and PD-L2 work as inhibitory factors which inhibit T cell activation. Certain types of cancer cells and virus-infected cells escape from host immune surveillance by expressing the ligands of PD-1 to thereby inhibit T cell activation.

PD-1 signal inhibitors are drugs which block PD-1 signals. By blocking PD-1 signals, PD-1 signal inhibitors cancel inhibition of T cell activation and enhance immune surveillance so as to exert therapeutic effect on cancers and virus infections (PD-1 blockade therapy). As PD-1 signal inhibitors, substances which specifically bind to PD-1, PD-L1 or PD-L2 may be given. Such substances include, but are not limited to, proteins, polypeptides, oligopeptides, nucleic acids (including natural-type and artificial nucleic acids), low molecular weight organic compounds, inorganic compounds, cell extracts, and extracts from animals, plants, soils or the like. These substances may be either natural or synthetic products. Preferable PD-1 signal inhibitors are antibodies. More preferably, antibodies such as anti-PD-1 antibody, anti-PD-L1 antibody and anti-PD-L2 antibody may be given. Any type of antibody may be used as long as it is capable of inhibiting PD-1 signal. The antibody may be any of polyclonal antibody, monoclonal antibody, chimeric antibody, single chain antibody, humanized antibody or human-type antibody. Methods for preparing such antibodies are known. The antibody may be derived from any organisms such as human, mouse, rat, rabbit, goat or guinea pig. As used herein, the term "antibody" is a concept encompassing antibodies of smaller molecular sizes such as Fab, F(ab)'₂, ScFv, Diabody, V_{H}, V_{L}, Sc(Fv)₂, Bispecific sc(Fv)₂, Minibody, scFv-Fc monomer or scFv-Fc dimer.

In the present invention, the PD-1 signal inhibitor-containing drug may be a PD-1 signal inhibitor as a single drug that does not contain other medical ingredients; alternatively, it may be a combined drug containing a PD-1 signal
inhibitor and other medical ingredients.

Other medical ingredients may be ones that enhance or reinforce the drug efficacy of PD-1 signal inhibitors, or ones that alleviate the adverse effect of PD-1 signal inhibitors. The drug efficacy of other medical ingredients is not particularly limited. Currently approved concomitant drugs include, but are not limited to, ipilimumab, cisplatin, carboplatin, paclitaxel and pemetrexed. A large number of concomitant drugs are under development, including those which are currently used as standard of care, immune regulation-related drugs (such as those relating to energy metabolism), and angiogenic inhibitors (1-3).

The PD-1 signal inhibitor-containing drug may be used as an active ingredient of an anticancer agent, an anti-infective agent or a combination thereof.

Subjects to be tested may be either persons who have already received administration of other drugs (such as anti-cancer agent) or persons who have not received such administration. Preferably, subjects have already received administration of other drugs.

In the present invention, prediction and/or judgement of the efficacy of therapy with a PD-1 signal inhibitor-containing drug may be made at any stage of the following: before start of treatment, after start of treatment, between a plurality of times of drug administration, etc.

In one preferred embodiment of the present invention, at least one member selected from the group consisting of hippuric acid, arabinose and acylcarnitine in the serum and/or plasma of a subject may be measured.

Hippuric acid is one of microbiota-derived metabolites and known to be produced preferentially by Clostridium (4, 5).

Arabinose is converted into xylulose-5-phosphate (an intermediate of pentose phosphate cycle) and enters into the metabolic pathway for synthesizing NADPH and nucleic acid. Enteric bacteria, in particular, are highly expressing enzymes for the above conversion, which potentially affects gut microbiota (6).

Acylcarnitine is converted from short-chain acyl-CoA and carnitine by carnitine-dependent enzymes, carnitine acetyltransferase and carnitine palmitoyltransferase 1 (CPT1) which are localized in the mitochondrial matrix. Acylcarnitine may be 4- to 6-carbon acylcarnitine; specifically, butyrylcarnitine, isovalerylcarnitine and hexanoylcarnitine. Butyrylcarnitine, the 4-carbone acylcarnitine, serves as a fatty acid transporter into mitochondria to generate ATP. Acylcarnitine species with various carbon numbers are released from cells once the function of fatty acid oxidation (FAO) is attenuated.

In the present invention, at least one member selected from the group consisting of cysteine, cystine, arginine and glutathione disulfide in serum and/or plasma may be used as the indicator of (i).

In the biosynthesis and metabolism of glutathione (GSH), cysteine binds to L-glutamate to form L-γ-glutamylcysteine, which then binds to glycine to yield GSH. GSH is oxidized into its oxidized form (GSSG) after reaction with reactive oxygen species (ROS) (Fig. 10).

Cystine is a component of glutathione (Fig. 10).

Arginine is important for composing cytoskeleton and as an energy source. It is believed that immunosuppression resulting from arginine depletion is occurring under tumor local environment where macrophage produces arginase highly.

Alanine, 4-cresol, cysteine, hippuric acid, oleic acid, indoxyl sulfate, ribose, indoleacetate, uric acid, trans-urocanic acid, pipecolic acid, N-acetylglucosamine, indolelactic acid, arabinose, arabitol, cystine, indoxyl sulfate, gluconic acid, citrulline, creatinine, N-acetylaspartic acid, pyroglutamic acid, trimethyllysine, asy-dimethylarginine, sym-dimethylarginine, methylhistidine, acylcarnitine, 3-aminoisobutyric acid, acetylcarnosine, arginine, N-acetylornitine, 3-hydroxyisovaleric acid, pyruvic acid, α-ketoglutaric acid, GSSG, 2-hydrobutyric acid, 1,5-anhydro-D-sorbitol, glutamine, glycine, lysine, taurine, AMP, acetylcarnosine, 3-hydroxybutyric acid, 2-hydroxyisovaleric acid, acetoacetic acid, tryptophan, 2-hydroxyglutaric acid, malic acid, quinolinic acid, caproic acid, isoleucine, GSH and 3-OH-kynurenine may be measured by mass spectrometry (e.g., GC-MS or LC-MS)

In the present invention, alanine, 4-cresol, cysteine, hippuric acid, oleic acid, indoxyl sulfate, ribose, indoleacetate, uric acid, trans-urocanic acid, pipecolic acid, N-acetylglucosamine, indolelactic acid, arabinose, arabitol, cystine, indoxyl sulfate, gluconic acid, citrulline, creatinine, N-acetylaspartic acid, pyroglutamic acid, trimethyllysine, asy-dimethylarginine, sym-dimethylarginine, methylhistidine, acylcarnitine, 3-aminoisobutyric acid, acetylcarnosine, arginine, N-acetylornitine, 3-hydroxyisovaleric acid, pyruvic acid, α-ketoglutaric acid, GSSG, 2-hydrobutyric acid, 1,5-anhydro-D-sorbitol, glutamine, glycine, lysine, taurine, AMP, acetylcarnosine, 3-hydroxybutyric acid, 2-hydroxyisovaleric acid, acetoacetic acid, tryptophan, 2-hydroxyglutaric acid, malic acid, quinolinic acid, caproic acid, isoleucine, GSH and 3-OH-kynurenine in serum and/or plasma may be used as biomarkers for predicting and/or judging the efficacy of therapy with a PD-1 signal inhibitor-containing drug.

In one embodiment of the present invention, it is possible to predict that therapy with a PD-1 signal inhibitor-containing drug is effective when cysteine level in serum and/or plasma before administration of the drug is high and hippuric acid level in serum and/or plasma before administration of the drug is high.

In another embodiment of the present invention, it is possible to judge that therapy with a PD-1 signal inhibitor-containing drug is effective when arabinose level in serum and/or plasma after 1^{st} administration of the drug is high; arginine level in serum and/or plasma after 1^{st} administration of the drug is high; and butyrylcarnitine level in serum and/or plasma after 1^{st} administration of the drug is low.

In still another embodiment of the present invention, it is possible to judge that therapy with a PD-1 signal inhibitor-containing drug is effective when hippuric acid level in serum and/or plasma before administration of the drug is high; cystine level in serum and/or plasma after 1^{st} administration of the drug is high; glutathione disulfide level in serum and/or plasma after 2^{nd} administration of the drug is high; and butyrylcarnitine level in serum and/or plasma after 2^{nd} administration of the drug is low.

Further, in one preferred embodiment of the present invention, at least one member selected from the group consisting of the frequency of PD-1 highly expressing CD8⁺ T cell population, the ratio of mitochondrial activation status in CD8⁺ T cells to mitochondrial activation status in CD4⁺ T cells, and PGC-1α and PGC-1β expression in CD8⁺ T cells in peripheral blood may be measured.

PD-1 highly expressing CD8⁺ T cell population is called exhausted cells, and is led to unresponsiveness after undergoing multiple times of division. High frequency of this cell population means that the ratio of exhausted, unresponsive cells is high, which is believed to result in weakened immune response to cancers.

As used herein, the expression "PD-1 highly expressing" means that the fluorescence intensity is higher than 10³ when PD-1 expression is detected by flow cytometry. However, this value is variable. The reference value may be determined using patient samples stained with isotype control, patient samples with no (or a few) cases of high positive, and patient samples with many cases of high positive.

The ratio of mitochondrial activation status in CD8⁺ T cells to mitochondrial activation status in CD4⁺ T cells is likely to reflect the intensity of immunity that injures tumors. The mitochondrial activity of CD8⁺ T cells (killer T cells) is deeply involved in the status of intracellular energy metabolism, and is capable of reflecting the cytotoxic activity of killer T cells that injure cancer cells. The mitochondrial activity is lowered when CD8⁺ T cells are exhausted to become nonresponsive (10).

PGC-1 is a major regulator for mitochondrial biogenesis and mitochondrial metabolic pathways (oxidative phosphorylation (OXPHOS), fatty acid oxidation (FAO), and others) (10, 11). PGC-1α and PGC-1β, called transcriptional coactivators, bind to multiple transcription factors to thereby promote mitochondrial biosynthesis, mitochondria-involving energy metabolism and mitochondrial activity-related transcription (11).

PGC-1α and PGC-1β expression in CD8⁺ T cells is reflecting the mitochondrial activity of CD8⁺ T cells. When CD8⁺ T cells have been led to the state of exhaustion and unresponsiveness, PGC-1 expression decreases. Conversely, unresponsiveness in CD8⁺ T cells can be recovered by allowing high expression of PGC-1 (10).

Peripheral blood CD8⁺ T cells may be collected as described below. Briefly, blood samples taken from patients are placed on Ficoll and centrifuged at 2000 rpm. The buffy coat between erythrocyte phase and plasma phase is collected and washed with a cell culture medium. From the resultant lymphocytes, CD8⁺ T cells are isolated with a magnetic cell sorter (Miltenyi Biotec).

Peripheral blood CD4⁺ T cells can be collected by isolating them with a magnetic cell sorter in the same manner as described for CD8⁺ T cells.

The frequency of PD-1 highly expressing CD8⁺ T cell population may be measured by staining leucocytes from patient's peripheral blood with fluorescently labeled antibodies against PD-1 and CD8, and conducting imaging, flow cytometry or microplate analysis (for details, see the method in Example described later).

The mitochondrial activation status of CD4⁺ T cells or CD8⁺ T cells may be detected using ROS (reactive oxygen species), OCR (oxygen consumption rate), membrane potential, mitochondrial mass, or the like as an indicator.

ROS may be detected by staining CD4⁺ T cells or CD8⁺ T cells with a dye Mito SOX, and conducting imaging, flow cytometry or microplate analysis.

OCR may be measured with XF96 Extracellular Flux Analyzer (Seahorse Biosciences). Briefly, isolated CD8⁺ T cells are plated on a dedicated cell culture plate. A sensor cartridge is placed on the plate. Oligomycin, FCCP, Antimycine A and Rotenone are poured into the injection port of the sensor cartridge. Then, the plate with the sensor cartridge is set in XF96 Extracellular Flux Analyzer. Oxygen concentration and hydrogen ion concentration in the semi-closed microenvironment between cells and the sensor are measured.

Membrane potential may be detected by staining CD4⁺ T cells or CD8⁺ T cells with a dye MitoTracker Deep Red and conducting imaging, flow cytometry or microplate analysis.

Mitochondrial mass may be detected by staining CD4⁺ T cells or CD8⁺ T cells with a dye MitoTracker Green and conducting imaging, flow cytometry or microplate analysis.

To calculate the ratio of mitochondrial activation status in CD8⁺ T cells to mitochondrial activation status in CD4⁺ T cells, a measured value which can be an indicator for mitochondrial activation status in CD8⁺ T cells may be divided by a measured value which can be an indicator for mitochondrial activation status in CD4⁺ T cells. As ratios of mitochondrial activation status in CD8⁺ T cells to mitochondrial activation status in CD4⁺ T cells, the ratio of mitochondrial reactive oxygen species production (Mito SOX level) between CD8⁺ T cells and CD4⁺ T cells (Mito SOX CD8/CD4), the ratio of mitochondrial mass in CD8⁺ T cells to mitochondrial mass in CD4⁺ T cells (Mito mass CD8/CD4), and the like may be enumerated.

PGC-1α and PGC-1β expression may be detected using a monoclonal antibody which recognizes both PGC-1α and PGC-1β (hereinafter, sometimes expressed as "PGC-1αβ").

In the present invention, the frequency of CD4⁺ T cells in peripheral blood mononuclear cells (PBMC) may be used as an indicator of (ii).

The frequency of CD4⁺ T cells in PBMC may be measured by staining leucocytes from patient's peripheral blood with a fluorescently labeled antibody against CD4, and conducting imaging, flow cytometry or microplate analysis (for details, see the method in Example described later).

The frequency of CD4⁺ T cells among peripheral blood mononuclear cells (PBMC) (% of CD4⁺ T cells among PBMC), the frequency of CD8⁺ T cells among PBMC (% of CD⁸⁺ T cells among PBMC), the frequency of naive T cells among CD8⁺ T cells (% of Tnaive among CD8⁺ T cells), the frequency of central memory T cells among CD4⁺ T cells (% of Tcm among CD4⁺ T cells), the frequency of central memory T cells among CD8⁺ T cells (% of Tcm among CD8⁺ T cells), the frequency of effector memory T cells among CD8⁺ T cells (% of Tem among CD8⁺ T cells), the frequency of terminally differentiated effector memory T cells among CD4⁺ T cells (% of Temra among CD4⁺ T cells), the frequency of terminally differentiated effector memory T cells among CD8⁺ T cells (% of Temra among CD8⁺ T cells), the ratio of mitochondrial mass in CD8⁺ T cells to mitochondrial mass in CD4⁺ T cells (Mito mass CD8/CD4), the frequency of FoxP3 lowly expressing CD45RA⁺ T cell population among CD4⁺ T cells (% of FoxP3^{low} CD45RA⁺ among CD4⁺ T cells), the frequency of T-bet highly expressing T cell population among CD4⁺ T cells (% of T-bet^{high} among CD4⁺ T cells), the frequency of T-bet lowly expressing T cell population among CD4⁺ T cells (% of T-bet^{low} among CD4⁺ T cells), the frequency of T-bet expressing T cell population among CD8⁺ T cells (% of T-bet⁺ among CD8⁺ T cells) and the frequency of T-bet and EOMES expressing T cell population among CD8⁺ T cells (% of T-bet⁺ EOMES⁺ among CD8⁺ T cells) may be measured by known methods.

The frequency of CD4⁺ T cells among PBMC (% of CD4⁺ T cells among PBMC) means the frequency of helper T cells. Helper T cells abundantly produce cytokines that help activation of CD8⁺ killer T cells.

The frequency of CD8⁺ T cells among PBMC (% of CD8⁺ T cells among PBMC) means the proportion of killer T cells that kill cancer cells.

The frequency of naive T cells among CD8⁺ T cells (% of Tnaive among CD8⁺ T cells) means the frequency of initial cells that generate effector CD8⁺ T cells which have a high capacity to kill cancer cells. When this frequency is high, it is highly possible that the number of effector CD8⁺ T cells will increase.

The frequency of central memory T cells among CD4⁺ T cells (% of Tcm among CD4⁺ T cells) means the proportion of initial cells that generate effector CD4⁺ T cells which have a high cytokine production capacity. When this frequency is high, it is highly possible that the number of effector CD4⁺ T cells will increase.

The frequency of central memory T cells among CD8⁺ T cells (% of Tcm among CD8⁺ T cells) means the proportion of initial cells that generate effector CD8⁺ T cells which have a high capacity to kill cancer cells. When this frequency is high, it is highly possible that the number of effector CD8⁺ T cells will increase.

The frequency of effector memory T cells among CD8⁺ T cells (% of Tem among CD8⁺ T cells) means the frequency of a cell population containing a large number of CD8⁺ T cells which have a high capacity to kill cancer cells.

The frequency of terminally differentiated effector memory T cells among CD4⁺ T cells (% of Temra among CD4⁺ T cells) means the frequency of a cell population containing those exhausted cells that have reached the final stage of differentiation and have a low cytokine production capacity.

The ratio of mitochondrial mass in CD8⁺ T cells to mitochondrial mass in CD4⁺ T cells (Mito mass CD8/CD4) is suggesting that mitochondria in CD8⁺ T cells are activated and showing that intracellular energy metabolism therein is activated.

The frequency of FoxP3 lowly expressing CD45RA⁺ T cell population among CD4⁺ T cells (% of FoxP3^{low} CD45RA⁺ among CD4⁺ T cells) shows the frequency of activated CD4+ T cells before antigen sensitization.

The frequency of T-bet highly expressing T cell population among CD4⁺ T cells (% of T-bet^{high} among CD4⁺ T cells) shows the frequency of Th1 helper T cells which help CD8⁺ killer T cells efficiently.

The frequency of T-bet lowly expressing T cell population among CD4⁺ T cells (% of T-bet^{low} among CD4⁺ T cells) shows the frequency of helper T cells whose T1 helper capacity is weak.

The frequency of T-bet expressing T cell population among CD8⁺ T cells (% of T-bet⁺ among CD8⁺ T cells) shows the frequency of CD8⁺ killer T cells which have a high capacity to kill cancers.

The frequency of T-bet and EOMES expressing T cell population among CD8⁺ T cells (% of T-bet⁺ EOMES⁺ among CD8⁺ T cells) shows the frequency of memory or exhausted CD8⁺ T cells.

In the present invention, the frequency of CD4⁺ T cells among peripheral blood mononuclear cells (% of CD4⁺ T cells among PBMC), the frequency of CD8⁺ T cells among peripheral blood mononuclear cells (% of CD8⁺ T cells among PBMC), the frequency of naive T cells among CD8⁺ T cells (% of Tnaive among CD8⁺ T cells), the frequency of central memory T cells among CD4⁺ T cells (% of Tcm among CD4⁺ T cells), the frequency of central memory T cells among CD8⁺ T cells (% of Tcm among CD8⁺ T cells), the frequency of effector memory T cells among CD8⁺ T cells (% of Tem among CD8⁺ T cells), the frequency of terminally differentiated effector memory T cells among CD4⁺ T cells (% of Temra among CD4⁺ T cells), the frequency of terminally differentiated effector memory T cells among CD8⁺ T cells (% of Temra among CD8⁺ T cells), the ratio of mitochondrial activation status in CD8⁺ T cells to mitochondrial activation status in CD4⁺ T cells [e.g., the ratio of mitochondrial reactive oxygen species production in CD8⁺ T cells to mitochondrial reactive oxygen species production in CD4⁺ T cells (Mito SOX CD8/CD4), the ratio of mitochondrial mass in CD8⁺ T cells to mitochondrial mass in CD4⁺ T cells (Mito mass CD8/CD4)], PGC-1α and PGC-1β expression in CD8⁺ T cells (PGC-1αβ (MF1) of CD8⁺ T cells), the frequency of PD-1 highly expressing CD8⁺ T cell population (% of PD-1^{high} among CD8⁺ T cells), the frequency of FoxP3 lowly expressing CD45RA⁺ T cell population among CD4⁺ T cells (% of FoxP3^{low} CD45RA⁺ among CD4⁺ T cells), the frequency of T-bet highly expressing T cell population among CD4⁺ T cells (% of T-bet^{high} among CD4⁺ T cells), the frequency of T-bet lowly expressing T cell population among CD4⁺ T cells (% of T-bet^{low} among CD4⁺ T cells), the frequency of T-bet expressing T cell population among CD8⁺ T cells (% of T-bet⁺ among CD8⁺ T cells) and the frequency of T-bet and EOMES expressing T cell population among CD8⁺ T cells (% of T-bet⁺ EOMES⁺ among CD8⁺ T cells) in peripheral blood may be used as cellular markers for predicting and/or judging the efficacy of therapy with a PD-1 signal inhibitor-containing drug.

In one embodiment of the present invention, therapy with a PD-1 signal inhibitor-containing drug can be predicted effective when the frequency of PD-1 highly expressing CD8⁺ T cell population in peripheral blood before administration of the drug is low and the ratio of mitochondrial activation status in CD8⁺ T cells to mitochondrial activation status in CD4⁺ T cells (e.g., Mito SOX CD8/CD4) in peripheral blood before administration of the drug is high.

In another embodiment of the present invention, therapy with a PD-1 signal inhibitor-containing drug can be judged effective when the frequency of PD-1 highly expressing CD8⁺ T cell population in peripheral blood before administration of the drug is low; the ratio of mitochondrial activation status in CD8⁺ T cells to mitochondrial activation status in CD4⁺ T cells (e.g., Mito SOX CD8/CD4) in peripheral blood before administration of the drug is high; the ratio of PGC-1α and PGC-1β expression in CD8⁺ T cells in peripheral blood after 1^{st} administration of the drug to the corresponding expression before administration of the drug is low; and the ratio of the frequency of CD4⁺ T cells in PBMC after 1^{st} administration of the drug to the corresponding frequency before administration of the drug is high.

In still another embodiment of the present invention, therapy with a PD-1 signal inhibitor-containing drug can be judged effective when the frequency of PD-1 highly expressing CD8⁺ T cell population in peripheral blood before administration of the drug is low; the ratio of mitochondrial activation status in CD8⁺ T cells to mitochondrial activation status in CD4⁺ T cells (e.g., Mito SOX CD8/CD4) in peripheral blood before administration of the drug is high; the ratio of PGC-1α and PGC-1β expression in CD8⁺ T cells in peripheral blood after 2^{nd} administration of the drug to the corresponding expression after 1^{st} administration of the drug is high; and the ratio of the frequency of CD4⁺ T cells in PBMC after 1^{st} administration of the drug to the corresponding frequency before administration of the drug is high.

As used herein, with respect to the value (level) of biomarker or cellular marker, "high" or "low" means that the value of responder is "high" or "low" relative to the value of non-responder. As regards criteria for judging "high" or "low", 95% confidence interval of quantitative values of responders or non-responders may be used as a reference value. Alternatively, a cut-off value may be set from ROC curves or statistical analysis (such as LDA analysis) and used as a reference value. Methods for determining these values are known.

In Example described later, the present inventors investigated into the concentrations of plasma metabolites and cellular markers in the blood from 60 non-small-cell lung cancer patients before and after administration of Nivolumab (anti-PD-1 antibody). As a result, the following calculation formulas were obtained by LDA analysis. These formulas may vary when the population to be analyzed has changed. Mark ^{∗} appearing in formulas 1 to 6 means multiplication.
- Cellular marker combination I (Cut-off value 0)
   - 0.3023987485021^{∗}[PD-1 high 1st]+2.94519844381265^{∗}[Mito SOX CD8/CD4 1st] - 1.87379126258675 (Formula 1)
- Cellular marker combination II (Cut-off value 0)
   - 0.2522084377427^{∗}[PD-1 high 1st]+3.58270233554892^{∗}[Mito SOX CD8/CD4 1st]+1.92950430842982^{∗}[CD4(%) 2nd / 1st]-1.2170886788589^{∗}[PGC1ab of CD8 2nd / 1st]
   - 3.29839771768711 (Formula 2)
- Cellular marker combination III (Cut-off value 0)
   - 0.2808914943715^{∗}[PD-1 high 1st]+3.29512762046372^{∗}[Mito SOX CD8/CD4 1st]+1.55202212350758^{∗}[CD4(%) 2nd / 1st]+2.00254807664124^{∗}[PGC1ab of CD8 3rd / 2nd] -5.97495334069991 (Formula 3)
- Metabolite combination I (Cut-off value 0)
   9.92415868963082^{∗}[Cysteine 1st]+0.00000054083199^{∗}[Hippuric acid (LC-MS) 1st]-44.661430966258^{∗}[Unk8 1st] -1.46007680395094 (Formula 4)
- Metabolite combination II (Cut-off value 0)
   207.062010223744^{∗}[Arabinose 2nd]+0.00000031741819^{∗}[Arginine 2nd]-0.0000003768213^{∗}[Butyrylcarnitine 2nd] -1.95925442024969 (Formula 5)
- Metabolite combination III (Cut-off value 0)
   0.00000044275294^{∗}[Hippuric acid (LC-MS) 1st]+12.0744069146569^{∗}[Cystine 2nd]+0.00003552481124^{∗}[GSSG 3rd]-0.00000008812702408^{∗}[Butyrylcarnitine 3rd] - 2.67048929974173 (Formula 6)

PD-1 ^{high} 1st: the frequency of PD-1 highly expressing CD8⁺ T cell population in peripheral blood before administration of a PD-1 signal inhibitor-containing drug.

Mito SOX CD8/CD4 1st: the ratio of mitochondrial reactive oxygen species production in CD8⁺ T cells to the corresponding production in CD4⁺ T cells in peripheral blood before administration of a PD-1 signal inhibitor-containing drug.

CD4(%) 2nd/1st: the ratio of the frequency of CD4⁺ T cells in PBMC after 1^{st} administration of a PD-1 signal inhibitor-containing drug to the frequency of CD4⁺ T cells in PBMC before administration of the drug. (When this ratio is larger than 1, it can be said that the frequency of CD4⁺ T cells in PBMC has increased after 1^{st} administration of the PD-1 signal inhibitor-containing drug, compared to the frequency before administration of the drug.)

PGClab of CD8 2nd/1st: the ratio of PGC-1α and PGC-1β expression in CD8⁺ T cells in peripheral blood after 1^{st} administration of a PD-1 signal inhibitor-containing drug to PGC-1α and PGC-1β expression in CD8⁺ T cells in peripheral blood before administration of the PD-1 signal inhibitor-containing drug. (When this ratio is smaller than 1, it can be said that PGC-1α and PGC-1β expression in CD8⁺ T cells in peripheral blood has decreased after 1^{st} administration of the PD-1 signal inhibitor-containing drug, compared to the expression before administration of the drug.)

PGClab of CD8 3rd/2nd: the ratio of PGC-1α and PGC-1β expression in CD8⁺ T cells in peripheral blood after 2^{nd} administration of a PD-1 signal inhibitor-containing drug to PGC-1α and PGC-1β expression in CD8⁺ T cells in peripheral blood after 1^{st} administration of the PD-1 signal inhibitor-containing drug. (When this ratio is larger than 1, it can be said that PGC-1α and PGC-1β expression in CD8⁺ T cells in peripheral blood has increased after 2^{nd} administration of the PD-1 signal inhibitor-containing drug, compared to the expression after 1^{st} administration of the drug.)

Cysteine 1st: plasma cysteine level before administration of a PD-1 signal inhibitor-containing drug (as measured by GC-MS).

Hippuric acid (LC-MS) 1^{st}: plasma hippuric acid level before administration of a PD-1 signal inhibitor-containing drug (as measured by LC-MS).

Unk8 1st: plasma Unk8 (substance unidentified) level before administration of a PD-1 signal inhibitor-containing drug (as measured by GC-MS).

Arabinose 2nd: plasma arabinose level after 1^{st} administration of a PD-1 signal inhibitor-containing drug (as measured by GC-MS).

Arginine 2nd: plasma arginine level after 1^{st} administration of a PD-1 signal inhibitor-containing drug (as measured by GC-MS).

Butyrylcarnitine 2nd: plasma butyrylcarnitine level after 1^{st} administration of a PD-1 signal inhibitor-containing drug (as measured by GC-MS).

Cystine 2nd: plasma cystine level after 1^{st} administration of a PD-1 signal inhibitor-containing drug (as measured by GC-MS).

GSSG 3rd: plasma glutathione disulfide level after 2^{nd} administration of a PD-1 signal inhibitor-containing drug (as measured by GC-MS).

Butyrylcarnitine 3rd: plasma butyrylcarnitine level after 2^{nd} administration of a PD-1 signal inhibitor-containing drug (as measured by GC-MS).

When therapy with a PD-1 signal inhibitor-containing drug has been predicted effective according to the method of the present invention, therapy with the drug may be started.

When therapy with a PD-1 signal inhibitor-containing drug has been judged effective according to the method of the present invention, therapy with the drug may be continued.

The present invention provides a method of diagnosis and therapy for a disease, comprising predicting and/or judging the efficacy of therapy with a PD-1 signal inhibitor-containing drug in a subject using the following (i) and/or (ii) as indicator(s), and administering to the subject in a therapeutically effective amount when the therapy with the PD-1 signal inhibitor-containing drug has been predicted and/or judged effective:
(i) at least one metabolite in serum and/or plasma selected from the group consisting of alanine, 4-cresol, cysteine, hippuric acid, oleic acid, indoxyl sulfate, ribose, indoleacetate, uric acid, trans-urocanic acid, pipecolic acid, N-acetylglucosamine, indolelactic acid, arabinose, arabitol, cystine, indoxyl sulfate, gluconic acid, citrulline, creatinine, N-acetylaspartic acid, pyroglutamic acid, trimethyllysine, asy-dimethylarginine, sym-dimethylarginine, methylhistidine, acylcarnitine, 3-aminoisobutyric acid, acetylcarnosine, arginine, N-acetylornitine, 3-hydroxyisovaleric acid, pyruvic acid, α-ketoglutaric acid, GSSG, 2-hydrobutyric acid, 1,5-anhydro-D-sorbitol, glutamine, glycine, lysine, taurine, AMP, acetylcarnosine, 3-hydroxybutyric acid, 2-hydroxyisovaleric acid, acetoacetic acid, tryptophan, 2-hydroxyglutaric acid, malic acid, quinolinic acid, caproic acid, isoleucine, GSH and 3-OH-kynurenine
(ii) at least one cellular marker in peripheral blood selected from the group consisting of the frequency of CD4⁺ T cells among peripheral blood mononuclear cells (% of CD4⁺ T cells among PBMC), the frequency of CD8⁺ T cells among peripheral blood mononuclear cells (% of CD8⁺ T cells among PBMC), the frequency of naive T cells among CD8⁺ T cells (% of Tnaive among CD8⁺ T cells), the frequency of central memory T cells among CD4⁺ T cells (% of Tcm among CD4⁺ T cells), the frequency of central memory T cells among CD8⁺ T cells (% of Tcm among CD8⁺ T cells), the frequency of effector memory T cells among CD8⁺ T cells (% of Tem among CD8⁺ T cells), the frequency of terminally differentiated effector memory T cells among CD4⁺ T cells (% of Temra among CD4⁺ T cells), the frequency of terminally differentiated effector memory T cells among CD8⁺ T cells (% of Temra among CD8⁺ T cells), the ratio of mitochondrial activation status in CD8⁺ T cells to mitochondrial activation status in CD4⁺ T cells [e.g., the ratio of mitochondrial reactive oxygen species production in CD8⁺ T cells to mitochondrial reactive oxygen species production in CD4⁺ T cells (Mito SOX CD8/CD4), the ratio of mitochondrial mass in CD8⁺ T cells to mitochondrial mass in CD4⁺ T cells (Mito mass CD8/CD4)], PGC-1α and PGC-1β expression in CD8⁺ T cells (PGC-1αβ (MF1) of CD8⁺ T cells), the frequency of PD-1 highly expressing CD8⁺ T cell population (% of PD-1^{high} among CD8⁺ T cells), the frequency of FoxP3 lowly expressing CD45RA⁺ T cell population among CD4⁺ T cells (% of FoxP3^{low} CD45RA⁺ among CD4⁺ T cells), the frequency of T-bet highly expressing T cell population among CD4⁺ T cells (% of T-bet^{high} among CD4⁺ T cells), the frequency of T-bet lowly expressing T cell population among CD4⁺ T cells (% of T-bet^{low} among CD4⁺ T cells), the frequency of T-bet expressing T cell population among CD8⁺ T cells (% of T-bet⁺ among CD8⁺ T cells) and the frequency of T-bet and EOMES expressing T cell population among CD8⁺ T cells (% of T-bet⁺ EOMES⁺ among CD8⁺ T cells).

The present invention also provides a pharmaceutical composition comprising a PD-1 signal inhibitor-containing drug as an active ingredient, which is for use in a method of diagnosis and therapy for a disease, comprising predicting and/or judging the efficacy of therapy with the PD-1 signal inhibitor-containing drug in a subject using the following (i) and/or (ii) as indicator(s), and administering to the subject in a therapeutically effective amount when the therapy with the PD-1 signal inhibitor-containing drug has been predicted and/or judged effective:
(i) at least one metabolite in serum and/or plasma selected from the group consisting of alanine, 4-cresol, cysteine, hippuric acid, oleic acid, indoxyl sulfate, ribose, indoleacetate, uric acid, trans-urocanic acid, pipecolic acid, N-acetylglucosamine, indolelactic acid, arabinose, arabitol, cystine, indoxyl sulfate, gluconic acid, citrulline, creatinine, N-acetylaspartic acid, pyroglutamic acid, trimethyllysine, asy-dimethylarginine, sym-dimethylarginine, methylhistidine, acylcarnitine, 3-aminoisobutyric acid, acetylcarnosine, arginine, N-acetylornitine, 3-hydroxyisovaleric acid, pyruvic acid, α-ketoglutaric acid, GSSG, 2-hydrobutyric acid, 1,5-anhydro-D-sorbitol, glutamine, glycine, lysine, taurine, AMP, acetylcarnosine, 3-hydroxybutyric acid, 2-hydroxyisovaleric acid, acetoacetic acid, tryptophan, 2-hydroxyglutaric acid, malic acid, quinolinic acid, caproic acid, isoleucine, GSH and 3-OH-kynurenine
(ii) at least one cellular marker in peripheral blood selected from the group consisting of the frequency of CD4⁺ T cells among peripheral blood mononuclear cells (% of CD4⁺ T cells among PBMC), the frequency of CD8⁺ T cells among peripheral blood mononuclear cells (% of CD8⁺ T cells among PBMC), the frequency of naive T cells among CD8⁺ T cells (% of Tnaive among CD8⁺ T cells), the frequency of central memory T cells among CD4⁺ T cells (% of Tcm among CD4⁺ T cells), the frequency of central memory T cells among CD8⁺ T cells (% of Tcm among CD8⁺ T cells), the frequency of effector memory T cells among CD8⁺ T cells (% of Tem among CD8⁺ T cells), the frequency of terminally differentiated effector memory T cells among CD4⁺ T cells (% of Temra among CD4⁺ T cells), the frequency of terminally differentiated effector memory T cells among CD8⁺ T cells (% of Temra among CD8⁺ T cells), the ratio of mitochondrial activation status in CD8⁺ T cells to mitochondrial activation status in CD4⁺ T cells [e.g., the ratio of mitochondrial reactive oxygen species production in CD8⁺ T cells to mitochondrial reactive oxygen species production in CD4⁺ T cells (Mito SOX CD8/CD4), the ratio of mitochondrial mass in CD8⁺ T cells to mitochondrial mass in CD4⁺ T cells (Mito mass CD8/CD4)], PGC-1α and PGC-1β expression in CD8⁺ T cells (PGC-1αβ (MF1) of CD8⁺ T cells), the frequency of PD-1 highly expressing CD8⁺ T cell population (% of PD-1^{high} among CD8⁺ T cells), the frequency of FoxP3 lowly expressing CD45RA⁺ T cell population among CD4⁺ T cells (% of FoxP3^{low} CD45RA⁺ among CD4⁺ T cells), the frequency of T-bet highly expressing T cell population among CD4⁺ T cells (% of T-bet^{high} among CD4⁺ T cells), the frequency of T-bet lowly expressing T cell population among CD4⁺ T cells (% of T-bet^{low} among CD4⁺ T cells), the frequency of T-bet expressing T cell population among CD8⁺ T cells (% of T-bet⁺ among CD8⁺ T cells) and the frequency of T-bet and EOMES expressing T cell population among CD8⁺ T cells (% of T-bet⁺ EOMES⁺ among CD8⁺ T cells).

The pharmaceutical composition of the present invention may be used as an anticancer agent, an anti-infective agent or a combination thereof.

When the pharmaceutical composition of the present invention is administered as an anticancer agent, target cancers or tumors includes, but are not limited to, leukemia, lymphoma (e.g., Hodgkin's disease, non-Hodgkin's lymphoma), multiple myeloma, brain tumors, breast cancer, endometrial cancer, cervical cancer, ovarian cancer, esophageal cancer, stomach cancer, appendix cancer, colon cancer, liver cancer, gallbladder cancer, bile duct cancer, pancreatic cancer, adrenal cancer, gastrointestinal stromal tumor, mesothelioma, head and neck cancer (such as laryngeal cancer), oral cancer (such as floor of mouth cancer), gingival cancer, tongue cancer, buccal mucosa cancer, salivary gland cancer, nasal sinus cancer (e.g., maxillary sinus cancer, frontal sinus cancer, ethmoid sinus cancer, sphenoid sinus cancer), thyroid cancer, renal cancer, lung cancer, osteosarcoma, prostate cancer, testicular tumor (testicular cancer), renal cell carcinoma, bladder cancer, rhabdomyosarcoma, skin cancer (e.g., basal cell cancer, squamous cell carcinoma, malignant melanoma, actinic keratosis, Bowen's disease, Paget's disease) and anal cancer.

When the pharmaceutical composition of the present invention is administered as an anti-infective agent, target infections include, but are not limited to, bacterial infections [various infections caused by Streptococcus (e.g., group A β hemolytic streptococcus, pneumococcus), Staphylococcus aureus (e.g., MSSA, MRSA), Staphylococcus epidermidis, Enterococcus, Listeria, Neisseria meningitis aureus, Neisseria gonorrhoeae, pathogenic Escherichia coli (e.g., 0157:H7), Klebsiella (Klebsiella pneumoniae), Proteus, Bordetella pertussis, Pseudomonas aeruginosa, Serratia, Citrobacter, Acinetobacter, Enterobacter, mycoplasma, Clostridium or the like]; tuberculosis, cholera, plague, diphtheria, dysentery, scarlet fever, anthrax, syphilis, tetanus, leprosy, Legionella pneumonia (legionellosis), leptospirosis, Lyme disease, tularemia, Q fever, and the like], rickettsial infections (e.g., epidemic typhus, scrub typhus, Japanese spotted fever), chlamydial infections (e.g., trachoma, genital chlamydial infection, psittacosis), fungal infections (e.g., aspergillosis, candidiasis, cryptococcosis, trichophytosis, histoplasmosis, Pneumocystis pneumonia), parasitic protozoan infections (e.g., amoebic dysentery, malaria, toxoplasmosis, leishmaniasis, cryptosporidiosis), parasitic helminthic infections (e.g., echinococcosis, schistosomiasis japonica, filariasis, ascariasis, diphyllobothriasis latum), and viral infections [e.g., influenza, viral hepatitis, viral meningitis, acquired immune deficiency syndrome (AIDS), adult T-cell leukemia, Ebola hemorrhagic fever, yellow fever, cold syndrome, rabies, cytomegalovirus infection, severe acute respiratory syndrome (SARS), progressive multifocal leukoencephalopathy, chickenpox, herpes zoster, hand-foot-and-mouth disease, dengue, erythema infectiosum, infectious mononucleosis, smallpox, rubella, acute anterior poliomyelitis (polio), measles, pharyngoconjunctival fever (pool fever), Marburg hemorrhagic fever, hantavirus renal hemorrhagic fever, Lassa fever, mumps, West Nile fever, herpangina and chikungunya fever].

The pharmaceutical composition of the present invention is administered to human or animal subjects systemically or locally by an oral or parenteral route.

PD-1 signal inhibitors (e.g., anti-PD-1 antibody, anti-PD-L1 antibody or anti-PD-L2 antibody) may be dissolved in buffers such as PBS, physiological saline or sterile water, optionally filter- or otherwise sterilized before being administered to human or animal subjects by injection or infusion. To the solution of PD-1 signal inhibitors, additives such as coloring agents, emulsifiers, suspending agents, surfactants, solubilizers, stabilizers, preservatives, antioxidants, buffers, isotonizing agents and the like may be added. As routes of administration, intravenous, intramuscular, intraperitoneal, subcutaneous or intradermal administration and the like may be selected.

The content of the PD-1 signal inhibitor (e.g., anti-PD-1 antibody, anti-PD-L1 antibody or anti-PD-L2 antibody) in a preparation varies with the type of the preparation and is usually 1-100% by weight, preferably 50-100% by weight. Such a preparation may be formulated into a unit dosage form.

The dose, frequency and number of administration of PD-1 signal inhibitor (e.g., anti-PD-1 antibody, anti-PD-L1 antibody or anti-PD-L2 antibody) may vary with the symptoms, age and body weight of the human or animal subject, the method of administration, dosage form and more. For example, in terms of the amount of the active ingredient usually ranges from 0.1 to 100 mg/kg body weight, preferably 1-10 mg/kg body weight, and may be administered once per adult at a frequency that enables confirmation of efficacy. Commencement, continuation or cancellation of administration of PD-1 signal inhibitor may be determined based on test results using the above-described bio-markers and/or cellular markers.

The present invention also provides use of reagents for measuring the biomarkers of the following (i) and/or (ii) in manufacturing a product for diagnosis for determining administration of a PD-1 signal inhibitor-containing drug:
(i) at least one metabolite in serum and/or plasma selected from the group consisting of alanine, 4-cresol, cysteine, hippuric acid, oleic acid, indoxyl sulfate, ribose, indoleacetate, uric acid, trans-urocanic acid, pipecolic acid, N-acetylglucosamine, indolelactic acid, arabinose, arabitol, cystine, indoxyl sulfate, gluconic acid, citrulline, creatinine, N-acetylaspartic acid, pyroglutamic acid, trimethyllysine, asy-dimethylarginine, sym-dimethylarginine, methylhistidine, acylcarnitine, 3-aminoisobutyric acid, acetylcarnosine, arginine, N-acetylornitine, 3-hydroxyisovaleric acid, pyruvic acid, α-ketoglutaric acid, GSSG, 2-hydrobutyric acid, 1,5-anhydro-D-sorbitol, glutamine, glycine, lysine, taurine, AMP, acetylcarnosine, 3-hydroxybutyric acid, 2-hydroxyisovaleric acid, acetoacetic acid, tryptophan, 2-hydroxyglutaric acid, malic acid, quinolinic acid, caproic acid, isoleucine, GSH and 3-OH-kynurenine
(ii) at least one cellular marker in peripheral blood selected from the group consisting of the frequency of CD4⁺ T cells among peripheral blood mononuclear cells (% of CD4⁺ T cells among PBMC), the frequency of CD8⁺ T cells among peripheral blood mononuclear cells (% of CD8⁺ T cells among PBMC), the frequency of naive T cells among CD8⁺ T cells (% of Tnaive among CD8⁺ T cells), the frequency of central memory T cells among CD4⁺ T cells (% of Tcm among CD4⁺ T cells), the frequency of central memory T cells among CD8⁺ T cells (% of Tcm among CD8⁺ T cells), the frequency of effector memory T cells among CD8⁺ T cells (% of Tem among CD8⁺ T cells), the frequency of terminally differentiated effector memory T cells among CD4⁺ T cells (% of Temra among CD4⁺ T cells), the frequency of terminally differentiated effector memory T cells among CD8⁺ T cells (% of Temra among CD8⁺ T cells), the ratio of mitochondrial activation status in CD8⁺ T cells to mitochondrial activation status in CD4⁺ T cells [e.g., the ratio of mitochondrial reactive oxygen species production in CD8⁺ T cells to mitochondrial reactive oxygen species production in CD4⁺ T cells (Mito SOX CD8/CD4), the ratio of mitochondrial mass in CD8⁺ T cells to mitochondrial mass in CD4⁺ T cells (Mito mass CD8/CD4)], PGC-1α and PGC-1β expression in CD8⁺ T cells (PGC-1αβ (MF1) of CD8⁺ T cells), the frequency of PD-1 highly expressing CD8⁺ T cell population (% of PD-1^{high} among CD8⁺ T cells), the frequency of FoxP3 lowly expressing CD45RA⁺ T cell population among CD4⁺ T cells (% of FoxP3^{low} CD45RA⁺ among CD4⁺ T cells), the frequency of T-bet highly expressing T cell population among CD4⁺ T cells (% of T-bet^{high} among CD4⁺ T cells), the frequency of T-bet lowly expressing T cell population among CD4⁺ T cells (% of T-bet^{low} among CD4⁺ T cells), the frequency of T-bet expressing T cell population among CD8⁺ T cells (% of T-bet⁺ among CD8⁺ T cells) and the frequency of T-bet and EOMES expressing T cell population among CD8⁺ T cells (% of T-bet⁺ EOMES⁺ among CD8⁺ T cells).

The "product for diagnosis" is a concept encompassing kits, equipment (mass spectrometer, imaging system, flow cytometry, microplate and other analytical equipment), etc. Products for diagnosis may suitably contain programs for analysis, operation manuals, and the like.

As regards reagents for measuring biomarkers, internal standards, reagents for derivatization (e.g., derivatizing reagent for GC/MS), organic solvents for extraction, and combinations thereof may be enumerated when the biomarker is a metabolite. When the biomarker is a cellular marker, reagents for isolating cells (e.g., beads coated with antibodies to cellular marker (CD4, CD8, CD45RA) proteins), antibodies to the protein as a target of detection (PGC-1α, PGC-1β, FoxP3, T-bet, or EOMES), antibodies for detecting the target protein (preferably, labeled with an enzyme or the like), substrates for the enzyme used as a label (those which generate color, fluorescence or luminescence upon reaction with the enzyme), standard samples, and combinations thereof may be enumerated.

### EXAMPLES

Hereinbelow, the present invention will be described more specifically with reference to the following Examples.

### [Example 1]

### (Abstract)

To identify reliable biomarkers for the host immunity, the present inventors investigated the levels of plasma metabolites and cellular markers in immune cells in the blood of patients with non-small cell lung cancer before and after administration of Nivolumab. A validation study using machine learning for the statistically selected biomarkers showed that a combination of four specific metabolites derived from gut microbiome, fatty acid oxidation and redox provided high probability (AUC=0.91). A combination of four T cell markers, including those related to mitochondrial activation and the frequencies of CD8⁺ PD-1^{high} and CD4⁺ T cells, demonstrated even higher prediction value (AUC=0.96). When the present inventors statistically selected the highest predictive combination from the pool of all markers, the above-mentioned four T cell markers were selected; and no metabolite was included therein. It was believed that this result is due to strong linkage between metabolites and T cell markers. These findings indicate that combination of biomarkers reflecting host immune activity is quite valuable for the responder prediction.

### (Introduction)

PD-1 and CTLA-4 are key players in immune suppression upon tumor growth (12-15). Blocking these molecules individually or together rejuvenates immune surveillance and can induce strong anti-tumor activity in mice and humans (12-14, 16, 17). Based on the promising outcomes in clinical trials using these immune checkpoint blockade mAbs, the FDA has approved antibodies against CTLA-4, PD-1, or its ligand, PD-L1, to treat various human cancers (3, 15). Regardless of the impressive clinical efficacy of the PD-1 blockade immunotherapy, a significant portion of cancer patients remain non-responsive (3, 15-17). Therefore, therapeutic efficacy prediction biomarkers (hereinafter, "responder biomarkers") to distinguish responders and non-responders are desperately required to save cost and time for those patients.

PD-L1 high expression on tumor tissue detectable by immunostaining has been used as a responder biomarker for NSCLC (18, 19). The FDA has recently approved microsatellite instability-high (MSI-H) or DNA mismatch repair deficiency (dMMR) as common responder biomarkers for various solid tumors (20, 21). These markers, however, cannot cover all of the responsive patients because the responses of tumor-reactive killer T cells are affected not only by tumor properties but also by host immune activity. Several groups have identified candidates for responder biomarkers by analysis of cell components at tumor sites or in peripheral blood. The proposed markers are the frequencies of CD8⁺ T cells, CD4⁺ T cells, eosinophils, neutrophils, subsets of suppressive macrophages, and subsets of T cells (22). Immune regulators such as specific cytokines or chemokines are also listed as candidates for biomarkers (22). However, the prediction value of each of these single markers is not high enough for the clinical use.

It has been known that gut microbiota and immune activity mutually affect as the present inventors have demonstrated using IgA-deficient or PD-1-deficient mouse models (23, 24). As regards tumor immunity, recent reports also suggested that gut microbiota and their metabolites are related to the efficacy of immune check point inhibitors (22, 25, and 26). Especially, a specific enteric bacterium, *Akkermansia muciniphila,* and diversity of gut flora are reported to correlate with the responsiveness to PD-1 blockade therapy (26). Although the detailed mechanism remains largely unknown, gut flora is believed to activate innate immunity and upregulate the baseline of anti-tumor immunity in the periphery (26-28). However, it is still elusive whether the above-mentioned microbiota or its associated factors could be responder biomarkers for PD-1 blockade therapy.

Recently, tight linkage between T cell energy metabolism and immune activity has been disclosed (29, 30). The present inventors have examined how energy metabolism in T cells regulates anti-tumor immunity (31, 32). From the results of measurement of oxygen consumption rate and dye staining of mitochondrial mass, reactive oxygen species (ROS) and membrane potential (these items are potential indicators for mitochondrial activity), the present inventors have found that during PD-1 blockade therapy, mitochondria in tumor-reactive T cells are activated. Furthermore, the present inventors showed that increase in mitochondria-derived ROS augments the efficacy of PD-1 blockade therapy and reported that this was mediated by peroxisome proliferator-activated receptor gamma coactivator 1-alpha (PGC-1α) activation (31). It had previously been known that the level of ROS signal was tightly linked to T cell activation and cytokine production (33). The present inventors therefore examined whether these mitochondria-related factors could be predictive biomarkers for host immune activity.

In the present study using blood samples from 60 NSCLC patients, the present inventors demonstrated that a combination of several plasma metabolites could serve as a good responder biomarker (AUC=0.91 by validation study using machine learning). These metabolites include those related to gut microbiota (hippuric acid), fatty acid oxidation

(butyrylcarnitine), and redox (cystine and glutathione disulfide). Further, the present inventors demonstrated that a combination of several T cell markers could serve as a better biomarker (AUC=0.96 by validation study using machine learning). These T cell markers were those related to suppressive function (the frequency of PD-1^{high} population) and mitochondrial activities (PGC-1α and ROS expression) in CD8⁺ killer T cells. These T cell markers strongly linked with the above-mentioned metabolite markers. These data indicate that a combination of gut microbiota and T cell energy metabolism could be a highly predictive biomarker for PD-1 blockade therapy.

### (Results)

Gut microbiota- and energy metabolism-related metabolites are correlating with responsiveness to PD-1 blockade cancer immunotherapy

Accumulated evidence indicates that there is a considerable association between immune responses and gut microbiota (30). However, it remains unknown whether specific metabolites can serve as predictive biomarkers for PD-1 blockade therapy in humans. In order to investigate how metabolites are associated with anti-tumor immunity, the present inventors analyzed metabolites and T cell functional markers in plasma and PBMC, respectively, in 60 NSLC patients before and after administration of Nivolumab (Fig. 1a and Tables 1-3). Blood samples were harvested immediately before the injection of Nivolumab at week 0, 2, and 4, and these samples were designated as the 1st, 2nd, and 3rd sample, respectively (Fig. 1a). The present inventors defined responsive and unresponsive patients based on the criteria of progression free survival (PFS) > 3 months or PFS ≤ 3 months according to the PFS data of phase III clinical studies of NSLC patients (Fig. 6) (34, 35). Although PFS > 6 months has often been used as a responder criterion, both criteria (PFS >3 months and PFS > 6 months) have given similar overall survival curves in the present study (Fig. 6a and b). Moreover, PD-L1 expression levels at tumor sites could not predict therapeutic efficacy clearly (Fig. 6c and d). The present inventors measured 247 metabolites from 60 patients. Of these patients, six dropped out and seven had to stop the therapy due to severe side effects, leaving data of 47 patients for analysis. Volcano plot analysis of these metabolites demonstrated that hippuric acid in the 1st sample, and hippuric acid, indoxyl sulfate, 4-cresol and glutathione disulfide (GSSG) in the 3rd sample were significantly elevated in responders compared to non-responders (Fig. 1b). On the other hand, the levels of α-ketoglutaric acid and butyrylcarnitine in the 3rd sample were significantly lower in responders. In the 2nd sample, no significant differences were observed between responders and non-responders (Fig. 1b). Hippuric acid, indoxyl sulfate and 4-cresol are reported to be almost exclusively produced by gut microbiota in mammals (36), which is consistent with the finding of the present study that patients treated with antibiotics within 3 months before the administration of Nivolumab had lower levels of these three metabolites (Fig. 7a). The above result that the levels of gut microbiota-derived metabolites were higher in responders than in non-responders suggests that gut microbiota-derived metabolites are related to antitumor immunity in the periphery (Fig. 1c and Fig. 7b). GSSG levels were significantly higher in responders than in non-responders, especially in the 3rd sample (Fig. 1b and 1d, left panel). GSSG is an oxidized form of glutathione (GSH), which controls the level of reactive oxygen species (ROS) appropriately in cells (37). Butyrylcarnitine levels were higher in non-responders than in responders (Fig. 1b and Fig. 1d). Butyrylcarnitine, the 4-carbon acyl-carnitine, serves as a fatty acid transporter to mitochondria to generate ATP. Acylcarnitine species with various numbers of carbon atoms are released from cells once the function of fatty acid oxidation (FAO) is attenuated (7-9).

It should be noted that butyrylcarnitine and other acylcarnitine species (isovalerylcarnitine and hexanoylcarnitine) showed a tendency to increase after treatment in non-responders (Fig. 7c). There was a tendency that the level of α-ketoglutaric acid was higher in non-responders than in responders (Fig. 1b and 1d). α-Ketoglutaric acid is a core metabolite in the tricarboxylic acid (TCA) cycle for ATP production in mitochondria, and is reduced in the blood as a result of consumption by activated T cells (31, 38). Therefore, these data indicate that anti-tumor immune responses by PD-1 blockade therapy are deeply linked to gut microbiota and energy metabolism.

### A combination of plasma metabolites can be a predictive biomarker.

The present inventors examined the probability for each of the predictive biomarker candidates listed in Fig. 1b by receiver operating characteristic curve (ROC) analysis with linear regression. However, the area under the curve (AUC) for each candidate was not high enough for clinical application (Fig. 1b, rightmost column in the table). Therefore, the present inventors decided to use stepwise regression to elucidate superior biomarkers based on a combination of metabolites. The present inventors first selected those items with a significant difference between responders and non-responders in their levels or in their ratio (fold change) of the different time points among 1482 items in total (247 items × 3 time points + 247 items × 3 ratios) (Table 4). The markers listed in Table 4 were analyzed with stepwise Akaike's information criterion (AIC) regression procedure. As a result, metabolite combinations I, II, and III which were most predictive in the 1st sample, 1st + 2nd samples and 1st + 2nd + 3rd samples, respectively, were obtained (Fig. 2a and Fig. 8a-c). Linear discrimination analysis (LDA) demonstrated that metabolite combination I distinguished between responders and non-responders at an error rate of 23% (Fig. 2b). To test the reliability of metabolite combination I, the present inventors assigned 47 samples to "responders (LDA-R)" or "non-responders (LDA-NR)" based on the LDA cut off value (Fig. 2b). As shown in Fig. 2c, LDA-R and LDA-NR discriminated by metabolite combination I showed a significant difference in PFS. Then, metabolite combination II better distinguished responders and non-responders at an error rate of 22% (Fig. 2d). Furthermore, LDA-R and LDA-NR discriminated by metabolite combination II showed a significant difference in both PFS and OS (Fig. 2e). Finally, metabolite combination III performed best in discriminating between responders and non-responders (error rate 19.6%) (Fig. 2f and 2g). The present inventors used machine learning to conduct a validation test using the same cohort, performing a five-fold cross validation with logistic regression. In the five-fold cross validation, the present inventors split the cohort into five folds, took the first 20% fold as test data, and trained the prediction model with the remaining 80% to predict the test. This procedure was iterated five times for each fold, and the model performance was evaluated with the mean of the AUC. This cross-validation assay gave mean AUC values of 0.77, 0.83, and 0.91 for the metabolite combinations I, II and III, respectively (Fig. 2h).

Considering that patients are administered Nivolumab every two weeks in the current clinical protocol for NSCLC, metabolite combinations I and II are useful as predictive biomarkers, while metabolite combination III may be less valuable for clinical use despite the highest reliability.

A combination of cellular markers including mitochondrial activities of CD8⁺ T cells can distinguish between responders and non-responders.

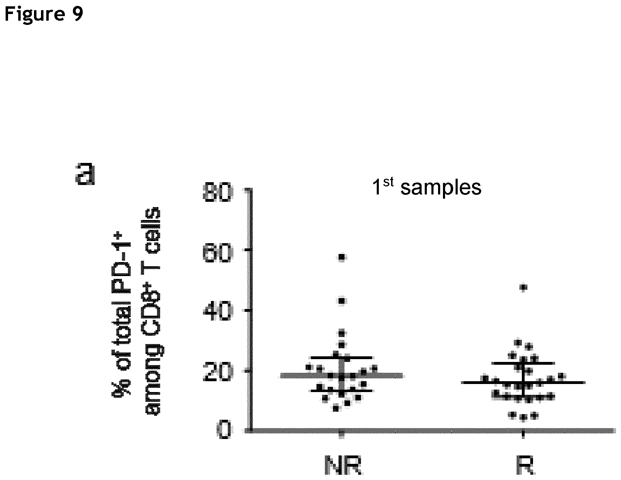

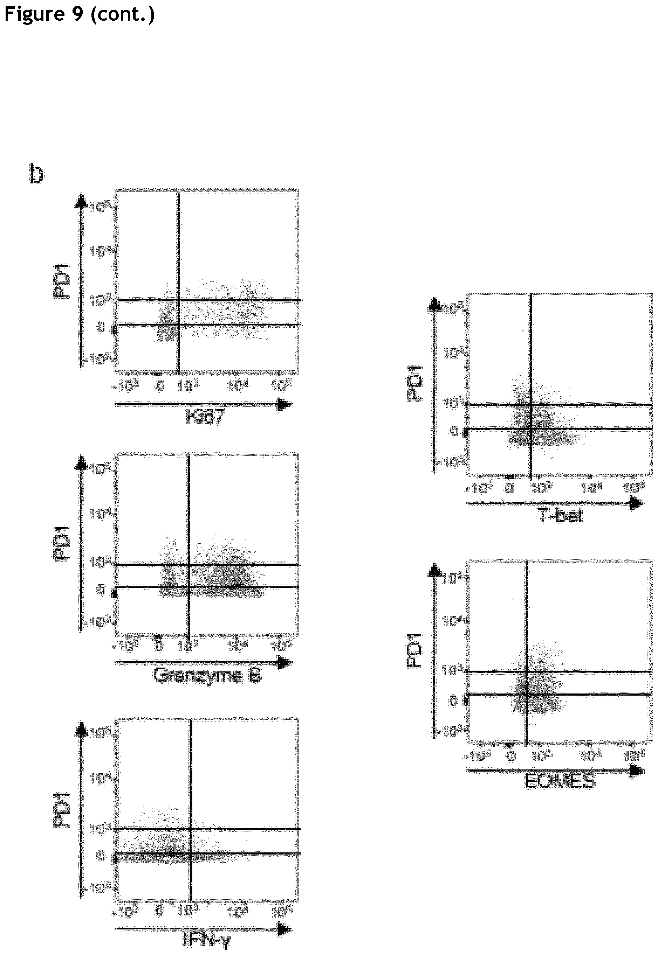

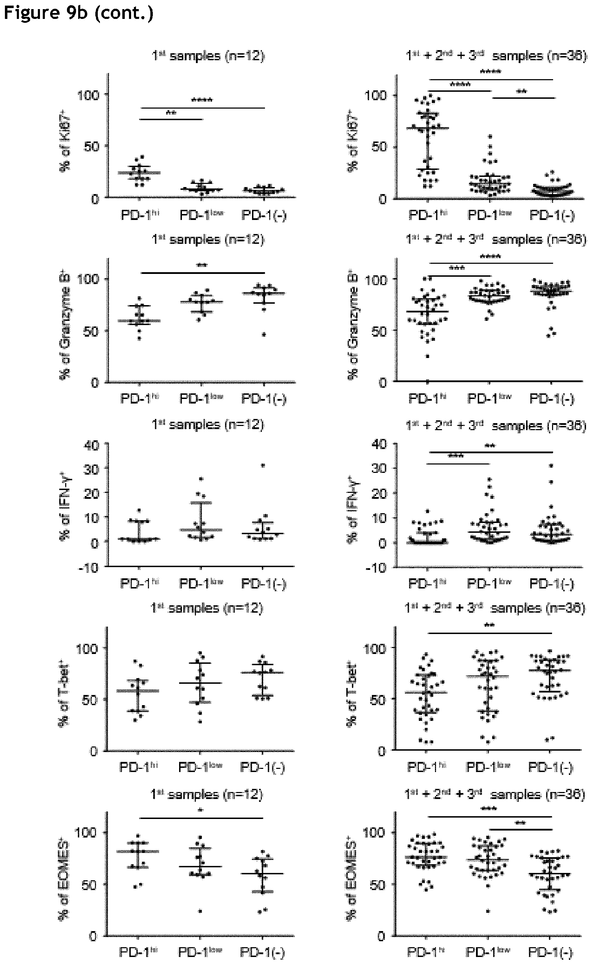

The present inventors' previous reports have shown that mitochondrial activation and energy metabolism in T cells are strongly associated with responses to PD-1 blockade therapy (31, 32). Therefore, the present inventors investigated cellular markers for effector function, energy metabolism, mitochondrial status, and immune activation in CD8⁺ T cells in patients' PBMC. Among the total 52 markers shown in Table 3 and their ratio (fold change) between each time point, the present inventors extracted 26 items with significant differences between responders and non-responders (Table 5). Further, from these 26 items, the best combinations of predictive biomarkers were selected using the stepwise regression method as described for metabolites. As shown in Fig. 3a, the best combinations to predict responders in the 1st sample, 1st + 2nd samples, and 1st + 2nd + 3rd samples were designated as cellular marker combination I, II, and III, respectively. These combinations contained two, four and four markers, respectively. The present inventors have found that the frequency of PD-1^{high} population among CD8⁺ T cells in the 1st sample is significantly low in responders and that this marker is contained in all the cellular marker combinations I, II and III (Fig. 3a and 3b). It should be noted that there is no significant difference between responders and non-responders in the frequency of total PD-1⁺ CD8⁺ T cells (Fig. 9a). In functional analysis, the PD-1^{high} CD8⁺ T cell population exhibited high proliferation capacity as the frequency of Ki-67 was higher than the other two groups (PD-1^{low} or PD-1⁻ CD8⁺ T cells). However, they produced less granzyme B and IFN-γ than the other two groups (Fig. 9b). In addition, T-bet expression was lower, but EOMES expression was higher in the PD-1^{high} CD8+ T cells than in the other two groups (Fig. 9b). These data suggest that PD-1^{high} CD8⁺ T cells may be "exhausted" through repeated division. In other words, less exhausted CD8⁺ T cells would be important for a robust anti-tumor response and for retaining the effector function. Mitochondrial ROS, which is measured by a dye called Mito SOX, is one of the mitochondrial activation indicators (31). The present inventors have found that the ratio of Mito SOX level in CD8⁺ T cells to the corresponding level in CD4⁺ T cells (Mito SOX CD8/CD4) in the 1st sample is significantly higher in responders, and that this marker, like PD-1^{high} CD8⁺ T cell marker, is shared by all marker combinations (Fig. 3a and 3c). These results suggest that higher mitochondrial activation status in CD8⁺ T cells than in CD4⁺ T cells before treatment is important for a better response to the treatment with the PD-1 antibody (Fig. 3c). PGC-1 is a master regulator of mitochondrial biogenesis and mitochondrial metabolic pathways, such as oxidative phosphorylation (OXPHOS) and fatty acid oxidation (FAO) (10, 11). The present inventors examined PGC-1 expression using mAb which recognizes both PGC-1α and PGC-1β (hereinafter, "PGC-1αβ"). While the PGC-1αβ expression in CD8⁺ T cells decreased in responders between the 1st and 2nd samples, it increased between the 2nd and 3rd samples (Fig. 3a and 3d upper panel). Therefore, the fold change of PGC-1αβ expression in 2nd relative to 1st samples was significantly lower in responders, but significantly higher in 2nd relative to 3rd samples (Fig. 3a and 3d lower panel). Although the temporal drop in PGC-1αβ expression in the 2nd responder samples might be due to the promotion of glycolysis through Akt signaling and temporal inhibition of OXPHOS (10, 31, 32, 39), it was found that this key mitochondrial regulator is recovered at least by the 3rd time point in the responders. Strong correlation of Mito SOX and PGC-1αβ markers with responsiveness to treatment suggested that mitochondrial activation in CD8⁺ T cells is important for better anti-tumor immune responses by PD-1 blockade, as shown in a previous mouse model (31, 32). Further, as other groups have already reported, the present inventors have also found that the frequency of CD4⁺ T cells was increased after Nivolumab treatment in responders (Fig. 3a and 3e) (40, 41). Further analysis revealed that Nivolumab treatment increased CD4⁺ CD45RO⁺ CCR7⁺ (central memory: Tcm) population in responders and decreased CD4⁺ CD45RO⁻ CCR7⁻(terminally differentiated effector memory CD45RA⁺ T cells: Temra) population (Fig. 9c).

A combination of T cell markers exhibited a high predictive value.

The present inventors assessed the error rates of cellular marker combinations I, II, and III by the method described above. LDA demonstrated clear separation between responders and non-responders at an error rate of 19.1% by cellular marker combination I (Fig. 4a). When the present inventors defined responders and non-responders based on the LDA criteria as done in the metabolite markers, LDA-R and LDA-NR discriminated by cellular marker combination I showed a significant difference in both PFS and OS (Fig. 4b). Further, as a result of LDA, cellular marker combinations II and III both showed an error rate of 4.3%, and LDA-R and LDA-NR showed a significant difference (p < 0.01) in both PFS and OS (Fig. 4c-f). A validation test using five-fold cross-validation with logistic regression within the same cohort gave mean AUCs of 0.85, 0.96, and 0.93 for cellular marker combinations I, II, and III, respectively (Fig. 4g). These data demonstrate that combinations of cellular biomarkers obtained by the 1st and 2nd treatments (administrations) are sufficient to discriminate between responders and non-responders.

Metabolites and cellular markers are correlated.

Subsequently, the present inventors conducted the stepwise regression method to select the best combination of markers among the total metabolic and cellular markers showing significant differences between responders and non-responders (Table 4 and Table 5). Surprisingly, all selected markers were cellular markers, resulting in the same combinations as shown in Fig. 3 and Fig. 4. Therefore, the present inventors hypothesized that there might be a strong linkage between responsible metabolite and cellular markers, which might lead to exclude metabolite markers. Since cellular marker combination II shows excellent discrimination capacity and is practical for clinical use as described above, the present inventors focused on cellular marker combination II, and investigated the correlation between the cellular markers and metabolites. The spearman correlation coefficients (r) were used to evaluate the correlation between the cellular markers and metabolite markers. Generally, |r| of greater than 0.4 is considered to have relatively strong correlation. The present inventors have found that PGC-1αβ expression in CD8⁺ T cells is correlated with gut microbiota-related metabolites, that the frequency of PD-1^{high} CD8⁺ T cells is correlated with FAO-related metabolites, and that the T cell Mito SOX marker is correlated with redox-related metabolites (Fig. 5a). Note that cystine and pyroglutamic acid are components of glutathione as shown in Fig. 10.

As a result of cluster analysis, cellular markers and metabolites were roughly divided into three groups (Fig. 5b). Metabolites could be classified into three groups of 1) gut microbiota-related metabolites, 2) FAO-related metabolites and 3) redox-related metabolites. Details of the correlation between cellular markers and metabolite markers are summarized in Fig. 5c. In conclusion, it was believed that stepwise regression analysis of all markers completely excluded the responsible metabolite markers because they were closely linked with particular cellular markers that have slightly higher predictive capacity than the metabolite markers. The current data support a strong linkage between gut microbiota and T cell energy metabolism, both of which contribute to the power of anti-tumor immunity and responsiveness to PD-1 blockade immunotherapy.

### (Discussion)

Anti-tumor immunity is regulated by both tumor and immune activity. As shown in this study, the functional activity of killer T cells is related to a complex network of different high-order function systems such as gut microbiome and energy metabolism. Therefore, a single biomarker is not sufficient, and a combination of multi-markers is required to improve the accuracy of prediction. Current clinical biomarkers approved by the FDA are PD-L1 high expression in NSCLC, cervical cancer, esophageal cancer, etc. and MSI-H and/or dMMR for various solid tumors. However, a significant portion of NSCLC patients with PD-L1 low expression is responsive and the number of patients with MSI-H and/or dMMR on their tumor is very limited (around 1 % of NSCLC patients) (35, 42). Considering that the expression frequency of PD-L1 in tumor could not significantly discriminate responders from non-responders in this study, currently approved biomarkers based solely on tumor properties are incomplete as predictive biomarkers for PD-1 blockade therapy. For investigating tumor factors, at least biopsy specimens are needed, which usually puts a huge burden on patients. On the other hand, blood-based tests to examine immune cell properties and metabolites would be much simpler and more patient-friendly. Although it has been reported that several markers associated with host immunity (such as the frequency of eosinophils, neutrophils, tumor-associated macrophages, CD4⁺ T cells in PBMC, and the frequency of infiltrated CD8⁺ T cells or PD-1⁺ CD8⁺ T cells in tumor sites) are related to responsiveness, these single immune-related markers are insufficient in terms of prediction accuracy (22, 43). In this study, the present inventors demonstrated for the first time that the combination of several cellular markers for T cell activation, including mitochondrial activation status in T cells, can discriminate responders from non-responders with high accuracy; this has led to the finding that the combination has a potential to serve as a good biomarker. Correlation analyses suggested that metabolites associated with gut microbiota, energy metabolism and redox are reflecting the anti-tumor activation status of peripheral killer T cells.

It has been known that the host immune activity and gut microbiota affect each other (23). Further, gut microbiota is known to be associated with various diseases, and the relationship between gut microbiota-derived blood metabolites and disease etiology has been studied (5, 44, 45). In the present study, it is noticeable that gut microbiota-derived metabolites are correlated with the behavior of PGC-1αβ, a master molecule for mitochondrial regulation in the peripheral CD8⁺ T cells (10, 11). It has been reported that hippuric acid, one of the microbiota-derived metabolites, can be an indicator of gut microbiota diversity and is produced abundantly by Clostridiales bacteria (4, 5). Recent studies have reported that the diversity of gut microbiota and the abundance of Clostridiales bacteria are correlated with responsiveness to PD-1 blockade therapy (25). The present study demonstrated that hippuric acid and other gut microbiota-derived metabolites show higher levels in responders. This may reflect increase of specific enterobacterial species which activate the immune system and the diversity of gut microbiota. Although the mechanism by which gut microbiota regulates anti-tumor immunity still remains largely unknown (26), the results of the present study suggest that gut microbiota and microbiota-derived metabolites are deeply correlated with the mitochondrial activation status of peripheral CD8⁺ T cells.

Changes in acylcarnitine levels showed correlation with the frequency of PD-1^{high} CD8⁺ T cells. Acylcarnitines are converted from short-chain acyl-CoAs and carnitine by carnitine-dependent enzymes, carnitine acetyltransferase and carnitine palmitory transferase I (CPT1), which are localized in the mitochondrial matrix. In other words, carnitine serves as a shuttle to transfer acyl-CoAs from the cytoplasm to the inside of mitochondria (7-9). When FAO is promoted, acylcarnitines are substantially transported into the mitochondrial matrix, resulting in decrease in plasma acylcarnitine levels (7-9, 46, 47). Therefore, it is held that plasma levels of acylcarnitine species could be an indicator for mitochondrial activity or FAO under inflammatory reaction (48-50). In the present study, acylcarnitines (butyrylcarnitine, isovalerylcarnitine, and hexanoylcarnitine) were elevated in non-responders after treatment with PD-1 antibody, suggesting decrease in FAO in killer T cells (47). Higher acylcarnitine levels and higher frequency of PD-1^{high} CD8⁺ T cells were observed in non-responders. These two markers were correlated with each other. This indicates the presence of a large number of exhausted killer T cells with reduced FAO in non-responders. These results are consistent with a previous report that FAO pathway in effector killer T cells is important for robust anti-tumor activity by PD-1 blockade (32).

Mitochondrial ROS upregulation is critical for T cell activation and cytokine production (31, 51). However, since an extremely high concentration of ROS is toxic to cells, they control ROS levels appropriately using the GSH/GSSG system (37). Oxidized GSH (GSSG) is extracellularly released into the plasma via a complex efflux system (52, 53). Therefore, it is known that plasma GSSG level is an indicator of cellular oxidation status and various immune-related diseases (52, 53). In the present study, responders saw an increase in mitochondrial ROS in killer T cells before treatment, and GSSG and GSH components (cystine and pyroglutamic acid) in responder plasma after treatment showed high levels. The correlation between these markers can be explained by ROS and the mechanism of the GSH/GSSG system (37).

To date, it has been believed that PD-1^{high} CD8⁺ T cells in PBMC contain tumor-specific T cells because their Ki-67 frequency is extremely high (54, 55). In the present study, production of granzyme B and IFN-γ was decreased in PD-1^{high} CD8⁺ T cells. This suggests that they are exhausted as a result of vigorous division. Indeed, PD-1^{high} CD8⁺ T cells expressed less T-bet, but expressed the exhaustion marker EOMES highly. Although it has been reported that PD-1^{high} CD8⁺ T cells are abundant in responder tumor tissues (56), the results of the present study show that PD-1^{high} CD8⁺ T cells are not abundant in the peripheral blood of responders. This discrepancy may be attributed to 1) the number of tumor-reactive CD8⁺ T cells in the periphery being less in responders because such T cells preferentially move to tumor sites, and 2) the function and the extent of exhaustion of PD-1^{high} CD8⁺ T cells being different between tumor sites and peripheral blood. Further analysis is necessary to clarify the reasons behind this discrepancy.

The results of the present study showed that among all metabolites and cellular makers tested, a combination of several cellular markers can provide the highest prediction accuracy. Metabolites which are capable of predicting therapeutic efficacy are strongly correlated with the cellular markers. However, considering the convenience of measuring specific metabolites and the difficulties involved in achieving stable measurements of cellular markers between different facilities, a combination of particular metabolites might be more suitable for clinical application. According to the present study, practical use of combinatorial biomarkers for cancer immunotherapy has been suggested. Through establishment of such biomarkers, those patients who are non-responsive to anti-PD-1 antibody monotherapy would be provided an opportunity to select different therapeutic options. As a consequence, enhancement of therapeutic effect can be expected.

### Methods

### Study Design and Participants

Among NSCLC patients receiving Nivolumab (anti-PD-1 antibody) at Kyoto University Hospital, those who consented to the collection and storage of blood samples during treatment, and allowed review of their medical records for past medical history, cancer tumor type, toxicity assessments, clinical response, survival, and laboratory data were selected as subj ects. The present study was approved by the Ethics Committee of Kyoto University. The present inventors enrolled 60 patients with NSCLC, all of which had previously received other chemotherapy. Patients received Nivolumab (3 mg/kg) every 2 weeks until disease progression or the emergence of an unacceptable side effect. Blood samples were collected immediately before the 1st, 2nd and 3rd Nivolumab administration. Among the 60 patients enrolled, 6 patients dropped out and 7 patients had to cancel the therapy due to severe side effect, leaving the data of 47 patients for analysis. Tumor size was measured by CT and evaluated for response using Response Evaluation Criteria in Solid Tumors 1.1 (RECIST 1.1).

For functional analysis of PD-1^{high} CD8⁺ T cell population, the present inventors enrolled 12 other patients with NSCLC receiving Nivolumab or Pembrolizumab.

### Sample preparation for plasma metabolome measurement

Peripheral blood samples were collected in 7 ml vacutainers containing EDTA (Venoject II, VP-NA070K, Terumo, Tokyo, Japan), immediately stored in a CubeCooler (Forte Grow Medical Co. Ltd., Tochigi, Japan) and kept at 4 °C until centrifugation at 4 °C at 3000 rpm for 15 min. All the harvested plasma samples were then stored at -80 °C until analysis. For GCMS analysis, 50 µl of plasma was mixed with 256 µl of a solvent mixture (methanol: water: chloroform = 2.5:1:1) containing 2.34 µg/ml of 2-isopropylmalic acid (Sigma-Aldrich), which was utilized as an internal standard. The obtained mixture was shaken at 1200 rpm for 30 min at 37 °C (Maximizer MBR-022UP, Taitec). After centrifugation at 16000 × g for 5 min at 25 °C, 150 µl of supernatant was collected and mixed with 140 µl of purified water followed by vortex mixing for 5 s. After centrifugation at 16,000 × g for 5 min at 25 °C, 180 µl of supernatant was dried in a centrifugal evaporator (CVE-3100, Tokyo Rikakikai Co. Ltd.). The dried sample was dissolved in 80 µl of methoxyamine solution (20 mg/ml in pyridine, Sigma-Aldrich) and shaken at 1,200 rpm for 30 min at 37 °C. Forty microliters of N-methyl-N-trimethylsilyltrifluoroacetamide solution (GL science) were added for trimethylsilyl derivatization, followed by agitation at 1,200 rpm for 30 min at 37 °C. After centrifugation, 50 µl of supernatant was transferred to a glass vial and subjected to GCMS measurement. For LCMS analysis, the metabolite extraction protocol was slightly changed. Fifty microliters of plasma was mixed with 256 µl of methanol and shaken at 1200 rpm for 10 min at 37 °C. After centrifugation at 16,000 × g for 30 min at 25 °C, 150 µl of supernatant was mixed with 90 µl of 1% acetic acid in water and 120 µl of chloroform, followed by a vortex mixing for 15 s. After centrifugation at 2,000 × g for 10 min at 25 °C, 150 µl of the upper layer was dried and solubilized in 50 µl of 0.1% formic acid in water, and then subjected to LCMS analysis.

### Plasma metabolite analysis

GCMS analysis was performed with a GCMS-QP2010 Ultra (Shimadzu). The derivatized metabolites were separated on a DB-5 column (30 m × 0.25 mm id, film thickness 1.0 µm, Agilent Technologies). The helium carrier gas was set at a flow rate of 39 cm/sec. The inlet temperature was 280 °C and the column temperature was first held at 80 °C for 2 min, then raised at a rate of 15 °C/min to 330 °C, and held for 6 min.

GC-MS measurement was performed in scan mode. Metabolite peaks were identified by agreement with spectrum library and semi-quantitated using internal standards. One microliter of the sample was injected into the GCMS in the split mode (split ratio 1:3). Mass spectra were obtained under the following conditions: electron ionization (ionization voltage 70 eV), ion source temperature 200 °C, full scan mode in the range of m/z 85-500, scan rate 0.3 sec/scan. Identification of chromatographic peaks was performed using the NIST library or Shimadzu GC/MS database, and further confirmed with authentic commercial standards. For semi-quantitative analysis, the area of each metabolite peak was calculated and divided by the area of the internal standard peak. LC separation was conducted on a Shim-pack GIST C18-AQ column (3 µm, 150 mm × 2.1 mm id, Shimadzu GLC) with a Nexera UHPLC system (Shimadzu). The mobile phase consisted of 0.1% formic acid in water (A) and 0.1% formic acid in acetonitrile (B). The gradient program was as follows: 0-3 min, 0% B; 3-15 min, linear gradient to 60% B; 15-17.5 min, 95% B; 17.5-20 min, linear gradient to 0% B; hold for 4 min; flow rate, 0.2 ml/min. The column oven temperature was maintained at 40 °C. The LC system was coupled with a triple-quadruple mass spectrometer LCMS-8060 (Shimadzu). LCMS-8060 was operated with the electrospray ionization and multiple reaction monitoring mode. All ion transitions and collision energies were optimized experimentally by using authentic standards of each metabolite. Three microliters of the sample were injected into the CLAMS system. Quality control (QC, a pooled plasma) samples were subjected to the same preparation protocol, and every 10 and 5 samples were injected for GEMS and CLAMS analysis, respectively. Each metabolite's signals were normalized with a QC-based correction method using the smooth-spline algorithm (57-59). Information on all the measured metabolites, including retention time, m/z, and ion transitions was summarized in Tables 1 and 2.

### Flow Cytometry

PBMCs were isolated from blood by Ficoll density gradient centrifugation. PBMCs were immediately stained using the following antibodies: anti-CD8a (RPA-T8), -CD8 (SKI), -CD4 (RPA-T4, SK3), -CD45RA (HI100), -CD45RO (UCHL1), -CCR7 (3D12), -PD-1 (EH12.2H7), -Tim3 (F38-2E2), -KLRG1 (13F12F2), -CD25 (BC96), -CXCR3 (G025H7), - CCR6 (G034E3), -T-bet (4B10), -EOMES (WD1928), -Ki-67(SolA15), -CTLA-4 (BNI3), -p-mTOR (MRRBY), -p-Akt1(Ser473) (SDRNR), -granzyme B (GB11), -IFN-γ (4S.B3), and - FOXP3 (236A/E7). PGC-1 expression was detected with anti-PGC-1αβ (rabbit polyclonal, Abcum, ab72230) which recognizes both PGC-1α and PGC-1β, followed by secondary staining with goat anti-rabbit IgG (Santa Cruz Biotech, sc-3739). Dead cell discrimination was performed using 7-AAD staining solution (TONBO, 13-6993). Intracellular staining was performed using a FOXP3 Fixation Kit (eBioscience). For staining intracellular phosphoproteins, cells were permeabilized with 0.5% Triton-X and fixed with 1.5% paraformaldehyde before staining. Samples were measured on BD Canto II flow cytometer (BD Bioscience). Data were collected using BD FACS Diva Software version 6.1.3 and further analyzed with FlowJo 10.4 (Tree Star Inc.). For data analysis, data were gated on live (7AAD negative) and single cells. Measurement of mitochondrial mass, membrane potential, mitochondrial superoxide, and cellular ROS were performed using MitoTracker Green, MitoTracker Deep Red, MitoSOX Red, and CellROX Green reagents, respectively (all from Life Technologies). These dyes were added to cells, which were then incubated at 37 °C in a 5% CO₂ humidified incubator for 30 min, followed by surface staining. As regards samples after Nivolumab administration, anti-PD-1 (EH12.2H7, APC-conjugated) antibody was added to cells, which were then incubated at 37 °C in a 5% CO₂ humidified incubator for 60 min, followed by other surface staining.

### Statistical Analysis

Data are shown as the median and interquartile range. For comparison of two groups, a Wilcoxon rank sum test was conducted. For comparison between multiple groups, a Kruskal-Wallis test followed by Dunn's test for multiple comparison was conducted. The stepwise AIC regression procedure was performed to select the best marker combination. Then, LDA was performed by using estimated biomarker combination to predict the reliability. The prediction model was evaluated with five-fold cross validation. The survival rates of different groups of patients were calculated with the Kaplan-Meier method and presented graphically as survival curves. Comparison of survival curves between two groups was analyzed by Gehan-Breslow-Wilcoxon test. The Spearman correlation coefficient was used to calculate the association between cellular markers and metabolite markers. JMP software (Version 12.0.0; SAS Institute Inc.; Cary, NC, USA), R software (Version 3.4.4), DataRobot (Version 4.3.0) and Prism software (Version 6.0h; GraphPad Software) were used for data management and statistical analyses. Significance level was set at 0.05 for all tests.

**Table 1**

| Metabolites meas | ured by | GC-MS. | | | | | | | | |
|---|---|---|---|---|---|---|---|---|---|---|
| **Name** | **Kegg lD** | **HMDB ID** | **Methyloxime/TMS derivate** | **m/z Retention time(min)** | **Name** | **Kegg ID** | **HMDB ID** | **Methyloxime/TMS derivative** | **m/z** | **Retention time(min)** |
| 1,5-Anhydro-D-sorbitol | C07326 | HMDB0002712 | 1,5-Anhydro-O-sorbitol-4TMS | 259.0 14.77 | Hypotaurine | C00519 | HMDB0000965 | Hypotaurine-3TMS | 188.1 | 12.92 |
| 1,6-Anhidroglucose | | HMDB0000640 | 1,6-Anhydro-beta-O-glucose-3TMS | 204.0 13.70 | Hypoxenthine | C00252 | HMDB0000157 | Hypoxanthine-2TMS | 265.0 | 14.50 |
| 1-Hexadecanol | C00323 | HMDB0003424 | 1-Hexadecanol-TMS | 299.0 15.37 | Indoleacetic acid | C00954 | HMDB0000197 | Indol-3-acetic acid-2TMS | 202.0 15.68 | |
| 2-Aminoadipic acid | C00856 | HMDB0000510 | 2-Aminoadipic acid-3TMS | 280.0 13.62 | Indolelactic acid | C02043 | HMDB0000671 | Indolelactic acid-3TMS | 202.0 | 17.08 |
| 2-Aminobutyric acid | C02356 | HMDB0000452 | 2-Aminobutyric acid-2TMS | 130.0 8.69 | Indoxyl sulfate | | HMDB0000682 | Indoxyl sulfate-2TMS | 277.0 | 13.84 |
| 2-Aminoethanol | C00189 | HMDB80000149 | 2-Aminoethanol-3TMS | 174.0 9.77 | Isocitric acid | C00311 | HMDB0000193 | lsocitric acid-4TSM | 245.0 | 14.41 |
| 2-Deoxytetronic acid | | HMDB0000337 | 2-Deoxytetronic acid-3TSM | 321.0 11.20 | Isoleucine | C16434 | HMDB0000172 | Isoleucine-2TSM | 232.0 | 9.99 |
| 2-Hydroxybutyric acid | C05984 | HMDB0000008 | 2-Hydroxybutyric acid-2TMS | 131.0 3.17 | Kynurenine | C00328 | HMDB0000684 | Kynurenine-3TSM | 307.0 | 17.07 |
| 2-Hidroxyisobutyric acid | | HMDB0000729 | 2-Hydroxyisobutyric acid-2TSM | 131.0 7.46 | Lactic acid | C00186 | HMDB0000190 | Lactic acid-2TSM | 219.0 | 7.39 |
| 2-Hydroxyisovaleric acid | | HMDB0000407 | 2-Hidroxyisovaleric acid-2TSM | 145.0 3.61 | Lauric acid | C02679 | HMDB0000638 | Lauric acid-TSM | 257.0 | 13.11 |
| 2-Hydroxypyridine | C02502 | HMDB0013751 | 2-Hidroxypyridine-TMS | 152.0 7.22 | Leucine | C00123 | HMDB0000687 | Leucine-2TMS | 232.0 | 9.74 |
| 2-Oxobutyric acid | C00109 | HMDB0000005 | 2-Oxobutyric acid methyloxime-TMS | 188.0 7.86 | Linoleic acid | C01595 | HMDB0000673 | Linoleic acid-TMS | 337.0 | 17.06 |
| 2-Oxoglutaric acid (α-Ketoglutaric acid) | C00026 | HMBD0000208 | 2-Oxoglutaric acid-methiloxime-2TMS | 198.0 12.47 | Lysine | C00047 | HMDB0000182 | Lysine-4TMS | 174.0 | 15.23 |
| 2-Oxoisocaproic acid | C00233 | HMBD0000695 | 2-Oxoisocaproic acid-methyloxime-TMS | 200.0 9.10 | Malic acid | C00711 | HMDB0000156 | Malic acid-3TSM | 233.0 | 11.73 |
| 3-(3-Hydroxyphenyl)-3- hydroxypropionic acid | | HMDB0002643 | 3-(3-Hydroxyphenyl)-3-hydroxypropionic acid-3TMS | 267.0 14.68 | Maltose Mannose | C00208 C00159 | HMDB0000163 HMDB0000169 | Maltose-methyloxime-8T MS Mannose-methyloxime-5TMS | 204.0 319.0 | 20.64 15.05 |
| 3-Aminoisobutyric acid | C05145 | HMDB0003911 | 3-Aminoisobutyric acid-3TMS | 304.0 11.67 | Margaric acid | | HMDB0002259 | Margaric acid-TMS | 117.0 | 16.58 |
| 3-Hydroxybutyric acid | C01069 | HMDB0000357 | 3-Hydroxybutyric acid-2TMS | 191.0 8.51 | Methionine | C00073 | HMDB0000696 | Methionine-2TMS | 250.0 | 12.14 |
| 3-Hydroxyisobutyric acid | C05001 | HMDB0000023 | 3-Hydroxyisobutyric acid-2TMS | 177.0 8.51 | myo-Inositol | C00137 | HMDB0000211 | myo-inositol-6TMS | 432.0 | 16.54 |
| 3-Hidroxyisovaloric acid | | HMDB0000754 | 3-Hidroxyisovaloric acid-2TSM | 131.0 9.09 | Miristic acid | C00424 | HMDB0000806 | Myristic acid-TMS | 285.0 | 14.60 |
| 3-Indolepropionic acid | | HMDB0002302 | 3-Indolepropionic acid-2TMS | 202.0 16.50 | Octanoic acid | C05423 | HMDB0000482 | Octanoic acid-TSM | 201.0 | 9.62 |
| 3-Methyl-2-oxobutyric acid | C00141 | HMDB0000019 | 3-Methyl-2-oxobutyric acid-methyloyme-TMS | 89.0 7.96 | Oleic acid | C00712 | HMDB0000207 | Oleic acid-TSM | 339.0 | 17.08 |
| | | | 3-Methyl-2-oxovaleric acid- | | O-Phosphoethanolamine | C00346 | HMDB0000224 | O-Phosphoethanolarnine-4TSM | 299.0 | 14.29 |
| 3-Methyl-2-oxovaleric acid | C00671 | HMDB0000491 | methiloxime-TMS | 200.0 5.97 | Ornithine | C00077 | HMDB0000214 | Omithine-4TMS | 420.0 | 14.52 |
| 3-Methylhistidine | C01152 | HMDB0000479 | 3-Methilhistidine-2TMS | 168.0 15.01 | Oxalic acid | C00209 | HMDB0002329 | Oxalic acid-2TMS | 219.0 | 8.13 |
| 4-Cresol | C01468 | HMDB0001853 | 4-Cresol-TMS | 180.0 8.56 | Palmitic acid | C00249 | HMDB0000220 | Palmitic acid TMS | 313.0 | 15.95 |
| 4-Hydroxyproline | C01157 | HMDB0000725 | 4-Hydroxyproline-3TMS | 140.0 12.15 | Palmitoleic acid | C08362 | HMDB0003229 | Palmitoleic acid-TMS | 311.0 | 15.85 |
| Acetoacetic acid | C00164 | HMDB0000050 | Acetoacetic acid-methyloxine-TMS | 203.0 8.01 | Paraxanthine | C13747 | HMDB0001860 | Paraxanthine-TMS | 237.0 | 15.77 |
| Aconitic acid | C02341 | HMDB0000958 | Aconitic acid-3TMS | 375.0 13.81 | Phenol | C00146 | HMDB0000228 | Phenol-TMS | 166.0 | 7.45 |
| Alanine | C00041 | HMDB0000161 | Alanine-2TMS | 218.0 7.91 | Phenylalanine | C00079 | HMDB0000159 | Phenylanine-2TMS | 192.0 | 13.12 |
| Alanine | C00041 | HMDB0000161 | Alanine-3TMS | 188.0 10.77 | Phosphoglycerol | C03189 | HMDB0000126 | Phosphogycerol-4TMS | 445.0 | 14.00 |
| Allose | C01487 | HMDB0001151 | Allose-methyloxime-5TMS | 205.0 15.00 | Phosphoric acid | C00009 | HMDB0002142 | Phosphoric acid-3TSM | 225.0 | 2.76 |
| Arabinonic acid | | HMDB0000539 | Arabinonic acid-5TMS | 292.0 14.23 | Pipecolinic acid | C00408 | HMDB0000070 | Pipecolinic acid-TSM | 156.0 | 10.44 |
| Arabinose | C00259 | HMDB0000645 | Arabinose-methyloxime-4TMS | 307.0 13.26 | Proline | C00148 | HMDB0000162 | Proline-2TMS | 216.0 | 10.12 |
| Arabitol | C01904 | HMDB0000558 | Arabitol-5TMS | 217.0 13.75 | Pyroglutamic acid | C01879 | HMDB0000267 | 5-Oxoproline-2TMS | 256.0 | 12.21 |
| Asparagine | C00152 | HMDB0000168 | Asparagine-3TMS | 231.0 13.32 | Pyrophosphate | | HMDB0000250 | Pyrophosphate-4TMS | 451.0 | 13.32 |
| Aspartic acid | C00049 | HMDB0000191 | Aspartic acid-3TMS | 232.0 12.03 | Pyruvic acid | C00022 | HMDB0000243 | Pyruvic acid-methyloxime-TMS | 174.0 | 7.23 |
| Benzoic acid | C00180 | HMDB0001870 | Banzoic acid-TMS | 179.0 9.60 | Ribitol | C00474 | HMDB0000508 | Ribitol-5TMS | 319.0 | 13.81 |
| beta-Alanine | C00089 | HMDB0000056 | beta-Alanine-3TMS | 290.0 11,31 | Ribose | C00121 | HMDB0000283 | Ribose-methyloxime-4TMS | 307.0 | 13.38 |
| Boric acid | C12486 | HMDB0035731 | Boric acid-3TMS | 263.0 8.49 | Ribulose | C00309 | HMDB0000621 | Ribulose-methyloxime-4TMS | 263.0 | 13.38 |
| Caffeina | C07481 | HMDB0001847 | Caffeine | 194.0 15.05 | scylla-inositol | C06153 | HMDB0006068 | scyllo-Inositol-5TMS | 318.0 | 16.11 |
| Caproic acid | C01565 | HMDB0000535 | Caproic acid-TMS | 173.0 7.52 | Serine | C00065 | HMDB0000187 | Serine-3TMS | 306.0 | 10.58 |
| Citric acid | C00158 | HMDB0000004 | Citric acid-4TMS | 363.0 14.45 | Stearic acid | C01530 | HMDB0000827 | Stearic-TMS | 341.0 | 17.18 |
| Citrulline | C00327 | HMDB0000904 | Citrulline-4TMS | 256.0 14.48 | Succinic acid | C00042 | HMDB0000254 | Succinic acid-2TMS | 247.0 10.10 | |
| Creatinine | C00791 | HMDB0000562 | Creatinine-3TMS | 115.0 12.58 | Sucrose | C00089 | HMDB0000258 | Sucrose-8TMS | 361.0 | 19.79 |
| Cysteine | C00097 | HMDB0000574 | Cysteine-3TMS | 218.0 12.44 | Taurine | C00245 | HMDB0000251 | 2-Aminoethanesulfonic acid-3TMS | 326.0 | 13.50 |
| Cysteinylglycine | C01419 | HMDB0000078 | Cysteinylglycine-3TMS | 220.0 16.14 | Threitol | C16884 | HMDB0004138 | Thereitol-4TMS | 217.0 | 11.84 |
| Cystine | C00491 | HMDB0000192 | Cystine-4TMS | 411.0 17,67 | Threonic acid | C01620 | HMDB0000943 | Threonic acid-4TMS | 292.0 12.38 | |
| Decanoic acid | C01571 | HMDB0000511 | Decanoic acid-TMS | 229.0 11.47 | Threonine | C00188 | HMDB0000167 | Threonine-3TMS | 201.0 | 10.86 |
| Elaidic acid | C01712 | HMDB0000573 | Elaidic acid-TMS | 93.0 17.11 | Tryptophan | C00078 | HMDB0000929 | Tryptophan-3TMS | 291.0 | 17.34 |
| Erithritol | C00503 | HMDB0002994 | Erythritol-4TMS | 217.0 11.92 | Tyrosine | C00082 | HMDB0000158 | Tyrosine-3TMS | 382.0 | 15.39 |
| Fructose | C00095 | HMDB0000660 | Fructose-methyloxime-5TMS | 307.0 14.92 | Unk12* | - | - | - | 196.0 | 14.50 |
| Fucose | C01019 | HMDB0000174 | Fucose-methyloxime-5TMS | 117.0 13.89 | Unk3* | - | - | - | 170.0 | 8.98 |
| Fumaric acid | C00122 | HMDB0000134 | Fumaric acid-2TMS | 245.0 10.37 | Unk4* | - | - | - | 319.0 | 9.56 |
| Gluconic acid | C00257 | HMDB0000625 | Gluconic acid-methyloxime-5TMS | 292.0 15.94 | Unk6* | - | - | - | 320.0 | 11.66 |
| Glucose | C00031 | HMDB0000122 | Glucose-methyloxime-5TMS | 229.0 15.32 | Unk8* | - | - | - | 116.0 | 13.96 |
| Glucuronic acid | C00191 | HMDB0000127 | Glucuronic acid-methyloxime-5TMS | 423.0 15.47 | Unk9* | - | - | - | 217.0 | 14.04 |
| Glutamic acid | C00025 | HMDB0000148 | Glutamic acid-3TMS | 246.0 12.85 | Urea | C00086 | HMDB0000294 | Urea-2TMS | 204.0 | 9.29 |
| Glutamine | C00064 | HMDB0000641 | Glutamine-3TMS | 362.0 14.11 | Uric acid | C00366 | HMDB0000289 | Uric acid-4TMS | 367.0 | 16.40 |
| Glutaric acid | C00430 | HMDB0000661 | Glutaric acid-2TMS | 261.0 10.94 | Uridine | C00299 | HMDB0000296 | Uridine-4TMS | 245.0 | 18.54 |
| Glyceric acid | C00258 | HMDB0000139 | Glyceric acid-3TSM | 292.0 10.28 | Valine | C00183 | HMDB0000883 | Valine-2TMS | 218.0 | 9.19 |
| Glycerol | C00116 | HMDB0000131 | Glycerol-3TMS | 218.0 9.69 | Xanthine | C00385 | HMDB0000292 | Xanthine-3TMS | 353.0 | 15.92 |
| Glycine | C00037 | HMDB0000123 | Glycine-3TMS | 174.0 10.20 | Xylitol | C00379 | HMDB0002917 | Xylitol-5TMS | 217.0 | 13.66 |
| Glycolic acid | C00160 | HMDB0000115 | Glycolic acid-2TMS | 205.0 7.53 | Xylose | C00181 | HMDB0000098 | Xylose-methyloxime-4TMS | 307.0 | 13.21 |
| Hippuric acid | C01586 | HMDB0000714 | Hippuric acid-TMS | 236.0 14.75 | | | | | | |
| Histidine | C00135 | HMDB0000177 | Histidine-3TMS | 371.0 15.28 | | | | | | |
| Homocysteine | C00155 | HMDB0000742 | Homocysteine-3TMS | 234.0 13.33 | | | | | | |
| * 'Unk' means | that the | metabolites | were not yet identified | at the i | time of analysis. | | | | | |

**Table 2**

| Metabolites measured by LC-MS. | | | | | | | | | |
|---|---|---|---|---|---|---|---|---|---|
| **Name** | **Kegg ID** | **HMDB ID** | **Ion transition** | **Retention time(min)** | **Name** | **Kegg ID** | **HMDB ID** | **Ion transition** | **Retention time(min)** |
| 4-Hydroxyproline | C01157 | HMDB0000725 | 132.10>68.05 | 1.89 | Valine | C00183 | HMDB0000883 | 118.10>55.10 | 3.15 |
| Acetylalanine | - | HMDB0000766 | 132.10>90.10 | 4.00 | Xanthosine | C01762 | HMDB0000299 | 295.10>153.20 | 8.61 |
| Acetylglycine | - | HMDB0000532 | 118.00>76.10 | 3.53 | Xanthurenic acid | C02470 | HMD60000881 | 206.00>160.00 | 9.75 |
| Adenosine | C00212 | HMDB0000050 | 268.10>136.10 | 8.35 | asy-Dimethylarginine | C03626 | HMDB0001539 | 203.10>46.10 | 2.47 |
| Adenosylhomocysteine | C00021 | HMDB0000939 | 385.10>13610 | 8.23 | sym-Dimethylarginine | - | HMDB0003334 | 203.10>172.20 | 2.57 |
| Adenosylmethionine | C00019 | HMDB0001185 | 399.10>250.10 | 2.18 | trans-urocanic acid | C00785 | HMDB0000301 | 139.10>93.10 | 4.70 |
| 2-Aminoadipic acid | C00956 | HMDB0000510 | 162.10>98.20 | 2.35 | 2-Hydroxy-3-methylvaleric acid | - | HMDB0000317 | 131.10>85.15 | 11.52 |
| Argininosuccinic acid | C03406 | HMDB0000052 | 201.10>70.10 | 2.08 | 2-Hydroxybutyric acid | C05984 | HMDB0000008 | 103.00>57.20 | 7.95 |
| Asparagine | C00152 | HMDB0000168 | 133.10>74.05 | 1.85 | | C01087 | HMDB0000606 | 147.00>129.05 | 4.27 |
| Aspartic acid | C00049 | HMDB0000191 | 134.10>88.00 | 1.55 | 2-Hydroxyisovaleric acid | - | HMDB0000407 | 117.10>71.10 | 9.89 |
| Betaine | C00719 | HMDB0000043 | 118.10>42.05 | 2.07 | 2-Oxogluteric acid α-Ketoglutaric acid | C00026 | HMDB0000208 | 145.10>57.10 | 3.24 |
| Creatine | C00300 | HMDB0000064 | 132.10>44.05 | 2.30 | 3-Hydroxybutyric acid | C01089 | HMDB0000357 | 103.00>59.10 | 6.64 |
| Creatinine | C00791 | HMDB0000562 | 114.10>86.10 | 2.18 | 3-Hydroxyisovaleric acid | - | HMDB0000754 | 117.10>59.10 | 8.89 |
| Cystathionine | C00542 | HMDB0000089 | 223.10>88.00 | 1.64 | 3-Methylglutaconic acid | - | HMDB0000522 | 143.00>99.05 | 10.07 |
| Cysteine | C00097 | HMDB0000574 | 122.00>59.00 | 2.04 | 4-Hydroxybenzoic acid | C00156 | HMDB0000500 | 137.00>93.00 | 10.98 |
| Cystine | C00491 | HMDB0000192 | 241.00>74.10 | 1.83 | AMP | - | HMDB00000045 | 346.10>79.05 | 4.14 |
| GSH | C00051 | HMDB0000125 | 308.10>179.20 | 4.32 | Aconitic acid | C02341 | HMDB0000958 | 173.00>85.15 | 8.17 |
| GSSG | C00127 | HMDB0003337 | 613.20>231.00 | 8.17 | Arabinonic acid | - | HMDB0000539 | 165.00>75.05 | 2.01 |
| Glutamic acid | C00025 | HMDB0000148 | 148.10>102.05 | 1.98 | Citric acid | C00158 | HMDB0000094 | 191.00>87.10 | 4.59 |
| Glulamine | C00064 | HMDB0000641 | 147.10>130.05 | 1.92 | Famaric acid | C00122 | HMDB0000134 | 115.00>71.10 | 5.93 |
| Glycine | C00037 | HMDB0000123 | 76.00>30.10 | 1.64 | Glutaric acid | C00489 | HMDB0000661 | 131.00>87.15 | 8.71 |
| Guanosine | C00387 | HMDB0000133 | 284.10>152.10 | 3.38 | Hippuric acid | C01686 | HMDB0000714 | 178.10>134.05 | 11.14 |
| Histidine | C00135 | HMDB0000177 | 156.10>83.20 | 1.84 | Indoxyl sulfate | - | HMDB0000682 | 212.00>132.20 | 10.58 |
| Indoleacetic acid | C00954 | HMDB0000197 | 176.10>130.10 | 13.67 | Isocitric acid | C00311 | HMDB0000193 | 191.00>73.10 | 2.96 |
| Indolelactic acid | C02043 | HMDB0000671 | 206.10>118.10 | 12.62 | Lactic acid | C00186 | HMDB0000190 | 89.00>43.10 | 3.67 |
| Indolepyruvic acid | C00331 | HMDB0060484 | 204.10>130.10 | 12.41 | Malic acid | C00711 | HMDB0000156 | 133.00>115.00 | 2.89 |
| Inosine | CD0294 | HMDB0000195 | 268.10>110.10 | 8.40 | Phenylactic acid | C05607 | HMPBMMi74 | 165.10>147.05 | 12.32 |
| Kynurenic acid | C01717 | HMDB0000715 | 190.10>144.00 | 10.13 | Pyridoxic acid | C00847 | HMDB0000017 | 187.00>105.05 | 8.56 |
| Kynurenine | C00326 | HMDB0000684 | 209.10>192.05 | 9.00 | Succinic acid | C00042 | HMDB0000254 | 117.00>73.05 | 5.90 |
| 3-Hydroxykynurenine | C02794 | HMDB0000732 | 225.10>110.15 | 7.48 | cAMP | C00575 | HMDB0000058 | 326.00>134.20 | 8.36 |
| N'-Formylkynurenine | C02700 | HMDB0001200 | 237.20>146.05 | 9.02 | Thyroxine | C01829 | HMDB0000248 | 777.70>731.50 | 15.07 |
| Leucine | C00123 | HMDB0000687 | 132.10>43.10 | 7.06 | Pipecolinic acid | C004G8 | HMDB0000073 | 130.10>84.30 | 3.41 |
| Isoleucine | C16434 | HMDB0000172 | 132.10>59.10 | 6.50 | 3-Indolepropionic acid | - | HMDB0002302 | 190.10>130.10 | 14.73 |
| Lysine | C00047 | HMDB0000182 | 147.10>84.10 | 1.64 | Quinolinic acid | C03722 | HMDB0000232 | 168.10>78.05 | 4.40 |
| Methionine | C00073 | HMDB0000696 | 150.10>104.00 | 3.83 | 3-Methylhistidine | C01152 | HMDB0000479 | 170.10>81.10 | 1.35 |
| S-Methylmethionine | C05319 | HMDB0039670 | 165.10>59.20 | 9.54 | Carnitine | C00318 | HMDB0000062 | 162.10>85.10 | 2.13 |
| N-Acetyl-Asp-Glu | C12270 | HMDB0001067 | 305.10>148.00 | 8.17 | Acetylcarnitine | C02571 | HMDB0000201 | 204.10>85.10 | 4.16 |
| N-Acetylaspartic acid | C01042 | HMDB0000812 | 176.10>134.00 | 4.06 | Propionylcarnitine | C03017 | HMDB0000824 | 218.10>85.10 | 8.49 |
| N-Acetythistidine | C02997 | HMDB0032055 | 198.10>110.10 | 2.81 | Isobutyrylcarnitine | - | HMDB0000736 | 232.20>85.15 | 9.38 |
| Nicotinamide | C00153 | HMDB0001406 | 123.10>80.05 | 5.13 | Butyrylcarnitine | C02862 | HMDB0002013 | 232.20>85.05 | 9.51 |
| Phenytalanine | C00079 | HMDB0000159 | 156.10>103.10 | 8.94 | 2-Methylbutylcarnitine | - | HMDB0000378 | 246.20>85.10 | 10.44 |
| Phosphocholine | C00588 | HMDB0001565 | | 1.85 | Isovalerycarnitine | C20826 | HMDB0000688 | 246.20>85.15 | 10.58 |
| Proline | C00148 | HMDB0000162 | 116.10>70.10 | 2.28 | Hexanoylcarnitine | - | HMDB0000705 | 250.20>85.15 | 11.88 |
| Pyroglutamic acid | C01879 | HMDB0000267 | 130.00>56.10 | 5.16 | Glutarylcarnitine | - | HMDB0013130 | 276.10>85.05 | 8.48 |
| Riboflavin | C00255 | HMDB0000244 | 377.10>243.20 | 10.59 | Adipoylcarnitine | - | HMDB0061677 | 290.20>85.00 | 8.96 |
| Serine | C00065 | HMDB0000187 | 106.10>60.00 | 1.85 | 2-Aminobutyric acid | C02356 | HMDB0000452 | 104.10>41.05 | 2.19 |
| Serotonin | C00780 | HMDB0000259 | 177.10>160.20 | 8.73 | Citoline | C00114 | HMDB0000097 | 104.10>60.00 | 1.99 |
| Putrescine | C00134 | HMDB0001414 | 89.10>72.10 | 1.60 | 3-Aminoisobutyric acid | C05145 | HMDB0003911 | 104.10>86.10 | 2.31 |
| Spermidine | C00315 | HMDB0001257 | 148.20>129.20 | 1.39 | Acetylcarnosine | - | HMDB0012881 | 269.10>110.10 | 4.25 |
| Spermine | C00750 | HMDB0001256 | 203.20>112.20 | 1.33 | Alanine | C00041 | HMDB0000161 | 90.00>44.10 | 1.91 |
| Taurine | C00245 | HMDB0000251 | 126.00>44.05 | 1.87 | Omithine | C00077 | HMDB0000214 | 133.10>115.05 | 1.85 |
| Threonine | C00188 | HMDB0000167 | 120.10>84.10 | 1.94 | Arginine | C00062 | HMDB0000517 | 175.10>70.10 | 1.83 |
| Trigonelline | C01004 | HMDB0000875 | 136.00>94.10 | 2.39 | Citrulline | C00327 | HMDB0000904 | 176.10>159.05 | 2.05 |
| Trimethyllysine | C03793 | HMDB0001325 | 189.20>50.25 | 1.82 | Glucuronic acid | C00191 | HMDB0000127 | 193.00>103.00 | 1.95 |
| Tryptophan | C00078 | HMDB0000929 | 205.10>115.20 | 9.84 | Gluconic acid | C00257 | HWDB0000625 | 195.10>129.00 | 1.96 |
| Tyrosine | C00082 | HMDB0000158 | 182.10>136.20 | 7.30 | N-Acetylglucosamine | C00140 | HMDB0000215 | 222.10>138.00 | 2.33 |
| Urea | C00088 | HMDB0000294 | 61.00>44.05 | 2.05 | N-Acetylneuraminic acid | C00270 | HMDB0000230 | 310.10>274.10 | 2.17 |
| Uric acid | C00366 | HMDB0000289 | 169.00>126.05 | 5.26 | 4-Guanidinobutaboic acid | C01035 | HMDB003464 | 146.10>86.15 | 3.42 |
| Uridine | C00299 | HMDB0000296 | 245.10>113.10 | 7.51 | N-Acetylomithine | C00437 | HMDB003357 | 175.10>70.05 | 2.67 |

**Table 3**

| Cellular markers measured by flow cytometry. |
|---|
| Cellular markers |
| % of CCR6⁺ cells among CD4⁺T cells |
| % of CD25⁺ cells among CD4⁺ T cells |
| % of CD4⁺ T cells among PBMC |
| % of CD8⁺ T cells among PBMC |
| % of CTLA4⁺ cells among CD4⁺ T cells |
| % of CXCR3⁺ cells among CD4⁺ T cells |
| % of FoxP3⁺ cells among CD4⁺ T cells |
| % of FoxP3^{high} cells among CD4⁺ T cells |
| % of FoxP3^{low} cells among CD4⁺ T cells |
| % of FoxP3⁺ CD25⁺ cells among CD4⁺ T cells |
| % of FoxP3^{low} CD45RA⁺ cells among CD4⁺ T cells |
| % of FoxP3⁺ CTLA4⁺ cells among CD4⁺ T cells |
| % of IFNγ⁺ cells among CD4⁺ T cells |
| % of IFNy⁺ cells among CDS⁺ T cells |
| % of KLRG1⁺ cells among CD4⁺ T cells |
| % of KLRG1⁺ CCR6⁺ cells among CD4⁺ T cells |
| % of KLRG1⁺ cells among CD8⁺ T cells |
| % of PD-1⁺ cells among CD4⁺ T cells |
| % of PD-1⁺ CD45⁺ cells among CD4⁺ T cells |
| % of PD-1⁺ FoxP3⁺ cells among CD4⁺ T cells |
| % of PD-1⁺ cells among CDB⁺ T cells |
| % of PD-1^{high} cells among CD8⁺ T cells |
| % of T-bet⁺ cells among CD4⁺ T cells |
| % ofT-bet^{high} cells among CD4⁺ T cells |
| % of T-bet⁺ KLRG1⁺ cells among CD4⁺ T cells |
| % of T-bet⁺ cells among CD8⁺ T cells |
| % of T-bet^{high} cells among CD8⁺ T cells |
| % of EOMES⁺ cells among CD8⁺ T cells |
| % of T-bet⁺ EOMES⁻ cells among CD8⁺ T cells |
| % of T-bet⁺ EOMES⁺ cells among CD8⁺ T cells |
| % of T-bet⁻ EOMES⁺ cells among CD8⁺ T cells |
| % of Tnaive among CD4⁺ T cells |
| % of Tcm among CD4⁺ T cells |
| % of Tem among CD4⁺ T cells |
| % of Temra among CD4⁺ T cells |
| % of Tnaive among CD8⁺ T cells |
| % of Tcm among CD8⁺ T cells |
| % of Tem among CD8⁺ T cells |
| % of Temra among CD8⁺ T cells |
| % of Tim3⁺ cells among CD4⁺ T cells |
| % of Tim3⁺ cells among CD8⁺ T cells |
| Cell ROX Green (MFI) of CD4⁺ T cells |
| Cell ROX Green (MFI) of CD8⁺ T cells |
| Mito SOX Red (MFI) of CD4⁺ T cells |
| Mito SOX Red (MFI) of CD8⁺ T cells |
| Mito Tracker Deep Red (MFI) of CD4⁺ T cells |
| Mito Tracker Deep Red (MFI) of CD8⁺ T cells |
| Mito Tracker Green (MFI) of CD4⁺ T cells |
| Mito Tracker Green (MFI) of CD8⁺ T cells |
| p-Akt (MFI) of CD8⁺ T cells |
| p-mTOR (MFI) of CD8⁺ T cells |
| PGC-1αβ (MFI) of CD8⁺ T cells |

**Table 4**

| Metabolites showing a significant dif fference betwe en respond ers (R) and no n-responders (NR). | | | | | | | | | |
|---|---|---|---|---|---|---|---|---|---|
| Metabolites | Time point | Changes in R relative to NR | Modality | p-value | Metabolites | Ratio of two time points | Changes in R retative to NR | Modality | p-value |
| Alanine | 1st | higher | GC | 0.0474 | | | | | |
| 4-Cresol | 1st | higher | GC | 0.0238 | Creatinine | 2nd/1st | lower | GC | 0.0329 |
| Cysteine | 1st | higher | GC | 0.0474 | 1,5-Anhydro-D-sorbitol | 2nd/1st | higher | GC | 0.0367 0.0419 |
| Hippuric acid | 1st | higher | GC/LC | 0.0062 / 0.0056 | Cystine Glutamine | 2nd/1st | higher | GC LC | 0.0114 |
| Oleic acid | 1st | higher | GC | 0.0281 | Glycine | 2nd/1st | higher | LC | 0.0095 |
| Indoxyl sulfate | 1st | higher | GC/LC | 0.0313 / 0.0225 | Lysine Pyroglutamic acid | 2nd/1st | higher | LC | 0.0329 0.0095 |
| Ribose | 1st | higher | GC | 0.0081 | Taurine | 2nd/1st | lower | LC | 0.0348 |
| Unk8 | 1st | lower | GC | 0.0273 | asy-Dimethylarginine | 2nd/1st | higher | LC | 0.0176 |
| indoleacetate | 1st | higher | LC | 0.018 | AMP | 2nd/1st | lower | LC | 0.0198 |
| Uric acid | 1st | higher | LC | 0.0126 | lsovalerylcarnitine | 2nd/1st | lower | LC | 0.0454 |
| Trans-urocanic acid | 1st | higher | LC | 0.0498 | Hexanoycamitine | 2nd/1st | lower | LC | 0.0367 |
| Pipecolic acid | 1st | lower | LC | 0.0252 | Acetylcarnosine | 2nd/1st | higher | LC | 0.0264 |
| N-Acetylglucosamine | 1st | higher | LC | 0.0281 | Arginine | 2nd/1st | higher | LC | 0.0329 |
| Uric acid | 2nd | higher | GC/LC | 0.0114 / 0.0176 | Citrulline N-accetylorinitine | 2nd/1st | higher | LC | 0.0348 0.0037 |
| Indolalactic acid | 2nd | higher | GC | 0.0387 | | | | | 0.0330 / |
| Atabinose | 2nd | higher | GC | 0.0186 | 3-Hydroxybutyric acid | 3rd/1st | lower | GC/LC | 0.0281 |
| Arabitol | 2nd | higher | GC | 0.0089 | | | | | 0.0040 / |
| Hippuric acid | 2nd | higher | GC | 0.0323 | 2-Hydroxyisovaleric acid | 3rd/1st | lower | GC/LC | 0.0028 |
| Cystine | 2nd | higher | GC | 0.0043 | Creatinine | 3rd/1st | lower | GC | 0.0099 |
| Indoxyle sulfate | 2nd | higher | GC/LC | 0.0454 / 0.0348 | Hippuric acid Oleic acid | 3rd/1st | higher lower | GC | 0.0016 0.0252 |
| Gluconic acid | 2nd | higher | GC | 0.0454 | Acatoacetic acid | 3rd/1st | lower | GC | 0.0483 |
| Citrulline | 2nd | higher | GC/LC | 0.0312 / 0.0122 | Ribose GSSG | 3rd/1st | lower higher | GC LC | 0.0008 0.0367 |
| Creatinine | 2nd | higher | LC | 0.0198 | Tryptophan | 3rd/1st | higher | LC | 0.0367 |
| N-Acetylaspartic acid | 2nd | higher | LC | 0.0311 | acid | 3rd/1st | lower | LC | 0.0099 |
| Pyroglutamic acid | 2nd | higher | LC | 0.0064 | 2-Hydroxyglutaric Malic acid | 3rd/1st | lower | LC | 0.0266 |
| Trimethyyllysine | 2nd | higher | LC | 0.0408 | Quinolinic acid | 3rd/1st | lower | LC | 0.0407 |
| Asy-Dimethylarginine | 2nd | higher | LC | 0.0028 | | 3rd/1st | lower | LC | 0.0043 |
| Sym-Dimethylarginine | 2nd | higher | LC | 0.0078 | Butyrylcarnitine Caproic acid | 3rd/1st | higher | GC | 0.0408 |
| Pipecolic acid | 2nd | lower | LC | 0.0329 | 4-Cresol | 3rd/2nd | higher | GC | 0.0138 |
| Methylhistidine | 2nd | higher | LC | 0.0176 | Isoleucine | 3rd/2nd | | GC | 0.0408 |
| Butyryllcarnitine | 2nd | lower | LC | 0.0101 | Arabinose | 3rd/2nd | higher lower | GC | 0.0114 |
| 3-Amitioisubutyric acid | 2nd | higher | LC | 0.0122 | Ribose | 3rd/2nd | lower | GC | 0.0028 |
| Acethykcamosine | 2nd | higher | LC | 0.0367 | GSH | 3rd/2nd | | LC | 0.0089 |
| Alanine | 2nd | higher | LC | 0.0348 | GSSG | 3rd/2nd | higher | LC | 0.0008 |
| Arginine | 2nd | higher | LC | 0.0114 | 3-OH-Kynurenine | 3rd/2nd | higher lower | LC | 0.0408 |
| N-accetyloriniline | 2nd | higher | LC | 0.0387 | acid | 3rd/2nd | | LC | 0.0165 |
| 4-Cresol | 3rd | higher | GC | 0.0023 | Hippuric Isobutyrylcarnitine | 3rd/2nd | higher | LC | 0.0095 |
| 3-Hydroxyisovaleric acid | 3rd | lower | GC/LC | 0.0213 / 0.0119 | | | | | |
| Pyruvic acid | 3rd | lower | GC | 0.0348 | | | | | |
| α-ketoglutaric acid | 3rd | lower | GC | 0.0238 0.0002 / | | | | | |
| Hippuric acid | 3rd | higher | GC/LC | 0.0003 | | | | | |
| Cystine | 3rd | higher | GC | 0.0201 0.0049 / | | | | | |
| Indoxyl sulfate | 3rd | higher | GC/LC | 0.0011 | | | | | |
| Unk13 | 3rd | higher | GC | 0.0451 | | | | | |
| GSSG | 3rd | higher | LC | 0.0068 | | | | | |
| Uric acid | 3rd | higher | LC | 0.0348 | | | | | |
| 2-Hydrobutyric acid | 3rd | lower | LC | 0.0281 | | | | | |
| Pipecolic acid | 3rd | lower | LC | 0.0348 | | | | | |
| Butyryllcarnitine | 3rd | lower | LC | 0.0015 | | | | | |
| *: p-value for distincti on beween R and NR (Wilc oxon rank s sum test). | | | | | | | | | |

**Table 5**

| Cellular markers showing a significant difference between responders (R) and non-responders (NR). | | | |
|---|---|---|---|
| Cellular markers | Time point or ratio of two time points | Changes in R relative to NR | p-value^{∗} |
| % of CD4⁺ T cells among PBMC | 2nd | higher | 0.0107 |
| % of CD4⁺ T cells among PBMC | 2nd/1st | higher | 0.0001 |
| % of CD8⁺ T cells among PBMC | 2nd | higher | 0.0478 |
| % of CD8⁺ T cells among PBMC | 2nd/1st | higher | 0.0348 |
| % of Tnaive among CD8⁺ T cells | 2nd/1st | higher | 0.0176 |
| % of Tem among CD4⁺ T cells | 2nd/1st | higher | 0.0095 |
| % of Tem among CD8⁺ T cells | 2nd/1st | higher | 0.0138 |
| % of Tem among CD8⁺ T cells | 3rd/1st | higher | 0.0213 |
| % of Temra among CD4⁺ T cells | 2nd/1st | lower | 0.0107 |
| % of Temra among CD4⁺ T cells | 3rd/2nd | higher | 0.0081 |
| % of Temra among CD8⁺ T cells | 2nd/1st | lower | 0.0009 |
| Mito SOX CD8/CD4 | 1st | higher | 0.0028 |
| Mito SOX CD8/CD4 | 2nd | higher | 0.0089 |
| Mito SOX CD8/CD4 | 3rd | higher | 0.018 |
| Mito SOX CD8/CD4 | 3rd/1st | lower | 0.019 |
| Mito mass CD8/CD4 | 1st | higher | 0.0451 |
| Mito mass CD8/CD4 | 3rd | higher | 0.0348 |
| PGC-1αβ (MFI) of CD8⁺ T cells | 2nd | lower | 0.0176 |
| PGC-1αβ (MFI) of CD8⁺ T cells | 2nd/1st | lower | 0.0052 |
| PGC-1αβ (MFI) of CD8⁺ T cells | 3rd/2nd | higher | 0.0001 |
| % of PD-1^{high} among CD8⁺ T cells | 1st | lower | 0.013 |
| % of FoxP3^{low} CD45RA⁺ among CD4⁺ T cells | 1st | lower | 0.027 |
| % of T-bet^{high} among CD4⁺ T cells | 3rd/1st | higher | 0.03 |
| % of T-bet^{low} among CD4⁺ T cells | 3rd/1st | higher | 0.0214 |
| % of T-bet⁺ among CD8⁺ T cells | 3rd/2nd | higher | 0.0295 |
| % of T-bet⁺ EOMES⁺ among CD8⁺ T cells | 3rd/2nd | higher | 0.0408 |
| *: p-value for distinction beween R and NR (Wilcoxon rank sum test). | | | |

### References

1. Tang J, et al. (2018) Trial watch: The clinical trial landscape for PD1/PDL1 immune checkpoint inhibitors. Nature reviews. Drug discovery 17(12):854-855.
2. Schmidt EV (2018) Developing combination strategies using PD-1 checkpoint inhibitors to treat cancer. Seminars in immunopathology.
3. Chowdhury PS, Chamoto K, & Honjo T (2018) Combination therapy strategies for improving PD-1 blockade efficacy: a new era in cancer immunotherapy. J Intern Med 283(2):110-120.
4. Pallister T, et al. (2017) Hippurate as a metabolomic marker of gut microbiome diversity: Modulation by diet and relationship to metabolic syndrome. Sci Rep 7(1):13670.
5. Li M, et al. (2008) Symbiotic gut microbes modulate human metabolic phenotypes. Proceedings of the National Academy of Sciences of the United States of America 105(6):2117-2122.
6. Schleif R (2010) AraC protein, regulation of the 1-arabinose operon in Escherichia coli, and the light switch mechanism of AraC action. FEMS microbiology reviews 34(5):779-796.
7. Jones LL, McDonald DA, & Borum PR (2010) Acylcarnitines: role in brain. Progress in lipid research 49(1):61-75.
8. Schooneman MG, Vaz FM, Houten SM, & Soeters MR (2013) Acylcarnitines: reflecting or inflicting insulin resistance? Diabetes 62(1):1-8.
9. Rinaldo P, Cowan TM, & Matern D (2008) Acylcarnitine profile analysis. Genetics in medicine : official journal of the American College of Medical Genetics 10(2):151-156.
10. Scharping NE, et al. (2016) The Tumor Microenvironment Represses T Cell Mitochondrial Biogenesis to Drive Intratumoral T Cell Metabolic Insufficiency and Dysfunction. Immunity 45(3):701-703.
11. Ventura-Clapier R, Garnier A, & Veksler V (2008) Transcriptional control of mitochondrial biogenesis: the central role of PGC-1alpha. Cardiovascular research 79(2):208-217.
12. Dunn GP, Old LJ, & Schreiber RD (2004) The immunobiology of cancer immunosurveillance and immunoediting. Immunity 21(2):137-148.
13. Leach DR, Krummel MF, & Allison JP (1996) Enhancement of antitumor immunity by CTLA-4 blockade. Science 271(5256):1734-1736.
14. Iwai Y, et al. (2002) Involvement of PD-L1 on tumor cells in the escape from host immune system and tumor immunotherapy by PD-L1 blockade. Proceedings of the National Academy of Sciences of the United States of America 99(19): 12293-12297.
15. Iwai Y, Hamanishi J, Chamoto K, & Honjo T (2017) Cancer immunotherapies targeting the PD-1 signaling pathway. Journal of biomedical science 24(1):26.
16. Hodi FS, et al. (2010) Improved survival with ipilimumab in patients with metastatic melanoma. The New England journal of medicine 363(8):711-723.
17. Topalian SL, et al. (2012) Safety, activity, and immune correlates of anti-PD-1 antibody in cancer. The New England journal of medicine 366(26):2443-2454.
18. Topalian SL, Drake CG, & Pardoll DM (2015) Immune checkpoint blockade: a common denominator approach to cancer therapy. Cancer cell 27(4):450-461.
19. Zou W, Wolchok JD, & Chen L (2016) PD-L1 (B7-H1) and PD-1 pathway blockade for cancer therapy: Mechanisms, response biomarkers, and combinations. Science translational medicine 8(328):328rv324.
20. Le DT, et al. (2015) PD-1 Blockade in Tumors with Mismatch-Repair Deficiency. The New England journal of medicine 372(26):2509-2520.
21. Le DT, et al. (2017) Mismatch repair deficiency predicts response of solid tumors to PD-1 blockade. Science 357(6349):409-413.
22. Mitsuhashi A & Okuma Y (2018) Perspective on immune oncology with liquid biopsy, peripheral blood mononuclear cells, and microbiome with non-invasive biomarkers in cancer patients. Clinical & translational oncology : official publication of the Federation of Spanish Oncology Societies and of the National Cancer Institute of Mexico 20(8):966-974.
23. Fagarasan S, et al. (2002) Critical roles of activation-induced cytidine deaminase in the homeostasis of gut flora. Science 298(5597): 1424-1427.
24. Kawamoto S, et al. (2012) The inhibitory receptor PD-1 regulates IgA selection and bacterial composition in the gut. Science 336(6080):485-489.
25. Gopalakrishnan V, et al. (2018) Gut microbiome modulates response to anti-PD-1 immunotherapy in melanoma patients. Science 359(6371):97-103.
26. Routy B, et al. (2018) Gut microbiome influences efficacy of PD-1-based immunotherapy against epithelial tumors. Science 359(6371):91-97.
27. Zitvogel L, Ayyoub M, Routy B, & Kroemer G (2016) Microbiome and Anticancer Immunosurveillance. Cell 165(2):276-287.
28. Johnson CH, Spilker ME, Goetz L, Peterson SN, & Siuzdak G (2016) Metabolite and Microbiome Interplay in Cancer Immunotherapy. Cancer research 76(21):6146-6152.
29. Pearce EL, et al. (2009) Enhancing CD8 T-cell memory by modulating fatty acid metabolism. Nature 460(7251): 103-107.
30. Geltink RIK, Kyle RL, & Pearce EL (2018) Unraveling the Complex Interplay Between T Cell Metabolism and Function. Annu Rev Immunol 36:461-488.
31. Chamoto K, et al. (2017) Mitochondrial activation chemicals synergize with surface receptor PD-1 blockade for T cell-dependent antitumor activity. Proceedings of the National Academy of Sciences of the United States of America 114(5):E761-E770.
32. Chowdhury PS, Chamoto K, Kumar A, & Honjo T (2018) PPAR-induced fatty acid oxidation in T cells increases the number of tumor-reactive CD8+ T cells and facilitates anti-PD-1 therapy. Cancer immunology research.
33. Murphy MP & Siegel RM (2013) Mitochondrial ROS fire up T cell activation. Immunity 38(2):201-202.
34. Brahmer J, et al. (2015) Nivolumab versus Docetaxel in Advanced Squamous-Cell Non-Small-Cell Lung Cancer. The New England journal of medicine 373(2):123-135.
35. Borghaei H, et al. (2015) Nivolumab versus Docetaxel in Advanced Nonsquamous Non-Small-Cell Lung Cancer. The New England journal of medicine 373(17):1627-1639.
36. Wikoff WR, et al. (2009) Metabolomics analysis reveals large effects of gut microflora on mammalian blood metabolites. Proceedings of the National Academy of Sciences of the United States of America 106(10):3698-3703.
37. Franchina DG, He F, & Brenner D (2018) Survival of the fittest: Cancer challenges T cell metabolism. Cancer letters 412:216-223.
38. Miyajima M, et al. (2017) Metabolic shift induced by systemic activation of T cells in PD-1-deficient mice perturbs brain monoamines and emotional behavior. Nature immunology 18(12):1342-1352.
39. Fernandez-Marcos PJ & Auwerx J (2011) Regulation of PGC-1alpha, a nodal regulator of mitochondrial biogenesis. The American journal of clinical nutrition 93(4):884S-890.
40. Takeuchi Y, et al. (2018) Clinical response to PD-1 blockade correlates with a sub-fraction of peripheral central memory CD4+ T cells in patients with malignant melanoma. Int Immunol 30(1):13-22.
41. Manjarrez-Orduno N, et al. (2018) Circulating T Cell Subpopulations Correlate With Immune Responses at the Tumor Site and Clinical Response to PD1 Inhibition in Non-Small Cell Lung Cancer. Front Immunol 9:1613.
42. Vanderwalde A, Spetzler D, Xiao N, Gatalica Z, & Marshall J (2018) Microsatellite instability status determined by next-generation sequencing and compared with PD-L1 and tumor mutational burden in 11,348 patients. Cancer Med 7(3):746-756.
43. Galluzzi L, Chan TA, Kroemer G, Wolchok JD, & Lopez-Soto A (2018) The hallmarks of successful anticancer immunotherapy. Science translational medicine 10(459).
44. Calvani R, et al. (2010) Gut microbiome-derived metabolites characterize a peculiar obese urinary metabotype. International journal of obesity 34(6):1095-1098.
45. Clayton TA, Baker D, Lindon JC, Everett JR, & Nicholson JK (2009) Pharmacometabonomic identification of a significant host-microbiome metabolic interaction affecting human drug metabolism. Proceedings of the National Academy of Sciences of the United States of America 106(34):14728-14733.
46. Koves TR, et al. (2008) Mitochondrial overload and incomplete fatty acid oxidation contribute to skeletal muscle insulin resistance. Cell metabolism 7(1):45-56.
47. Gatza E, et al. (2011) Manipulating the bioenergetics of alloreactive T cells causes their selective apoptosis and arrests graft-versus-host disease. Science translational medicine 3(67):67ra68.
48. Mihalik SJ, et al. (2010) Increased levels of plasma acylcarnitines in obesity and type 2 diabetes and identification of a marker of glucolipotoxicity. Obesity 18(9):1695-1700.
49. Sun L, et al. (2016) Early Prediction of Developing Type 2 Diabetes by Plasma Acylcarnitines: A Population-Based Study. Diabetes care 39(9):1563-1570.
50. Ruiz M, et al. (2017) Circulating acylcarnitine profile in human heart failure: a surrogate of fatty acid metabolic dysregulation in mitochondria and beyond. American journal of physiology. Heart and circulatory physiology 313(4):H768-H781.
51. Sena LA, et al. (2013) Mitochondria are required for antigen-specific T cell activation through reactive oxygen species signaling. Immunity 38(2):225-236.
52. Ballatori N, et al. (2009) Glutathione dysregulation and the etiology and progression of human diseases. Biological chemistry 390(3):191-214.
53. Franco R & Cidlowski JA (2012) Glutathione efflux and cell death. Antioxidants & redox signaling 17(12):1694-1713.
54. Huang AC, et al. (2017) T-cell invigoration to tumour burden ratio associated with anti-PD-1 response. Nature 545(7652):60-65.
55. Kamphorst AO, et al. (2017) Proliferation of PD-1+CD8 T cells in peripheral blood after PD-1-targeted therapy in lung cancer patients. Proceedings of the National Academy of Sciences of the United States of America 114(19):4993-4998.
56. Thommen DS, et al. (2018) A transcriptionally and functionally distinct PD-1(+) CD8(+) T cell pool with predictive potential in non-small-cell lung cancer treated with PD-1 blockade. Nat Med 24(7):994-1004.
57. Dunn WB, et al. (2011) Procedures for large-scale metabolic profiling of serum and plasma using gas chromatography and liquid chromatography coupled to mass spectrometry. Nat Protoc 6(7):1060-1083.
58. Kirwan JA, Broadhurst DI, Davidson RL, & Viant MR (2013) Characterising and correcting batch variation in an automated direct infusion mass spectrometry (DIMS) metabolomics workflow. Anal Bioanal Chem 405(15):5147-5157.
59. Rusilowicz M, Dickinson M, Charlton A, O'Keefe S, & Wilson J (2016) A batch correction method for liquid chromatography-mass spectrometry data that does not depend on quality control samples. Metabolomics 12:56.

### [Example 2] Definition of PD-1^{high}

CD8⁺ T cells from age matched 30 healthy donors were gated and PD-1 staining data were overlaid (Fig. 11A, upper panel). X axis represents PD-1 expression level and Y axis relative cell count. Vertical lines indicate the 50^{th}, 90^{th}, 97^{th} and 99^{th} percentiles of PD-1 expression average. The value of correlation coefficient (r) between the frequency (%) of PD-1^{high} CD⁸⁺ T cells and the gene expression of CD8⁺ T cell exhaustion markers (CTLA-4, Tim-3 and Lag-3) in patients (the patients of Example 1) at each percentile is shown in the table (Fig. 11A, lower panel). The right panel (Fig. 11B) shows correlation diagrams. At 97^{th} percentile, there was high correlation with expression of any of the exhaustion marker genes. Therefore, 97^{th} percentile was determined as the PD-1^{high} cut-off value.

### [Example 3]

It is a known fact that PD-1 antibody therapy does not work well when lung cancer cells have an EGFR mutation. LDA analysis was performed to determine whether the cellular marker combination II of the present invention can discriminate the non-responsiveness resulting from the presence of EGFR mutation (8 patients having an EGFR mutation were selected as subjects from the patients of Example 1). As a result, the error rate was 0%, making it clear that the cellular marker combination II is capable of discriminating the non-responsiveness due to EGFR mutation (Fig. 12).

### [Example 4]

In order to examine whether the cellular marker combination II can also judge therapeutic efficacy in other cancer species, samples from 11 head & neck cancer patients (after or before administration of Nivolumab at Kyoto University Hospital in the second-line treatment or thereafter in the same manner as in treatment of lung cancer) were stained in the same manner and subjected to LDA analysis. As a result, efficacy could be judged at an error rate of 9.1% (Fig. 13).

All publications, patents and patent applications cited herein are incorporated herein by reference in their entirety.

### INDUSTRIAL APPLICABILITY

By using the biomarkers and/or cellular markers of the present invention, the efficacy of therapy with a PD-1 signal inhibitor-containing drug can be predicted and/or judged before or at an early stage of the therapy. As a result, the efficiency of therapy can be improved while reducing its cost.

## Claims

1. A test method comprising predicting and/or judging the efficacy of therapy with a PD-1 signal inhibitor-containing drug using the following (i) and/or (ii) as indicator(s):
(i) at least one metabolite in serum and/or plasma selected from the group consisting of alanine, 4-cresol, cysteine, hippuric acid, oleic acid, indoxyl sulfate, ribose, indoleacetate, uric acid, trans-urocanic acid, pipecolic acid, N-acetylglucosamine, indolelactic acid, arabinose, arabitol, cystine, indoxyl sulfate, gluconic acid, citrulline, creatinine, N-acetylaspartic acid, pyroglutamic acid, trimethyllysine, asy-dimethylarginine, sym-dimethylarginine, methylhistidine, acylcamitine, 3-aminoisobutyric acid, acetylcarnosine, arginine, N-acetylornitine, 3-hydroxyisovaleric acid, pyruvic acid, α-ketoglutaric acid, GSSG, 2-hydrobutyric acid, 1,5-anhydro-D-sorbitol, glutamine, glycine, lysine, taurine, AMP, acetylcarnosine, 3-hydroxybutyric acid, 2-hydroxyisovaleric acid, acetoacetic acid, tryptophan, 2-hydroxyglutaric acid, malic acid, quinolinic acid, caproic acid, isoleucine, GSH and 3-OH-kynurenine
(ii) at least one cellular marker in peripheral blood selected from the group consisting of the frequency of CD4⁺ T cells among peripheral blood mononuclear cells (% of CD4⁺ T cells among PBMC), the frequency of CD8⁺ T cells among peripheral blood mononuclear cells (% of CD8⁺ T cells among PBMC), the frequency of naive T cells among CD8⁺ T cells (% of Tnaive among CD8⁺ T cells), the frequency of central memory T cells among CD4⁺ T cells (% of Tcm among CD4⁺ T cells), the frequency of central memory T cells among CD8⁺ T cells (% of Tcm among CD8⁺ T cells), the frequency of effector memory T cells among CD8⁺ T cells (% of Tem among CD8⁺ T cells), the frequency of terminally differentiated effector memory T cells among CD4⁺ T cells (% of Temra among CD4⁺ T cells), the frequency of terminally differentiated effector memory T cells among CD8⁺ T cells (% of Temra among CD8⁺ T cells), the ratio of mitochondrial activation status in CD8⁺ T cells to mitochondrial activation status in CD4⁺ T cells [e.g., the ratio of mitochondrial reactive oxygen species production in CD8⁺ T cells to mitochondrial reactive oxygen species production in CD4⁺ T cells (Mito SOX CD8/CD4), the ratio of mitochondrial mass in CD8⁺ T cells to mitochondrial mass in CD4⁺ T cells (Mito mass CD8/CD4)], PGC-la and PGC-1β expression in CD8⁺ T cells (PGC-1αβ (MF1) of CD8⁺ T cells), the frequency of PD-1 highly expressing CD8⁺ T cell population (% of PD-1^{high} among CD8⁺ T cells), the frequency of FoxP3 lowly expressing CD45RA⁺ T cell population among CD4⁺ T cells (% of FoxP3^{low} CD45RA⁺ among CD4⁺ T cells), the frequency of T-bet highly expressing T cell population among CD4⁺ T cells (% of T-bet^{high} among CD4⁺ T cells), the frequency of T-bet lowly expressing T cell population among CD4⁺ T cells (% of T-bet^{low} among CD4⁺ T cells), the frequency of T-bet expressing T cell population among CD8⁺ T cells (% of T-bet⁺ among CD8⁺ T cells) and the frequency of T-bet and EOMES expressing T cell population among CD8⁺ T cells (% of T-bet⁺ EOMES⁺ among CD8⁺ T cells).

2. The method according to claim 1, comprising predicting and/or judging the efficacy of therapy with a PD-1 signal inhibitor-containing drug based on a criterion whether the level(s) of metabolite and/or cellular marker at the time point or the ratio of two time points as indicated in Tables 4 and 5 is(are) high or low (Changes in R relative to NR).

3. The method according to claim 1 or 2, wherein the metabolite of (i) is at least one metabolite selected from the group consisting of hippuric acid, arabinose and acylcarnitine, and the cellular marker of (ii) is at least one cellular marker selected from the group consisting of the frequency of PD-1 highly expressing CD⁸⁺ T cell population, the ratio of mitochondrial reactive oxygen species production in CD⁸⁺ T cells to mitochondrial reactive oxygen species production in CD⁴⁺ T cells, and PGC-la and PGC-1β expression in CD⁸⁺ T cells.

4. The method according to claim 1, wherein the therapy with the drug is predicted effective when the level of cysteine in serum and/or plasma before administration of the drug is high and the level of hippuric acid in serum and/or plasma before administration of the drug is high.

5. The method according to claim 1, wherein the therapy with the drug is judged effective when the level of arabinose in serum and/or plasma after 1^{st} administration of the drug is high; the level of arginine in serum and/or plasma after 1^{st} administration of the drug is high; and the level of butyrylcarnitine in serum and/or plasma after 1^{st} administration of the drug is low.

6. The method according to claim 1, wherein the therapy with the drug is judged effective when the level of hippuric acid in serum and/or plasma before administration of the drug is high; the level of cystine in serum and/or plasma after 1^{st} administration of the drug is high; the level of glutathione disulfide in serum and/or plasma after 2^{nd} administration of the drug is high; and the level of butyrylcarnitine in serum and/or plasma after 2^{nd} administration of the drug is low.

7. The method according to any one of claims 1 to 6, wherein the therapy with the drug is predicted effective when the frequency of PD-1 highly expressing CD⁸⁺ T cell population in peripheral blood before administration of the drug is low and the ratio of mitochondrial reactive oxygen species production in CD⁸⁺ T cells to mitochondrial reactive oxygen species production in CD⁴⁺ T cells in peripheral blood before administration of the drug is high.

8. The method according to any one of claims 1 to 6, wherein the therapy with the drug is judged effective when the frequency of PD-1 highly expressing CD⁸⁺ T cell population in peripheral blood before administration of the drug is low; the ratio of mitochondrial reactive oxygen species production in CD⁸⁺ T cells to mitochondrial reactive oxygen species production in CD⁴⁺ T cells in peripheral blood before administration of the drug is high; the ratio of PGC-la and PGC-1β expression in CD⁸⁺ T cells in peripheral blood after 1^{st} administration of the drug to the corresponding expression before administration of the drug is low; and the ratio of the frequency of CD⁴⁺ T cells in PBMC after 1^{st} administration of the drug to the corresponding frequency before administration of the drug is high.

9. The method according to any one of claims 1 to 6, wherein the therapy with the drug is judged effective when the frequency of PD-1 highly expressing CD⁸⁺ T cell population in peripheral blood before administration of the drug is low; the ratio of mitochondrial reactive oxygen species production in CD⁸⁺ T cells to mitochondrial reactive oxygen species production in CD⁴⁺ T cells in peripheral blood before administration of the drug is high; the ratio of PGC-la and PGC-1β expression in CD⁸⁺ T cells in peripheral blood after 2^{nd} administration of the drug to the corresponding expression after 1^{st} administration of the drug is high; and the ratio of the frequency of CD⁴⁺ T cells in PBMC after 1^{st} administration of the drug to the corresponding frequency before administration of the drug is high.

10. The method according to any one of claims 1 to 9, wherein the PD-1 signal inhibitor is an antibody.

11. The method according to claim 10, wherein the antibody is at least one antibody selected from the group consisting of anti-PD-1 antibody, anti-PD-Ll antibody and anti-PD-L2 antibody.

12. The method according to any one of claims 1 to 11, wherein the PD-1 signal inhibitor-containing drug is used as an active ingredient of an anticancer agent, an anti-infective agent or a combination thereof.

13. A method of diagnosis and therapy for a disease, comprising predicting and/or judging the efficacy of therapy with a PD-1 signal inhibitor-containing drug in a subject using the following (i) and/or (ii) as indicator(s), and administering to the subject in a therapeutically effective amount when the therapy with the PD-1 signal inhibitor-containing drug has been predicted and/or judged effective:
(i) at least one metabolite in serum and/or plasma selected from the group consisting of alanine, 4-cresol, cysteine, hippuric acid, oleic acid, indoxyl sulfate, ribose, indoleacetate, uric acid, trans-urocanic acid, pipecolic acid, N-acetylglucosamine, indolelactic acid, arabinose, arabitol, cystine, indoxyl sulfate, gluconic acid, citrulline, creatinine, N-acetylaspartic acid, pyroglutamic acid, trimethyllysine, asy-dimethylarginine, sym-dimethylarginine, methylhistidine, acylcarnitine, 3-aminoisobutyric acid, acetylcarnosine, arginine, N-acetylornitine, 3-hydroxyisovaleric acid, pyruvic acid, α-ketoglutaric acid, GSSG, 2-hydrobutyric acid, 1,5-anhydro-D-sorbitol, glutamine, glycine, lysine, taurine, AMP, acetylcarnosine, 3-hydroxybutyric acid, 2-hydroxyisovaleric acid, acetoacetic acid, tryptophan, 2-hydroxyglutaric acid, malic acid, quinolinic acid, caproic acid, isoleucine, GSH and 3-OH-kynurenine
(ii) at least one cellular marker in peripheral blood selected from the group consisting of the frequency of CD4⁺ T cells among peripheral blood mononuclear cells (% of CD4⁺ T cells among PBMC), the frequency of CD8⁺ T cells among peripheral blood mononuclear cells (% of CD8⁺ T cells among PBMC), the frequency of naive T cells among CD8⁺ T cells (% of Tnaive among CD8⁺ T cells), the frequency of central memory T cells among CD4⁺ T cells (% of Tcm among CD4⁺ T cells), the frequency of central memory T cells among CD8⁺ T cells (% of Tcm among CD8⁺ T cells), the frequency of effector memory T cells among CD8⁺ T cells (% of Tem among CD8⁺ T cells), the frequency of terminally differentiated effector memory T cells among CD4⁺ T cells (% of Temra among CD4⁺ T cells), the frequency of terminally differentiated effector memory T cells among CD8⁺ T cells (% of Temra among CD8⁺ T cells), the ratio of mitochondrial activation status in CD8⁺ T cells to mitochondrial activation status in CD4⁺ T cells [e.g., the ratio of mitochondrial reactive oxygen species production in CD8⁺ T cells to mitochondrial reactive oxygen species production in CD4⁺ T cells (Mito SOX CD8/CD4), the ratio of mitochondrial mass in CD8⁺ T cells to mitochondrial mass in CD4⁺ T cells (Mito mass CD8/CD4)], PGC-la and PGC-1β expression in CD8⁺ T cells (PGC-1αβ (MF1) of CD8⁺ T cells), the frequency of PD-1 highly expressing CD8⁺ T cell population (% of PD-1^{high} among CD8⁺ T cells), the frequency of FoxP3 lowly expressing CD45RA⁺ T cell population among CD4⁺ T cells (% of FoxP3^{low} CD45RA⁺ among CD4⁺ T cells), the frequency of T-bet highly expressing T cell population among CD4⁺ T cells (% of T-bet^{high} among CD4⁺ T cells), the frequency of T-bet lowly expressing T cell population among CD4⁺ T cells (% of T-bet^{low} among CD4⁺ T cells), the frequency of T-bet expressing T cell population among CD8⁺ T cells (% of T-bet⁺ among CD8⁺ T cells) and the frequency of T-bet and EOMES expressing T cell population among CD8⁺ T cells (% of T-bet⁺ EOMES⁺ among CD8⁺ T cells).

14. A pharmaceutical composition comprising a PD-1 signal inhibitor-containing drug as an active ingredient, which is for use in a method of diagnosis and therapy for a disease, comprising predicting and/or judging the efficacy of therapy with the PD-1 signal inhibitor-containing drug in a subject using the following (i) and/or (ii) as indicator(s), and administering to the subject in a therapeutically effective amount when the therapy with the PD-1 signal inhibitor-containing drug has been predicted and/or judged effective:
(i) at least one metabolite in serum and/or plasma selected from the group consisting of alanine, 4-cresol, cysteine, hippuric acid, oleic acid, indoxyl sulfate, ribose, indoleacetate, uric acid, trans-urocanic acid, pipecolic acid, N-acetylglucosamine, indolelactic acid, arabinose, arabitol, cystine, indoxyl sulfate, gluconic acid, citrulline, creatinine, N-acetylaspartic acid, pyroglutamic acid, trimethyllysine, asy-dimethylarginine, sym-dimethylarginine, methylhistidine, acylcarnitine, 3-aminoisobutyric acid, acetylcarnosine, arginine, N-acetylornitine, 3-hydroxyisovaleric acid, pyruvic acid, α-ketoglutaric acid, GSSG, 2-hydrobutyric acid, 1,5-anhydro-D-sorbitol, glutamine, glycine, lysine, taurine, AMP, acetylcarnosine, 3-hydroxybutyric acid, 2-hydroxyisovaleric acid, acetoacetic acid, tryptophan, 2-hydroxyglutaric acid, malic acid, quinolinic acid, caproic acid, isoleucine, GSH and 3-OH-kynurenine
(ii) at least one cellular marker selected in peripheral blood from the group consisting of the frequency of CD4⁺ T cells among peripheral blood mononuclear cells (% of CD4⁺ T cells among PBMC), the frequency of CD8⁺ T cells among peripheral blood mononuclear cells (% of CD8⁺ T cells among PBMC), the frequency of naive T cells among CD8⁺ T cells (% of Tnaive among CD8⁺ T cells), the frequency of central memory T cells among CD4⁺ T cells (% of Tcm among CD4⁺ T cells), the frequency of central memory T cells among CD8⁺ T cells (% of Tcm among CD8⁺ T cells), the frequency of effector memory T cells among CD8⁺ T cells (% of Tem among CD8⁺ T cells), the frequency of terminally differentiated effector memory T cells among CD4⁺ T cells (% of Temra among CD4⁺ T cells), the frequency of terminally differentiated effector memory T cells among CD8⁺ T cells (% of Temra among CD8⁺ T cells), the ratio of mitochondrial activation status in CD8⁺ T cells to mitochondrial activation status in CD4⁺ T cells [e.g., the ratio of mitochondrial reactive oxygen species production in CD8⁺ T cells to mitochondrial reactive oxygen species production in CD4⁺ T cells (Mito SOX CD8/CD4), the ratio of mitochondrial mass in CD8⁺ T cells to mitochondrial mass in CD4⁺ T cells (Mito mass CD8/CD4)], PGC-la and PGC-1β expression in CD8⁺ T cells (PGC-1αβ (MF1) of CD8⁺ T cells), the frequency of PD-1 highly expressing CD8⁺ T cell population (% of PD-1^{high} among CD8⁺ T cells), the frequency of FoxP3 lowly expressing CD45RA⁺ T cell population among CD4⁺ T cells (% of FoxP3^{low} CD45RA⁺ among CD4⁺ T cells), the frequency of T-bet highly expressing T cell population among CD4⁺ T cells (% of T-bet^{high} among CD4⁺ T cells), the frequency of T-bet lowly expressing T cell population among CD4⁺ T cells (% of T-bet^{low} among CD4⁺ T cells), the frequency of T-bet expressing T cell population among CD8⁺ T cells (% of T-bet⁺ among CD8⁺ T cells) and the frequency of T-bet and EOMES expressing T cell population among CD8⁺ T cells (% of T-bet⁺ EOMES⁺ among CD8⁺ T cells).
